# EUROPEAN PATENT SPECIFICATION

(11) **EP 3 443 085 B1**
(45) Date of publication and mention of the grant of the patent: **14.09.2022**
(21) Application number: 17783170.8
(22) Date of filing: 13.04.2017
(51) Int. Cl.: C12N 15/09, C12N 15/90, C12N 5/0797, A61K 48/00, A61K 35/30

(54) **GENOME EDITING OF HUMAN NEURAL STEM CELLS USING NUCLEASES**
GENOMÄNDERUNG VON MENSCHLICHEN NEURONALEN STAMMZELLEN UNTER VERWENDUNG VON NUKLEASEN
ÉDITION DU GÉNOME DE CELLULES SOUCHES NEURALES HUMAINES À L'AIDE DE NUCLÉASES

(30) Priority: 14.04.2016 US 201662322652 P
(43) Date of publication of application: 20.02.2019
(73) Proprietor: BOCO Silicon Valley, Inc., Palo Alto CA 94303 (US); The Board of Trustees of the Leland Stanford Junior University, Stanford, CA 94305-2038 (US)
(72) Inventor: PORTEUS, Matthew, H., Stanford, CA 94305-2038 (US); KILDEBECK, Eric, J., Stanford, CA 94305-2038 (US); DEVER, Daniel, P., Stanford, CA 94305-2038 (US); CLARK, Joseph, T., Stanford, CA 94305-2038 (US); TSUKAMOTO, Ann, Palo Alto, CA 94303 (US); UCHIDA, Nobuko, Palo Alto, CA 94303 (US)
(74) Representative: Mewburn Ellis LLP
(86) International application number: PCT/US2017/027504
(87) International publication number: WO 2017/180926

(56) References cited:
- WO-A1-2015/057976
- WO-A1-2015/073867
- WO-A1-2015/073867
- WO-A1-2015/188056
- ANGELO LOMBARDO ET AL: "Site-specific integration and tailoring of cassette design for sustainable gene transfer", NATURE METHODS, vol. 8, no. 10, 1 October 2011 (2011-10-01), pages 861-869, XP055602217, New York ISSN: 1548-7091, DOI: 10.1038/nmeth.1674
- TREVOR CERBINI ET AL: "Transcription Activator-Like Effector Nuclease (TALEN)-Mediated CLYBL Targeting Enables Enhanced Transgene Expression and One-Step Generation of Dual Reporter Human Induced Pluripotent Stem Cell (iPSC) and Neural Stem Cell (NSC) Lines", PLOS ONE, vol. 10, no. 1, 14 January 2015 (2015-01-14), page e0116032, XP055602218, DOI: 10.1371/journal.pone.0116032
- Daniel P Dever ET AL: "CRISPR/Cas9 Genome Engineering in Engraftable Human Brain-Derived Neural Stem Cells", iScience, 31 May 2019 (2019-05-31), pages 524-535, XP055611437, United States DOI: 10.1016/j.isci.2019.04.036 Retrieved from the Internet: URL:https://www.ncbi.nlm.nih.gov/pmc/artic les/PMC6538928/pdf/main.pdf
- MALI, P. et al.: "RNA-guided human genome engineering via Cas9", Science, vol. 339, no. 6121, 2013, pages 823-826, XP055469277,
- LATORRE, A. et al.: "Modified RNAs m CRISPR/Cas9: An Old Trick Works Again.", Angewandte Chemie International Edition, vol. 55, no. 11, 16 February 2016 (2016-02-16), pages 3548-3550, XP055602216,
- LOMBARDO, A. et al.: "Site-specific integration and tailoring of cassette design for sustainable gene transfer.", Nature Methods, vol. 8, no. 10, 2011, pages 861-869, XP055602217,
- CERBINI, T. et al.: "Transcription activator-like effector nuclease (TALEN)- mediated CLYBL targeting enables enhanced transgene expression and one-step generation of dual reporter human induced pluripotent stem cell (iPSC) and neural stem cell (NSC) lines", PLoS One, vol. 10, no. 1, 2015, page e0116032, XP055602218,
- BRESSAN, R. et al.: "Efficient CRISPR/Cas9-assisted gene targeting enables rapid and precise genetic manipulation of mammalian neural stem cells", Development, vol. 144, no. 4, 17 January 2017 (2017-01-17), pages 635-648, XP055602219,
- T. J. Cradick ET AL: "CRISPR/Cas9 systems targeting ?-globin and CCR5 genes have substantial off-target activity", Nucleic Acids Research, vol. 41, no. 20, 1 November 2013 (2013-11-01), pages 9584-9592, XP055186069, ISSN: 0305-1048, DOI: 10.1093/nar/gkt714
- Ishida Kentaro ET AL: "Minimizing off-Target Mutagenesis Risks Caused by Programmable Nucleases", International Journal of Molecular Sciences, vol. 16, no. 10, 1 January 2015 (2015-01-01), pages 24751-24771, XP55843526, DOI: 10.3390/ijms161024751 Retrieved from the Internet: URL:https://www.ncbi.nlm.nih.gov/pmc/artic les/PMC4632775/pdf/ijms-16-24751.pdf>

## Description

### TECHNICAL FIELD

The invention relates to methods for generating genetically modified neural stem cells.

### BACKGROUND

Genome editing with engineered nucleases is a breakthrough technology for modifying essentially any genomic sequence of interest (Porteus et al., Nature Biotechnology 23, 967-973 (2005)). This technology exploits engineered nucleases to generate site-specific double-strand breaks (DSBs) followed by resolution of DSBs by endogenous cellular repair mechanisms. The outcome can be either mutation of a specific site through mutagenic nonhomologous end-joining (NHEJ), creating insertions or deletions (in/dels) at the site of the break, or precise change of a genomic sequence through homologous recombination (HR) using an exogenously introduced donor template (Hendel et al., Trends in Biotechnology 33, 132-140 (2015)).

A recent major addition to this platform is the clustered regularly interspaced palindromic repeat (CRISPR)/Cas system consisting of an RNA-guided nuclease (Cas) and a short guide RNA (sgRNA) (Jinek et al., Science 337, 816-821 (2012); Mali et al., Science 339, 823-826 (2013); Cong et al., Science 339, 819-823 (2013); Hsu et al., Cell 157, 1262-1278 (2014)). The guide RNA is composed of two RNAs termed CRISPR RNA (crRNA) and transactivating crRNA (tracrRNA), which are typically fused in a chimeric single guide RNA (sgRNA). sgRNAs for genome editing can consist of 100 nucleotides (nt) of which 20 nt at the 5' end hybridize to a target DNA sequence by means of Watson-Crick base pairing and guide the Cas endonuclease to cleave the target genomic DNA.

Unfortunately, genome editing using the CRISPR/Cas system as well as other nuclease-mediated techniques remains inefficient, especially in primary cells such as human neural stem cells. As such, there remains a need in the art for compositions and methods based on genome editing that can be used for genetically modifying primary cells including human neural stem cells. Aspects of the disclosure satisfy this need and provides additional advantages.

Neural stem cells are discussed, for example, in US Application Publication Nos. 2001/0044122, 2003/0109039, 2004/0137535, US Patent No. 7037719, and Uchida et al., Proc Natl Acad Sci USA, 2000, 97(26):14720-14725*.*

### BRIEF SUMMARY

Aspects of the disclosure provides a method for generating a genetically modified (i.e., genome-edited) human neural stem cell, the method comprising: introducing into an isolated human neural stem cell: (a) a donor template comprising: (i) a transgene cassette comprising a transgene (for example, a transgene operably linked to a promoter, such as a heterologous promoter); and (ii) two nucleotide sequences comprising two non-overlapping, homologous portions of a safe harbor gene, wherein the nucleotide sequences are located at the 5' and 3' ends of the transgene cassette; and

(b) a DNA nuclease or a nucleotide sequence encoding the DNA nuclease, wherein the DNA nuclease is capable of creating a double-strand break in the safe harbor gene to induce insertion of the transgene into the safe harbor gene, thereby generating a genetically modified human neural stem cell.

Aspects of the disclosure also provides a genetically modified human neural stem cell produced by a method of the disclosure. Aspects of the disclosure also provides a genetically modified human neural stem cell comprising (i) a transgene cassette comprising a transgene (for example, a transgene operably linked to a promoter, such as a heterologous promoter); wherein the transgene cassette is located within a safe harbor gene. Also provided is a pharmaceutical composition comprising the genetically modified human neural stem cell of the disclosure and a pharmaceutically acceptable carrier.

Aspects of the disclosure also provides a method for preventing or treating a neurodegenerative disease or a neurological injury in a human subject in need thereof, the method comprising: administering to the human subject an effective amount of the pharmaceutical composition of the disclosure to prevent or alleviate one or more symptoms of the neurodegenerative disease or the neurological injury.

Also provided by the a certain aspect of the disclosure is a kit comprising: (a) a donor template comprising: (i) a transgene cassette comprising a transgene (for example, a transgene operably linked to a promoter, such as a heterologous promoter); and (ii) two nucleotide sequences comprising two non-overlapping, homologous portions of a safe harbor gene, wherein the nucleotide sequences are located at the 5' and 3' ends of the transgene cassette; (b) a DNA nuclease or a nucleotide sequence encoding the DNA nuclease; and (c) an isolated human neural stem cell.

Aspects of the disclosure also provides for the use of the genetically modified human neural stem cell of the disclosure or the genetically modified human neural stem cell produced by the method of the disclosure in a method of identifying or developing a potential therapeutic molecule. This may involve the screening of small molecules or biological molecules to identify a potential therapeutic molecule that acts on human neural stem cells or their differentiated progeny. In an one aspect of the disclosure, the potential therapeutic molecule is a molecule that promotes the proliferation and/or viability of a human neural stem cell and/or the differentiated progeny of a human neural stem cell.

Aspects of the disclosure also provides for the use of the genetically modified human neural stem cell of the disclosure or the genetically modified human neural stem cell produced by the method of the disclosure in research. Research applications include basic biological research into the fundamental biology of neural stem cells and applied research directed to potential therapies.

Other objects, features, and advantages of the disclosure will be apparent to one of skill in the art from the following detailed description and figures.

### BRIEF DESCRIPTION OF THE DRAWINGS

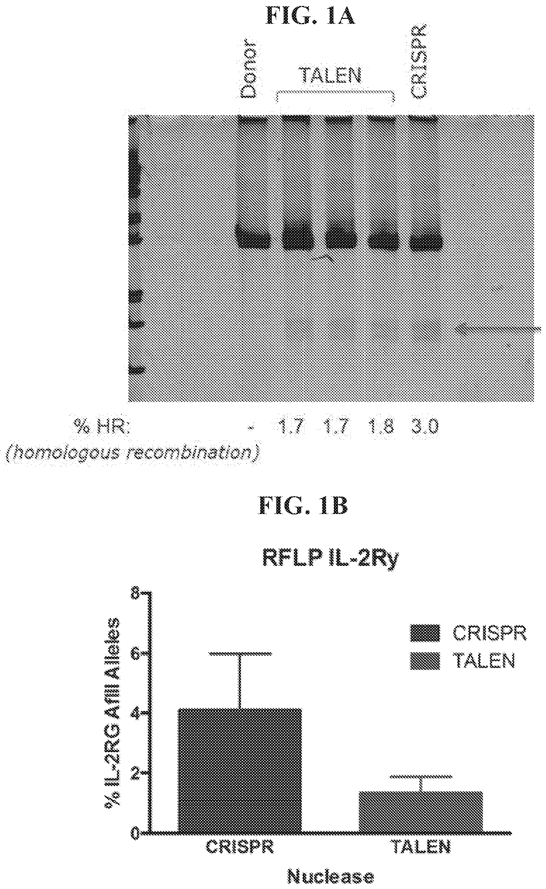

FIGS. 1A and 1B show that the GFP transgene was inserted into the IL2Ry locus via homologous recombination using engineered nucleases such as TALEN or CRISPR. FIG. 1A provides RFLP data. FIG. 1B shows that CRISPR/Cas9 stimulates a higher frequency of gene editing than TALENs at the IL2Ry locus.

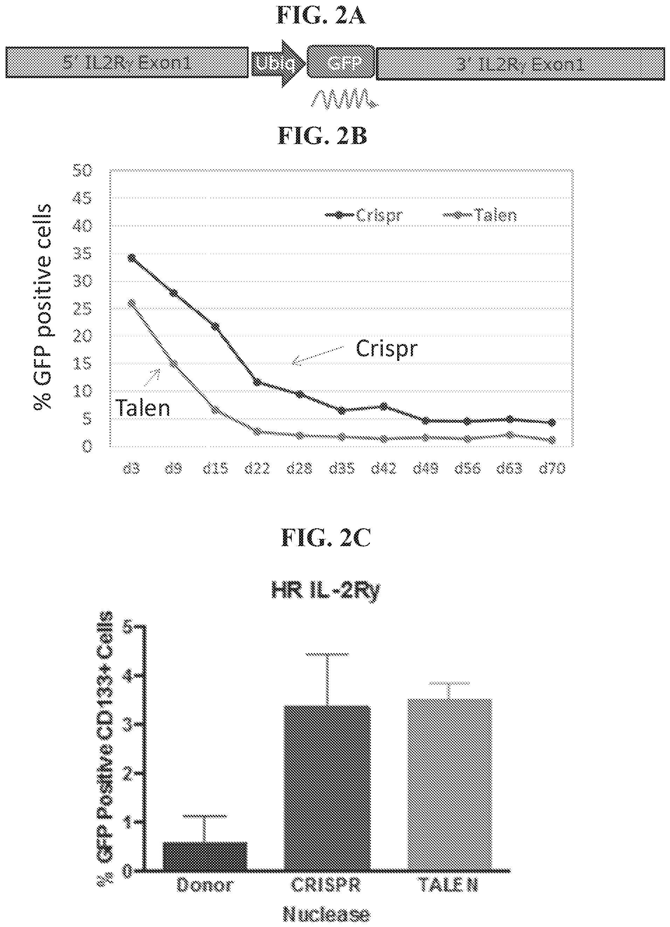

FIGS. 2A-2C show targeted integration of a GFP transgene into neural stem cells (NSCs) using engineered nucleases such as CRISPR/Cas9 and TALEN. FIG. 2A provides a scheme of an exemplary IL2Ry-GFP donor template. FIG. 2B shows stable expression of GFP⁺ cells in long term cultures post nucleofection. FIG. 2C shows that about 2-4% of the NSCs undergoing genome editing, as described herein were GFP positive and CD133 positive.

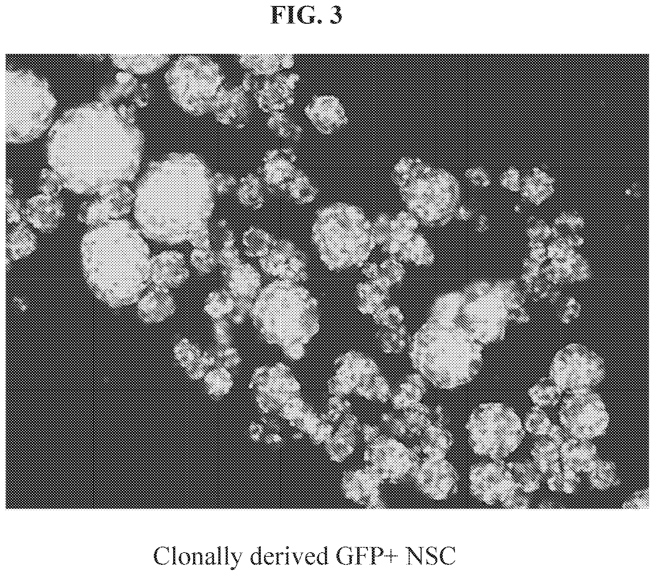

FIG. 3 depicts clonally derived GFP positive genetically modified NSCs (GM-NSCs).

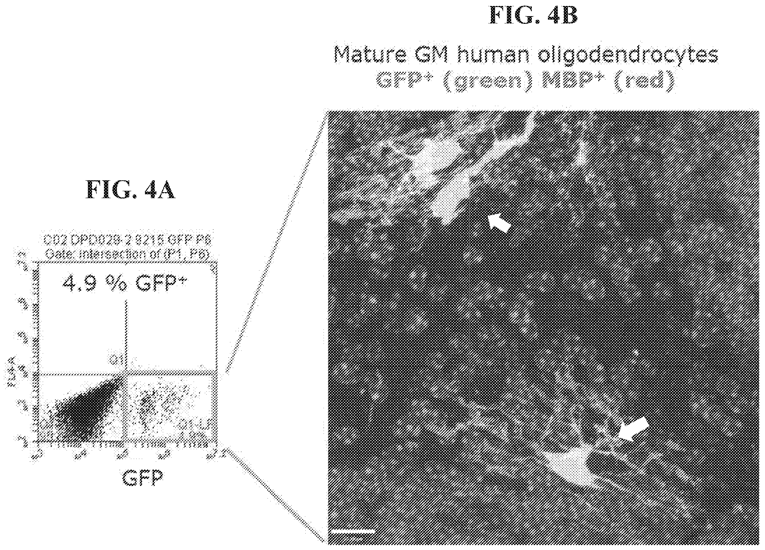

FIGS. 4A and 4B show oligodendrocytes derived from sorted and expanded GFP⁺ GM-NSCs. FIG. 4A shows GFP⁺ GM-NSCs. FIG. 4B shows oligodendrocytes derived from GM-NSCs expressing GFP and myelin basic protein (MBP). GM-NSCs were transplanted directly into the cerebellum of juvenile shi/shi-id mutant mouse brains. Brain sections were processed 8 week post transplantation. Higher magnification reveals that the progeny of human cells differentiate into mature myelinating oligodendrocytes with myelin sheat, indicating that differentiation potential is maintained following genome editing.

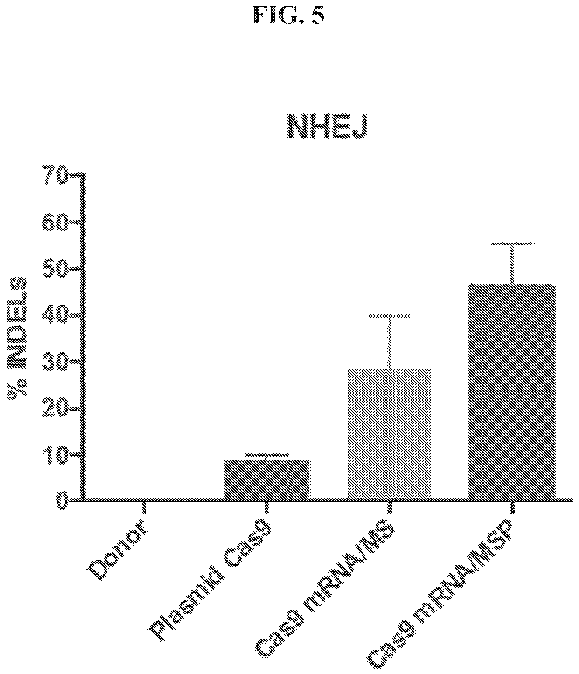

FIG. 5 illustrates the percentage of INDELs generated using a plasmid-based CRISPR/Cas9 system and RNA-based system with chemically modified sgRNAs targeted at the IL2Ry locus.

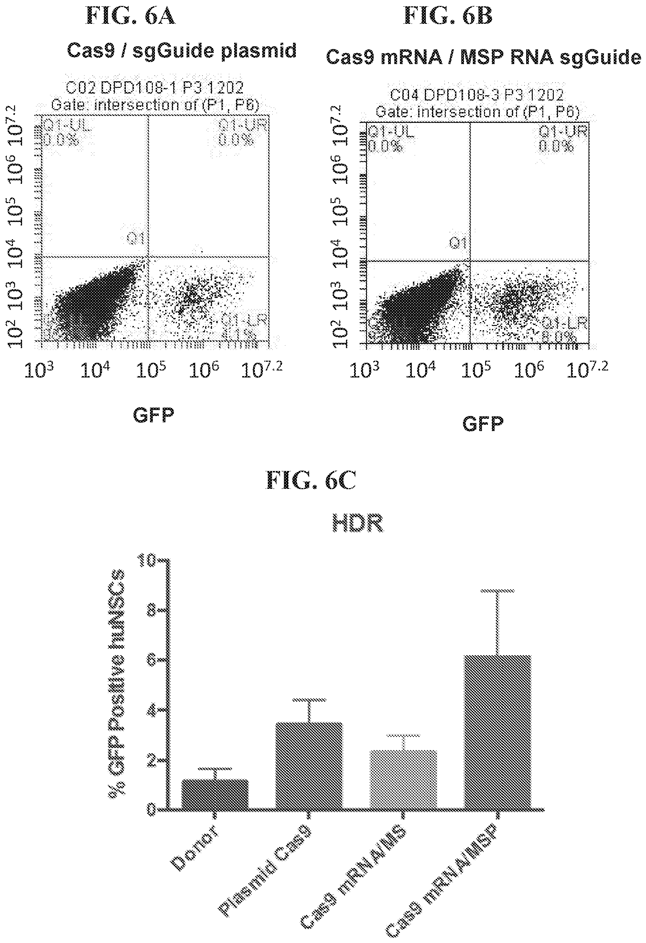

FIGS. 6A-6C show that delivery of the CRISPR/Cas9 platform using an RNA-based system with chemically modified sgRNAs yielded more GFP⁺ GM-NSCs than a plasmid-based system targeted at the IL2Ry locus. FIG. 6A shows the percentage of GFP⁺ cells generated using the plasmid-based system. FIG. 6B shows the percentage of GFP⁺ cells produced using the RNA-based system with chemically modified sgRNAs. FIG. 6C provides a comparison of the systems tested.

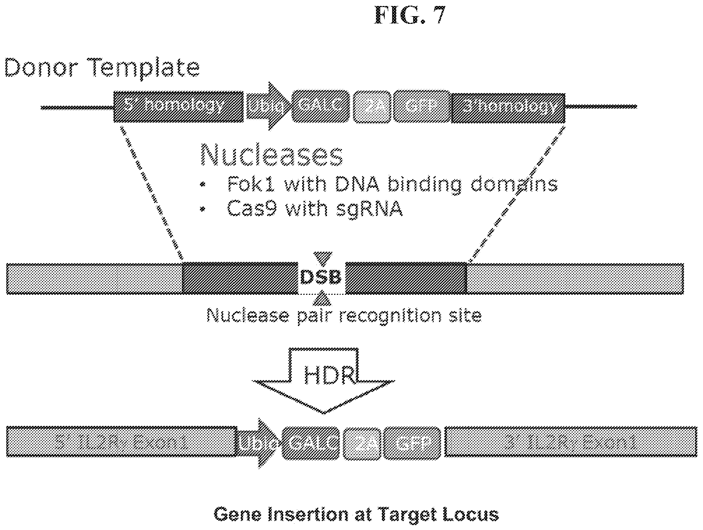

FIG. 7 provides a schematic diagram of an exemplary genome editing method described herein.

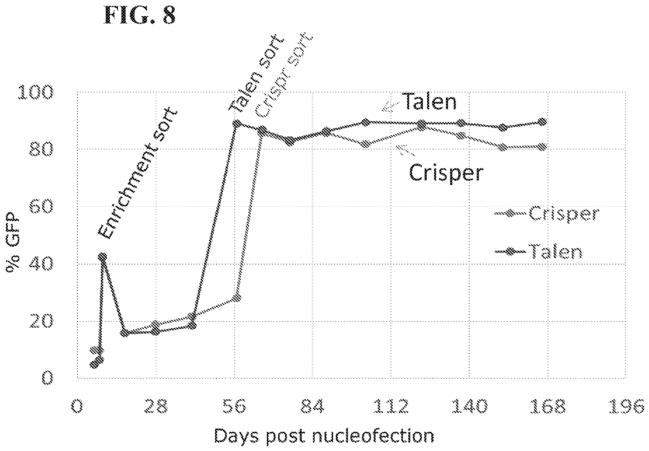

FIG. 8 shows stable expression of the UbC-GalC-2A-eGFP transgene in purified GM-NSCs upon long-term culture (about 168 days post nucleofection) targeted at the IL2Ry locus. GM-NSCs are enriched by FACS as indicated.

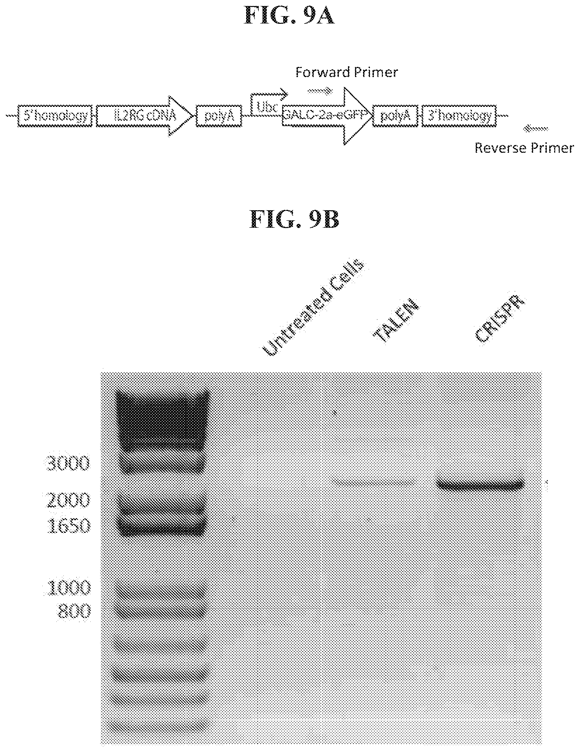

FIGS. 9A and 9B illustrates targeted integration of the UbC-GalC-2A-eGFP transgene into the safe harbor gene IL2Ry. FIG. 9A provides a scheme of an exemplary IL2Ry-UbC-GalC-2A-eGFP donor template. FIG. 9B shows PCR analysis of the targeted integration site. The expected amplicon size for targeted integration is 2355 bases.

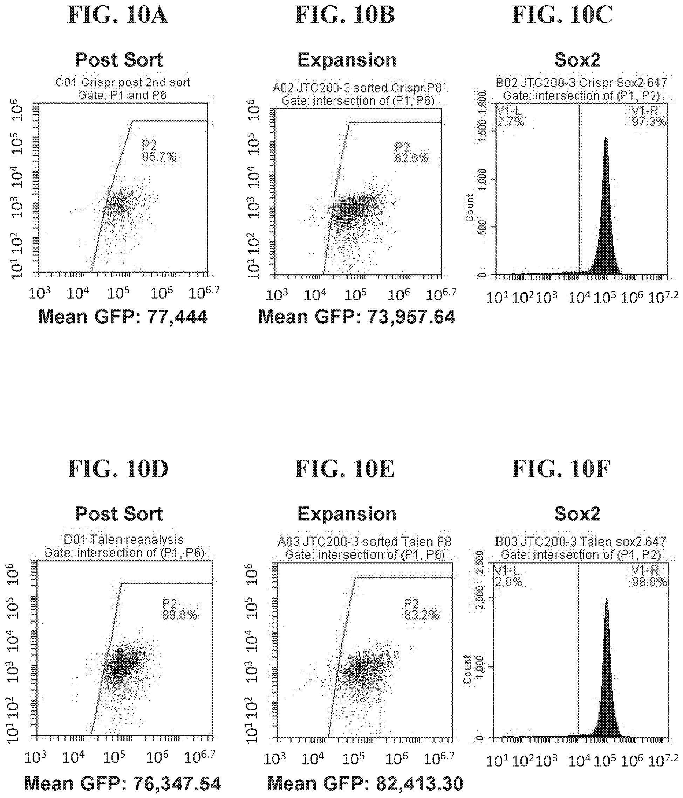

FIGS. 10A-10F show GM-NSCs carrying the UbC-GalC-2A-eGFP targeted transgene. The CRISPR modified NSCs were FACS purified to select GFP⁺ cells (FIG. 10A). The sorted GFP⁺ cells were expanded (FIG. 10B), and most were positive for the neural stem cell marker Sox2 (FIG. 10C). The TALEN modified NSCs were FACS purified to select GFP⁺ cells (FIG. 10D). The sorted GFP⁺ cells were expanded and remained GFP⁺ (FIG. 10D). Most of the expanded GFP⁺ cells were also positive for Sox2 (FIG. 10F).

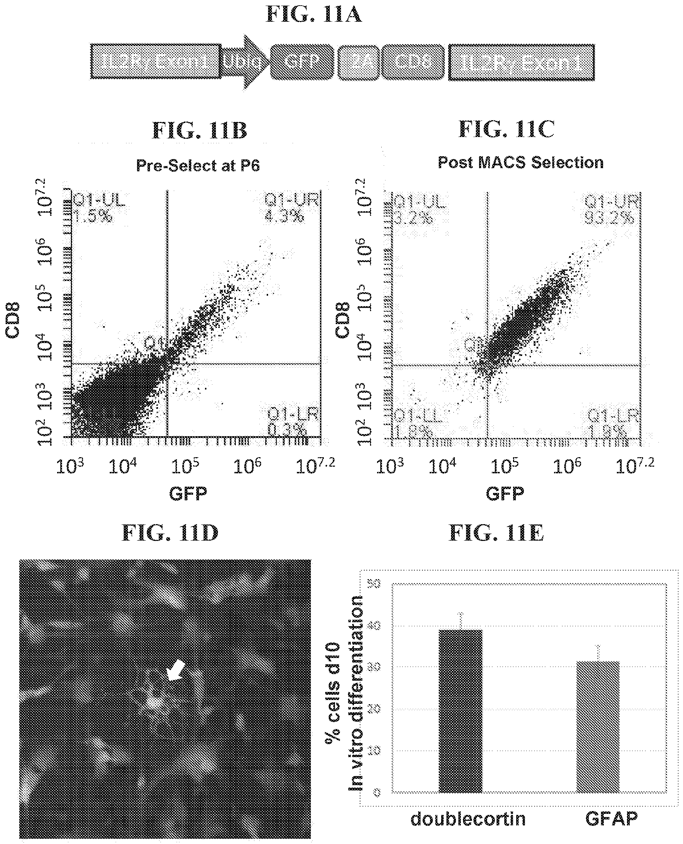

FIGS. 11A-11E illustrate the isolated and purification of CD8⁺GFP⁺ GM-NSCs. FIG. 11A provides a scheme of an exemplary IL2Ry-UbC-GFP-2A-CD8 donor template. FIG. 11B shows the percentage of CD8⁺ and GFP⁺ cells before selection at passage 6 after nucleofection. FIG. 11C shows the percentage of CD8⁺ and GFP⁺ cells after MACS CD8 selection. CD8⁺GFP⁺ GM-NSCs were differentiated *in vitro* into oligodendrocytes. FIG. 11D provides an image of a GM-NSCs cell-derived oligodendrocyte. FIG. 11E shows the percentages of cells that differentiation into neuronal cells (doublecortin) and astrocytes (GFAP).

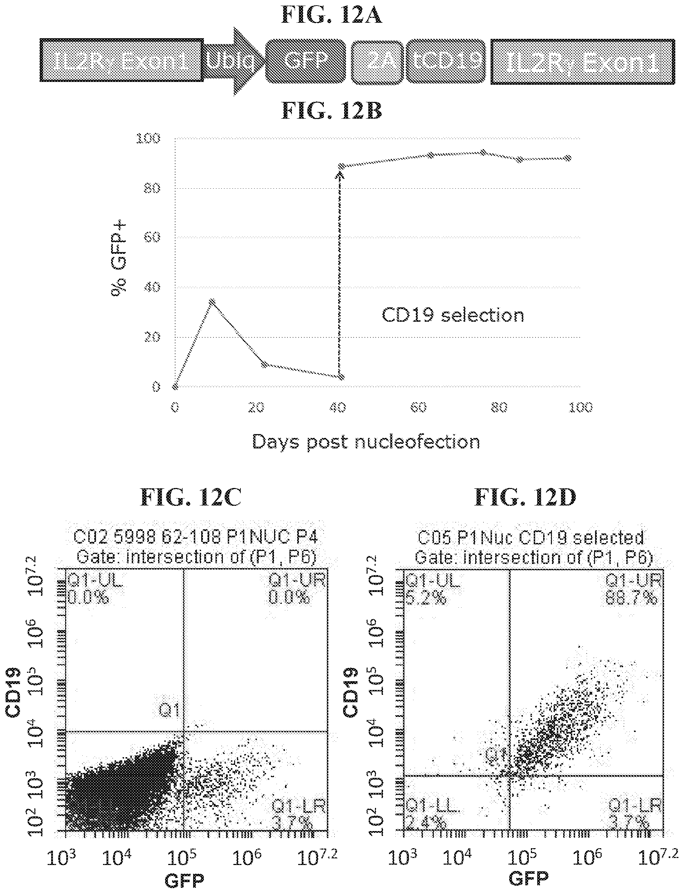

FIGS. 12A-12C illustrate the isolation and purification of CD19⁺GFP⁺ GM-NSCs. FIG. 12A provides a scheme of an exemplary IL2Ry-GFP-2A-truncated CD19 donor template. FIG. 12B shows the percentage of GFP⁺ cells after CD19 selection on day 40 post nucleofection. FIG. 12C shows that most cells were CD19-negative, GFP-negative before selection. FIG. 12D shows that most cells were CD19⁺ and GFP⁺ after MACS CD19 selection.

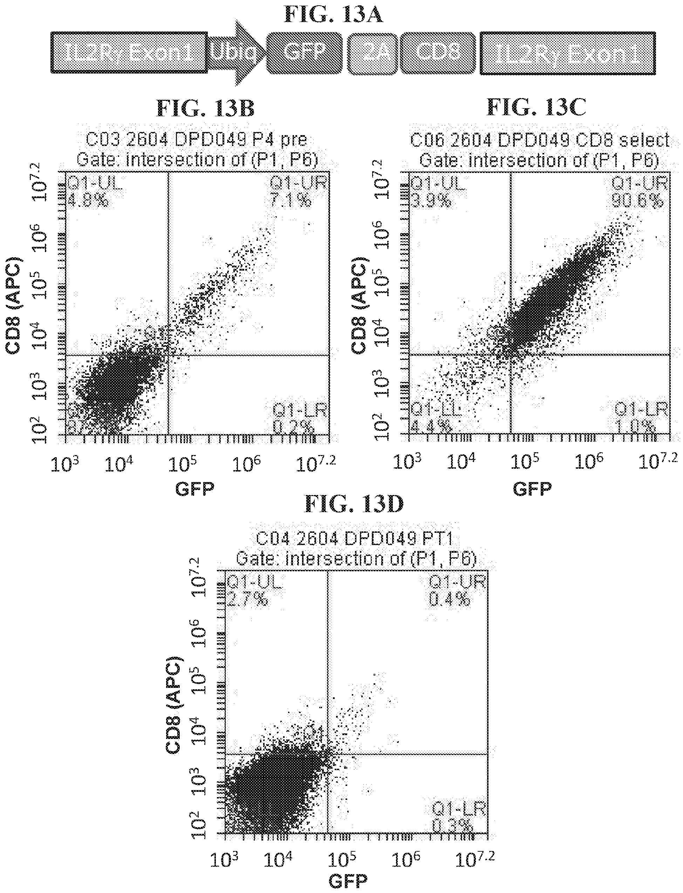

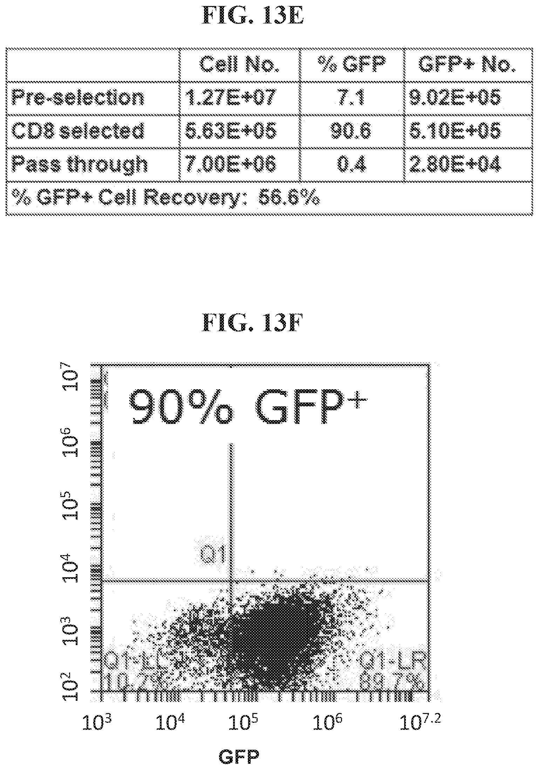

FIGS. 13A-13F show the isolation and purification of CD8⁺GFP⁺ GM-NSCs. FIG. 13A provides a scheme of an exemplary IL2Ry-UbC-GFP-2A-CD8 donor template. FIG. 13B shows the percentage of CD8⁺ and GFP⁺ cells before selection. FIG. 13C shows the percentage of CD8⁺ and GFP⁺ cells after selection. FIG. 13D shows the percentage of CD8-negative and GFP-negative cells that passed through the MACS CD8 selection column. FIG. 13E shows that a high percentage of the CD8 selected cells are also GFP+. FIG. 13F shows that 90% of the CD8-selected GM-NSCs are GFP⁺.

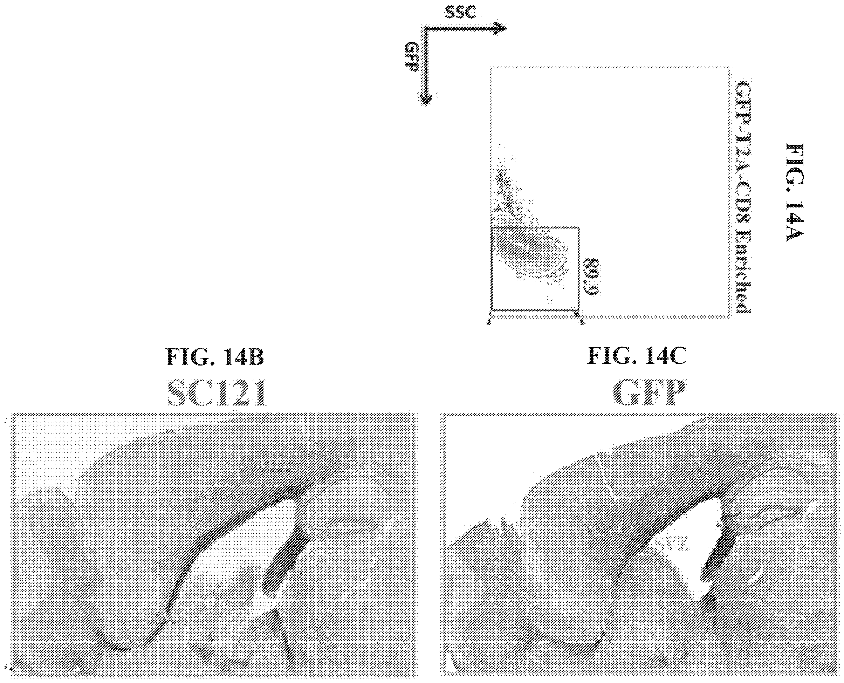

FIGS. 14A-14C show engraftment, migration, and differentiation of GM-NSCs into myelin producing oligodendrocytes after transplantation into an oligodendrocyte mutant shiverer mouse. NSCs were targeted with a bicistronic IL2RG HR cassette was created that separated GFP from CD8. FIG. 14A shows GM-NSCs were MACS selected with CD8 microbeads. GM-NSCs were then transplanted into 8 week old juvenile shiverer-id mice. Cells were transplanted bilaterally that targeted the subventricualr zone (SVZ). FIG. 14B shows a section of a mouse brain stained with a human specific mAb SC121. SC121 staining at 12 weeks post-transplant demonstrated extensive migration of GM-NSCs in the RMS to olfactory bulb and white matter tracts included the corpus callosum, fimbria of the fornix, and those in the cerebellum. FIG. 14C shows a section of a mouse brain stained with an anti-GFP antibody. The transplanted GM-NSCs continued to express the GFP transgene in the mouse. The robust GFP transgene expression is similar to SC121 staining. Sagittal brain sections show that the grafted cells are present in the SVZ. In addition, migrating human cells are detected in the rostral migratory stream (RMS), corpus collusum, and in the cortex.

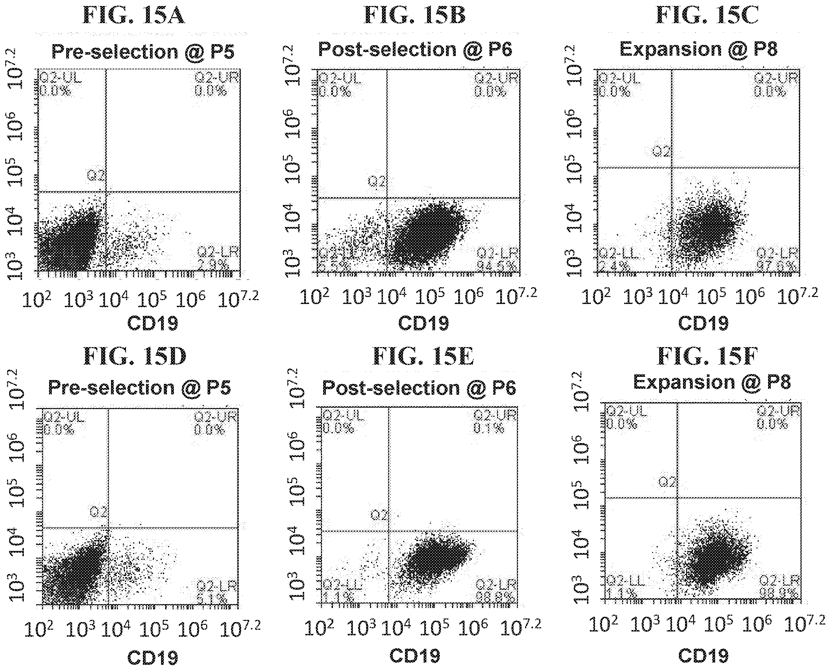

FIGS. 15A-15F show CD19⁺ GM-NSCs carrying the UbC-GalC-T2A-tCD19 transgene targeted at the IL2Rγ locus. GM-NSCs expressing the cell surface marker CD19 were purified using MACS CD19 selection microbeads. Flow cytometry of GM-NSCs generated using a plasmid-based sgRNA/CRISPR nuclease are shown in FIGS. 15A-15C (pre-selection - FIG. 15A; post-selection - FIG. 15B; expansion - FIG. 15C). Flow cytometry of GM-NSCs generated using a MSP sgRNA with a Cas9 mRNA are shown in FIGS. 15D-15F (pre-selection - FIG. 15D; post-selection - FIG. 15E; expansion - FIG. 15F).

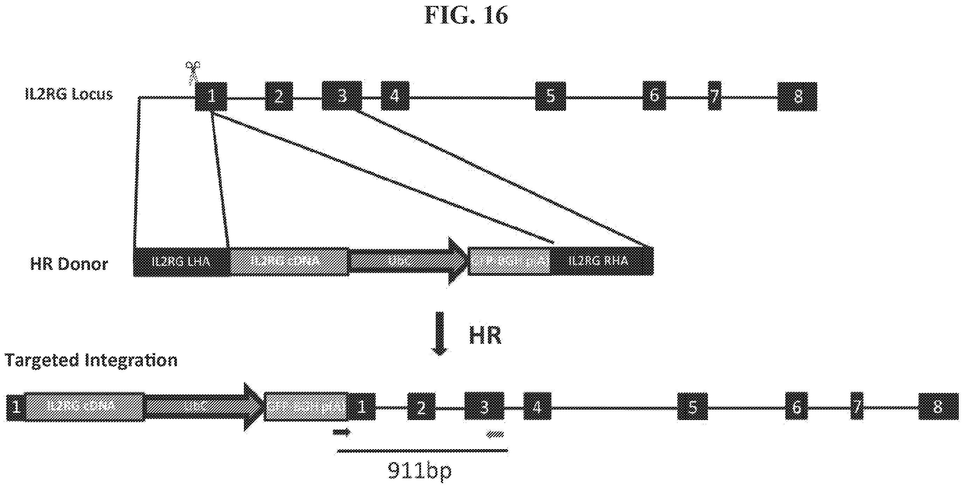

FIG. 16 shows CRISPR/Cas9-mediated homologous recombination (HR) targeting IL2RG locus in NSC. The IL2RG locus was targeted for homologous recombination (HR) by creating double strand breaks (DSBs) using Cas9 (scissors) and supplying a homologous donor template (with flanking 800bp arms around the transgene to be inserted). Following HR, IL2RG cDNA is knocked in to the endogenous start codon with a UbC promoter driving GFP downstream to assess efficiencies of HR in human NSCs. IL2RG exons are shown in black boxes.

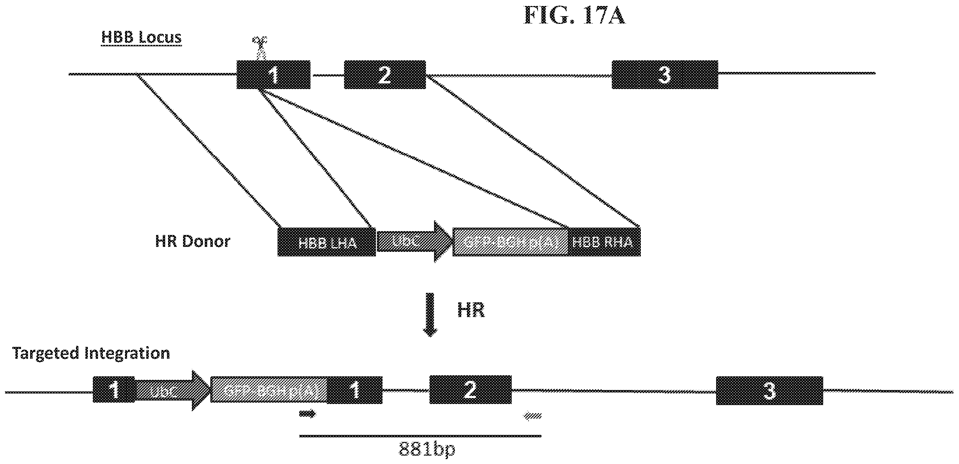

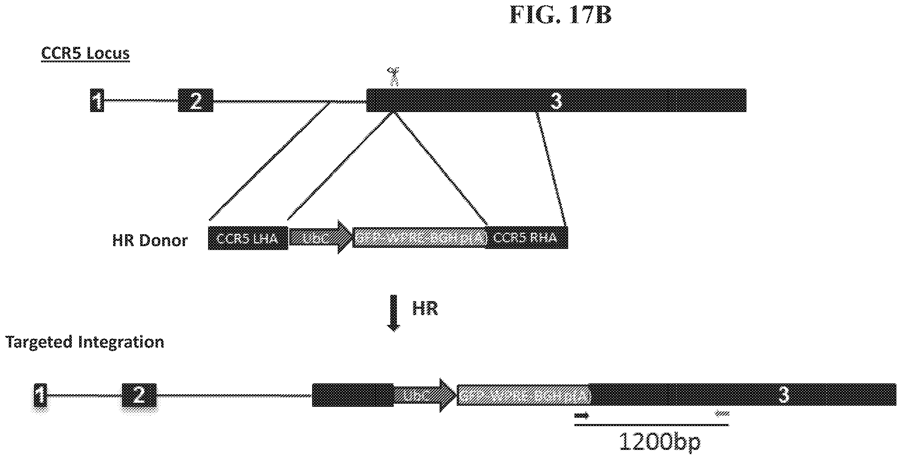

FIGS. 17A-17B show CRISPR/Cas9-mediated homologous recombination (HR) targeting *HBB* locus (FIG. 17A) and *CCR5* locus (FIG. 17B) in NSCs. FIG. 17A shows that the *HBB* locus was targeted by creating a DSB in exon 1 via Cas9 (scissors) and supplying a UbC-GFP -homologous donor template. Alleles with integrations were identified by PCR (881bp) using an In (black) - Out (red) primer set. FIG. 17B shows that the *CCR5* locus was targeted in exon 3 as described above. PCR identified integrated (1200bp) alleles using In (black) - Out (red). Loading control for all In-Out PCRs was the wildtype *CCR5* allele.

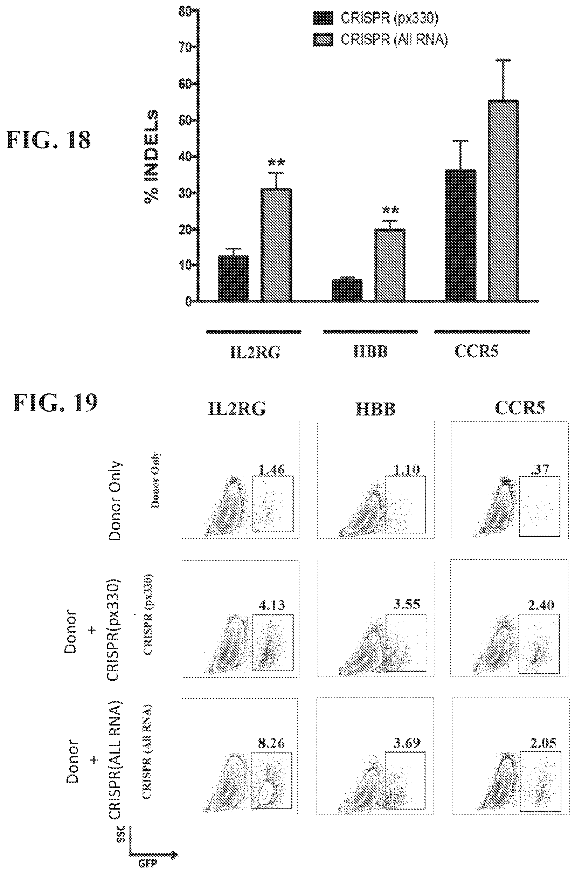

FIG. 18 shows Cas9 as mRNA and chemically modified sgRNAs induced more INDELs than plasmid delivery at all three loci tested. NSCs were electroporated with 1ug plasmid (px330) encoding Cas9 and sgRNA specific for *IL2RG, HBB,* or *CCR5.* For "All RNA" experiments, Cas9 was delivered as mRNA (5ug) and the locus-specific sgRNAs (10ug) were delivered with terminal chemical modifications comprising 2'-O-methyl 3'phosphorothioate (MS), or 2'-O-methyl 3'thioPACE (MSP). Following 7 days in culture, gDNA was harvested, PCR was carried out to amplify the region of interest, and INDELs were calculated using the Tracking of Indels by Decomposition (TIDE) software. (N=2-14), ^{∗∗} p < 0.01, Student's T-test.
FIG. 19 shows all 3 loci were amenable to HR in NSCs using plasmid or All RNA delivery of CRISPR-Cas9 components. NSCs were nucleofected as described above with either 2ug HR donor or 2ug HR and locus-specific CRISPR components. Locus-specific HR donor plasmids have at least 400bp of homology flanking UbC-GFP to assess HR efficiencies. Cells were grown for at least 30 days until episomal HR donor was lost by dilution during proliferation to confirm stable expression of integrated cassettes into the *IL2RG, HBB,* and *CCR5* loci. Representative FACS plots are shown, which highlight stable integration of HR donors with site-specific nucleases.

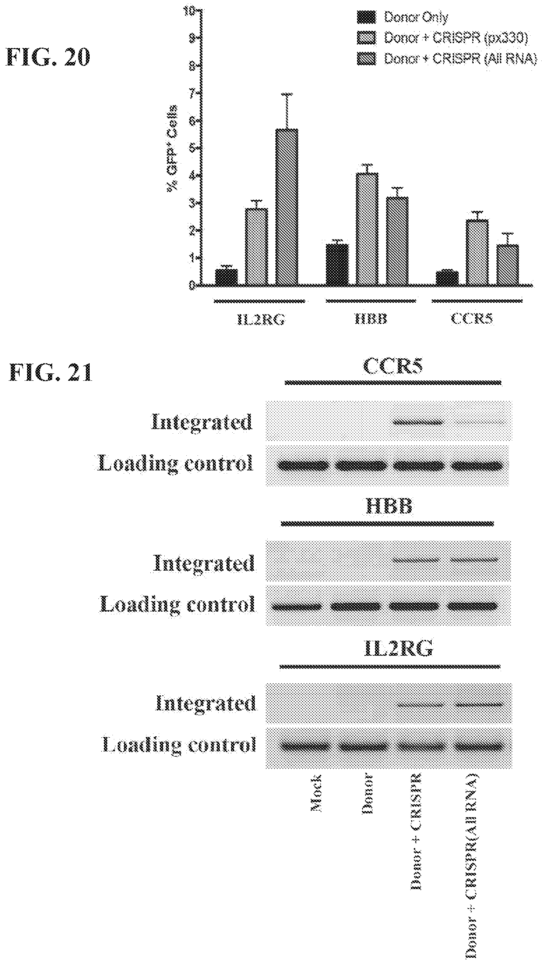

FIG. 20 shows targeting NSCs at *IL2RG, HBB,* and *CCR5* loci with plasmid delivery of CRISPR, resulted in an average of 2.8%, 4.1%, and 2.4% GFP⁺ NSCs after at least 30 days in culture, respectively. HR frequencies obtained from all experiments targeting *IL2RG, HBB,* and *CCR5* carried out as above. (N=3-7), ^{∗} p < 0.05, Student's T-test.
FIG. 21 shows while PCR amplification was not detected with mock or donor only samples, on-target integration was evident when NSCs were co-electroporated with CRISPR and a homologous donor, confirming HR at the intended locus. gDNA was harvested from experimental groups at day 30 post culture to evaluate site-specific integration of HR donors on the DNA level. Agarose gel electrophoresis of In/Out PCR products confirms on-target integration of HR donors in the presence of a site-specific nuclease.

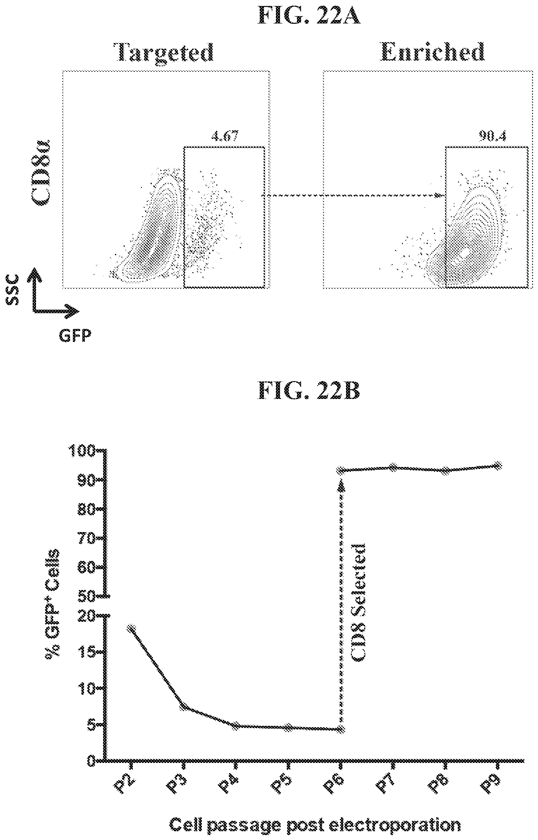

FIGS. 22A-22B show enrichment of GM-NSCs by magnetic activated cell sorting (MACS) selection with CD8α. A bicistronic *IL2RG* HR cassette was created that separate GFP from CD8α via a T2A peptide motif to allow robust magnetic bead enrichment of IL2RG-targeted NSCs. NSCs were nucleofected with 2ug HR donor and 1ug plasmid encoding Cas9 and sgRNA. Cells were grown for 30 days to allow episomal HR donor to dilute out during proliferation of NSCs. Cells were selected using CD8 microbead technologies at passage 6-post targeting. Representative FACS plots (FIG. 22A) show a population of NSCs with stable integration of the bicistronic GFP-T2A-CD8 cassette before enrichment at day 30 after electroporation (left), and 30 days after enrichment using Magnetic Activated Cell Sorting (MACS) CD8 Microbead technologies (right). GM-NSCs were cultured for nine passages post electroporation while being analysed for GFP expression at every cell passage. (FIG. 22B)

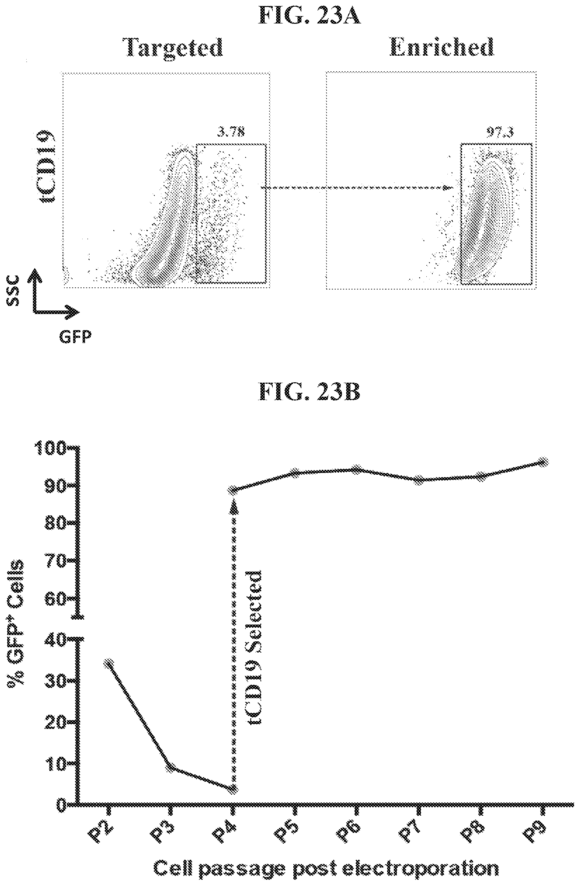

FIGS. 23A-23B show enrichment of GM-NSCs by magnetic activated cell sorting (MACS) selection with truncated CD19 (tCD19). A bicistronic IL2RG HR cassette was created that separate GFP from truncated CD19 (tCD19) via a T2A peptide motif to allow robust magnetic bead enrichment of IL2RG-targeted NSCs. NSCs were nucleofected with 2ug HR donor and 1ug plasmid encoding Cas9 and sgRNA. Cells were grown for 30 days to allow episomal HR donor to dilute out during proliferation of NSCs. Representative FACS plots (FIG. 23A) showing a population of NSCs with stable integration of the bicistronic GFP-T2A-tCD19 cassette before enrichment at day 30 after electroporation (left), and a population enriched of GM-NSCs using MACS CD19 Microbead technologies (right) GM-NSCs were cultured for 9 passages while being analysed for GFP expression. (FIG. 23B)

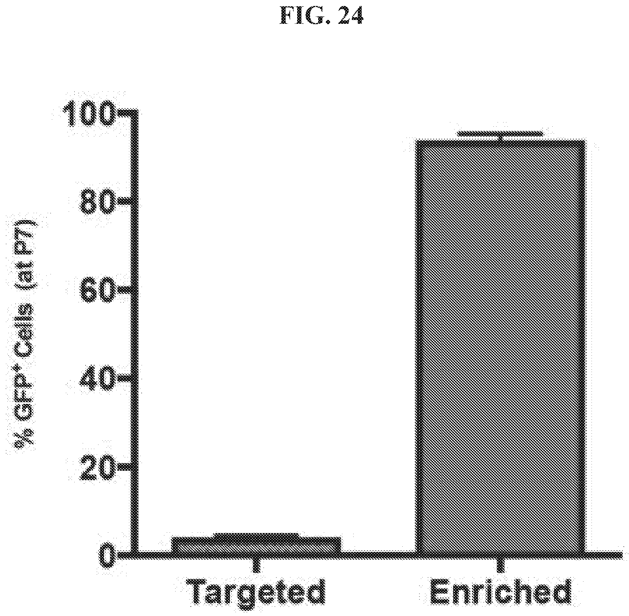

FIG. 24 shows frequencies of GM-NSCs targeted at IL2Ry locus using the bicistronic donor cassettes before (targeted) and after MACS selection (enriched). N=3.

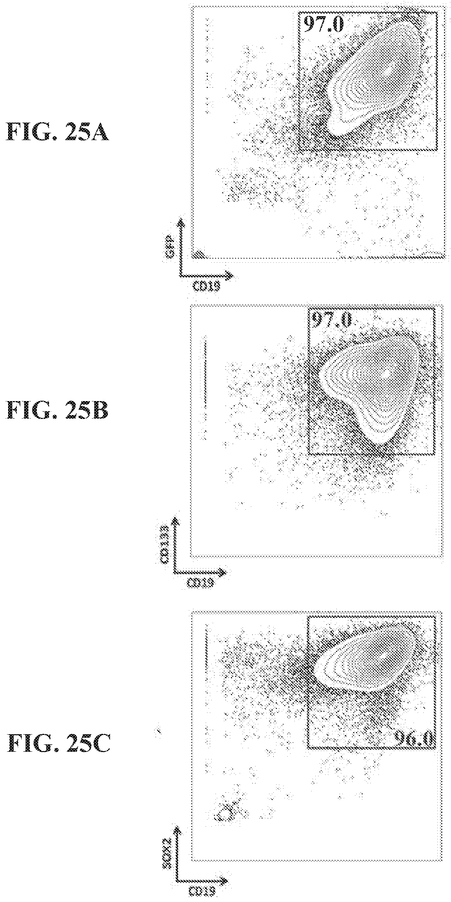

FIGS. 25A-25C show GM-NSCs maintained their NSCs characteristics. Expanded/MACS enriched GM-NSCs with bicstronic GFP-T2a-tCD19 cassette were stained for the cell surface markers, CD133 (PE) and CD19 (APC) and analysed by FACS CD19 vs GFP (FIG. 25A), CD19 vs CD133 (FIG. 25B), alternatively, cells were stained for CD19 (APC), permeabilised, fixed and then stained again for SOX2 (PE) (FIG. 25C).

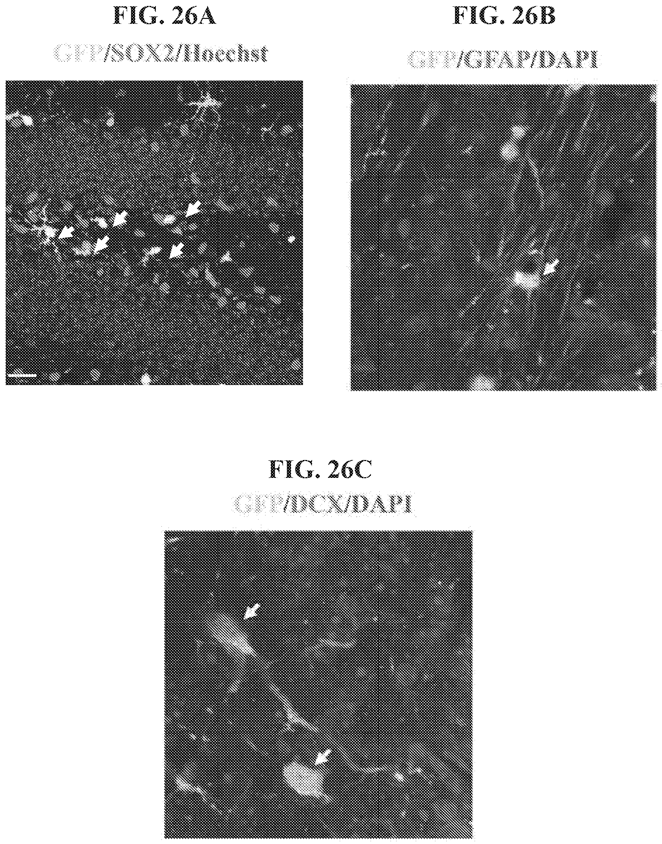

FIGS. 26A-26C show GM-NSCs maintain migration, NSCs marker and tri-lineage differentiation potential *in vivo* in site appropriate manner. NSCs were targeted with a bicistronic IL2Ry HR cassette (GFP-T2A-CD8), then MACS-selected with CD8 microbeads, and transplanted bilaterally targeted the SVZ of neonatal shi/shi-id or shi/+ heterozygous id mouse brains. Confocal images (FIGS. 26A-26C) of the dentate gyrus of the hippocampus stained with anti-GFP (green) and anti-Sox2 (red) Hoechst 33345 counter staining (blue) revealing that the GM-NSCs migrated and maintain NSC marker, Sox 2 (arrows) (FIG. 26A), anti-GFP (green), anti-human GFAP, SC123 (red) and DAPI counter staining (blue) in the white tract bundle of the striatum (FIG. 26B), anti-GFP (green), anti-Doublecortin (DCX), SC123 (red) and DAPI counter staining (blue) in the olfactory bulb (FIG. 26C).

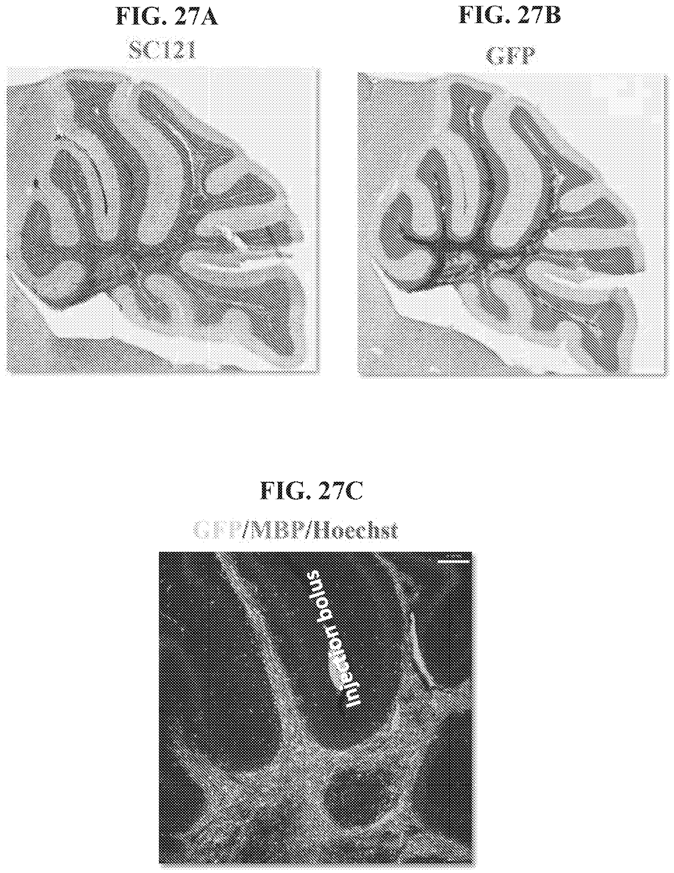

FIGS. 27A-27C show oligodendrocyte differentiation potential of GM-NSCs. GM-NSCs were transplanted directly into the cerebellum of juvenile shi/shi-id mutant mouse brains. Brain sections were processed 8 week post transplantation. Immunostaining with human-specific SC121 mAbs at 8 wks post-transplant demonstrated extensive migration of GM-NSCs within white matter tracts of the cerebellum (FIG. 27A). Immunoperoxidase staining of a sibling section with a anti-GFP reveals a similar distribution of transgene expression as SC121 staining (FIG. 27B). Confocal images of the white tract of cerebellum with anti-GFP (green) and anti-MBP (red) demonstrate continuous GFP expression and myelin production (FIG. 27C). Injection site is indicated as shown.

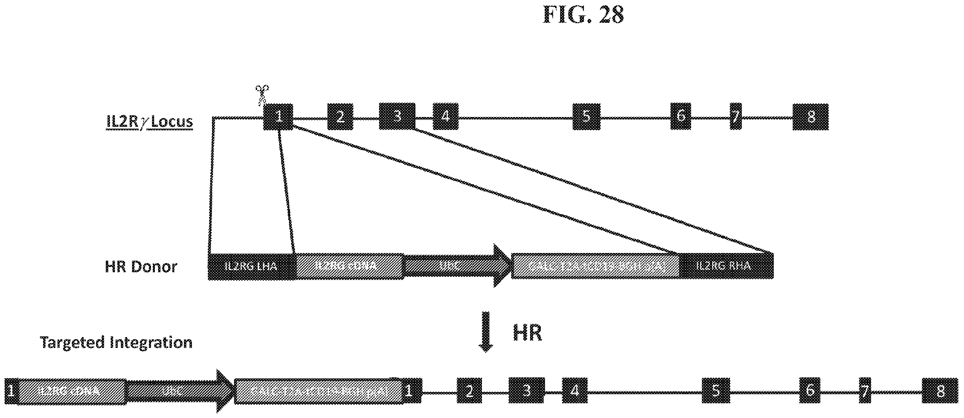

FIG. 28 shows using a HR donor to knock-in *GALC* (*IL2RG* cDNA-UbC-GALC-T2A-tCD19) into the IL2Rylocus and also a tCD19 selection cassette to enable robust MACS-based enrichment. The IL2Rylocus was targeted for homologous recombination (HR) by creating double strand breaks (DSBs) using Cas9 (scissors) and supplying a homologous donor template (with flanking 800bp arms around the transgene to be inserted). Following HR, *IL2Rγ*cDNA is knocked in to the endogenous start codon followed by a UbC-driven cassette with GALC-T2A-tCD19 for overexpression of GalC enzyme and enrichment of targeted cells using CD19 MACS selection.

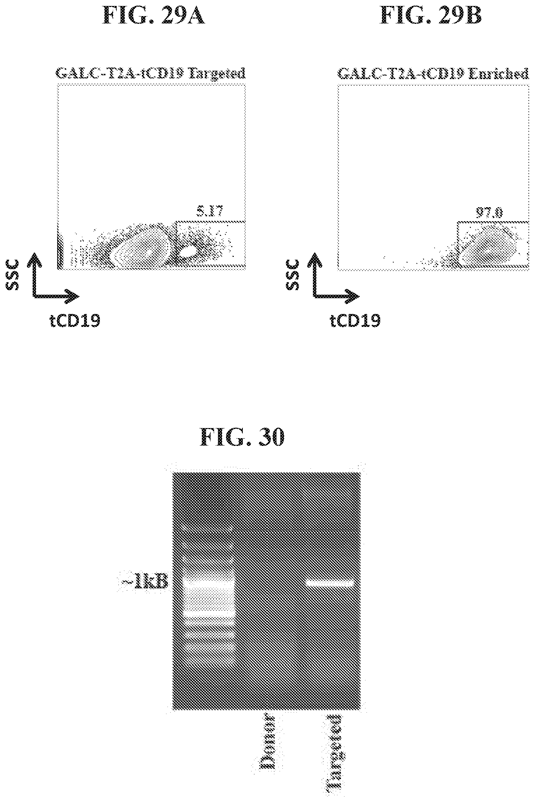

FIGS. 29A-29B show stably integrated *GALC* construct. NSCs were nucleofected as described previously with Cas9 mRNA, MSP *IL2Rγ* sgRNA, and the GALC-T2A-tCD19 HR donor construct. Representative FACS plots highlight stably integrated *GALC* construct as measured by tCD19 expression at 30 days post-nucleofection before (FIG. 29A) and after (FIG. 29B) enrichment of tCD19⁺ cells.
FIG. 30 shows on-target integration of the *GALC* cassette into the *IL2Rγ* locus. gDNA was harvested from experimental groups at day 30 post electroporation to evaluate integration of the GALC donor on the DNA level. Agarose gel electrophoresis of In/Out PCR products show on-target integration of the HR donor in the presence of a site-specific nuclease.

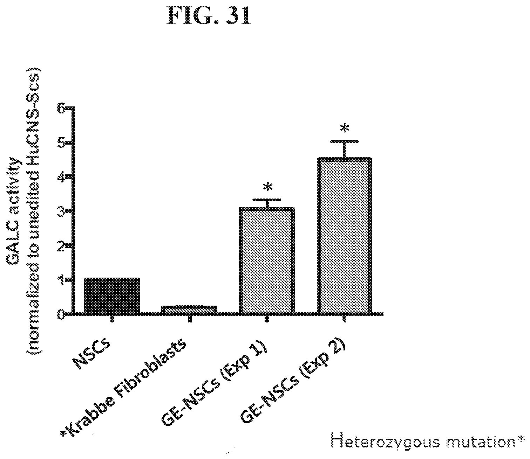

FIG. 31 shows *GALC* NSCs were overexpressing functional enzyme. GalC enzyme assay was performed on cellular protein lysates from the following experimental groups: unedited NSCs, Krabbe disease patient fibroblasts, GALC GM-NSCs (two different experiments). GalC enzyme activity is presented as pmol/min/mg (normalised to unedited NSCs). N=3, two independent biological experiments, ^{∗} p < 0.05, Student's T-test.

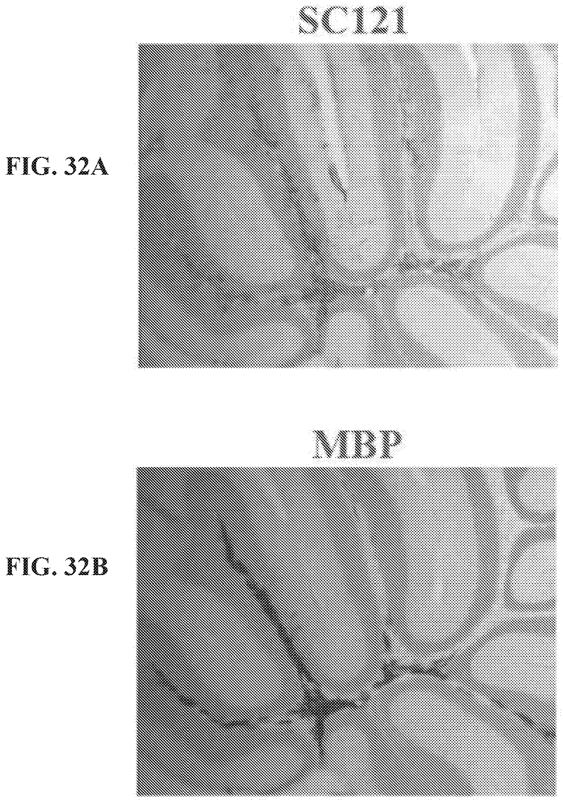

FIGS. 32A-32B show *GALC* GM-NSCs retained their NSC biological characteristics. Immunoperoxidase staining with the human-specific mAb SC121 (brown) detects engraftment of human cells in the white matter in the cerebellum (FIG. 32A). Immunoperoxidase staining of a sibling section with anti-MBP (brown) reveals a similar distribution of grafted GM-NSCs (FIG. 32B).

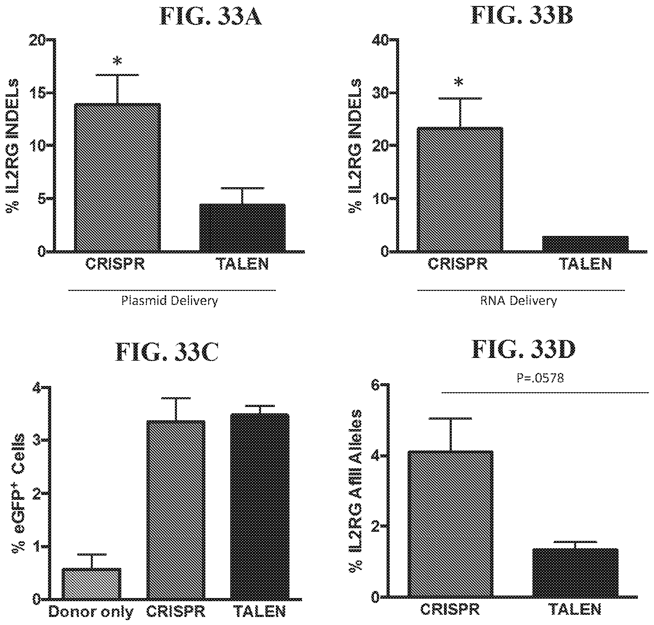

FIGS. 33A-33D show comparison between CRISPR and Talen system. 500,000 NSCs were electroporated (plasmid delivery) with either the CRISPR/Cas9 system or TALEN pairs that were designed to recognize the human *IL2RG* locus. Seven days post targeting, gDNA was harvested, *IL2RG* alleles were amplified by PCR with primers that overlapped the cut site, and TIDE was run to analyze INDEL frequencies (FIG. 33A). (N= 4-7), ^{∗} p < 0.05, Student's T-test. NSCs were electroporated as described above with Cas9 mRNA and MSP sgRNA or TALEN pairs delivered as mRNA, and then *IL2RG* alleles were analysed by TIDE software for INDELs (FIG. 33B). (N= 2-5), ^{∗} p < 0.05, Student's T-test. NSCs were electroporated with *IL2RG* engineered nucleases along with UbC-GFP donor templates with *IL2RG* homology arms. 30 days post-targeting, cells were harvested for FACS GFP analyses (FIG. 33C). (N= 4-6). NSCs were electroporated with *IL2RG* engineered nucleases along with a *IL2RG* homologous donor template intended to introduce a AfIII restriction site following homologous recombination. 7 days post-targeting, gDNA was harvested, then *IL2RG* alleles were amplified to produce a 1.6kb product. Amplified alleles were digested with AfIII and run a PAGE gel. The number of HR alleles were calculated as follows: ((digested alleles/ digested alleles) + undigested alleles). (FIG. 33D) (N= 4-6), Student's T-test.

### DETAILED DESCRIPTION

### I. Introduction

The invention is defined by the claims. This disclosure is based, in part, on the discovery that genome editing technologies enhance the therapeutic potential of human neural stem cells. Neural stem cells (NSCs) have therapeutic potential for unmet medical needs for neurological disorders with one-time intervention with a life-long impact. For example, HuCNS-SC^{®} cells (StemCells, Inc.) are a highly purified fetal brain-derived human central nervous system stem cell population that have biological NSC activities with multiple mechanism of actions, providing neuroprotection, myelination, and retinal preservation via global migration and site-appropriate differentiation into mature neurons, astrocytes and myelin producing oligodendrocytes. NSCs have been tested in four Phase I/II clinical trials with promising outcomes for safety, donor cell survival and/or preliminary efficacy. The recent advances in genome editing technologies, namely the TALEN and CRISPR/Cas9 platforms, have accelerated opportunities for producing genetically modified (GM) cells for cell therapy, which ultimately broadens the therapeutic potential of NSCs, *e.g.,* by serving as mini-factories of neuroprotective proteins to overcome the blood brain barrier. NSCs and methods of isolating NSCs are described, e.g., in US Patent Application Publication Nos. 2001/0044122, 2003/0109039, and 2004/0137535 A1; and US Patent No. 7,037,719.

As described herein, the inventors have targeted "safe harbor loci" IL2Rγ, and HBB, in NSC for homologous recombination (HR) of a GFP cassette by inducing site-specific double-strand breaks (DSBs), along with homologous plasmid donor templates. GM-NSCs displayed robust long-term GFP expression, and importantly, genomic analysis by in-out PCR confirmed on-target integration. Truncated CD19 enabled the purification of the GM-NSCs population at >90%. Furthermore, delivering the CRISPR platform as an "all RNA" system with chemically modified sgRNAs significantly improved the frequency of DSBs, which subsequently improved HR targeting rates two-fold over plasmid delivery of CRISPR. Most importantly, transplantation of GM-NSCs into oligodendrocyte mutant shiver mice showed that GM-NSCs migrate and differentiate into myelin producing oligodendrocytes, comparable to non-genetically modified-NSCs, indicating that GM-NSCs retain their NSC characteristics. The self-renewal and global migration properties of the GM-NSCs indicate that GM-NSCs could serve as mini-factories for the continuous delivery of proteins to broaden treatment options since these proteins cannot cross the blood-brain barrier. In conclusion, the success of the studies described herein provide methods, compositions, and kits for transplantable GM-NSCs that enable the treatment of a battery of neurodegenerative disorders and injuries for the brain, spinal cord, and eye.

### II. General

Practicing aspects of the disclosure utilizes routine techniques in the field of molecular biology. Basic texts disclosing these general methods use in this invention include Sambrook and Russell, Molecular Cloning, A Laboratory Manual (3rd ed. 2001); Kriegler, Gene Transfer and Expression: A Laboratory Manual (1990); and Current Protocols in Molecular Biology (Ausubel et al., eds., 1994)).

For nucleic acids, sizes are given in either kilobases (kb), base pairs (bp), or nucleotides (nt). Sizes of single-stranded DNA and/or RNA can be given in nucleotides. These are estimates derived from agarose or acrylamide gel electrophoresis, from sequenced nucleic acids, or from published DNA sequences. For proteins, sizes are given in kilodaltons (kDa) or amino acid residue numbers. Protein sizes are estimated from gel electrophoresis, from sequenced proteins, from derived amino acid sequences, or from published protein sequences.

Oligonucleotides that are not commercially available can be chemically synthesised, *e*.*g*.*,* according to the solid phase phosphoramidite triester method first described by Beaucage and Caruthers, Tetrahedron Lett. 22:1859-1862 (1981), using an automated synthesiser, as described in Van Devanter et. al., Nucleic Acids Res. 12:6159-6168 (1984). Purification of oligonucleotides is performed using any art-recognised strategy, *e*.*g*., native acrylamide gel electrophoresis or anion-exchange high performance liquid chromatography (HPLC) as described in Pearson and Reanier, J. Chrom. 255: 137-149 (1983).

### III. Definitions

.Unless specifically indicated otherwise, all technical and scientific terms used herein have the same meaning as commonly understood by those of ordinary skill in the art to which this disclosure belongs. In addition, any method or material similar or equivalent to a method or material described herein can be used in the practice of specific aspects of the disclosure. For the purposes of the disclosure, the following terms are defined.

The terms "a," "an," or "the" as used herein not only include aspects with one member, but also include aspects with more than one member. For instance, the singular forms "a," "an," and "the" include plural referents unless the context clearly dictates otherwise. Thus, for example, reference to "a cell" includes a plurality of such cells and reference to "the agent" includes reference to one or more agents known to those skilled in the art, and so forth.

The term "human neural stem cell" refers to a cell of the neural lineage with self-renewal and multilineage differentiation properties obtained from a human source or derived from a cell of human origin. Similarly, the term "human neural stem cells" refers to a purified cell population with self-renewal and multilineage differentiation properties. Neural stem cells are capable of dividing either symmetrically, or asymmetrically. When dividing symmetrically, the neural stem cell divides to form two daughter neural stem cells or two committed progenitors, though unless otherwise specified symmetrical division refers herein to symmetrical self-renewal; when dividing asymmetrically, the neural stem cell divides to form one daughter neural stem cell, and one committed progenitor (*e*.*g*., either a neuron or a glial progenitor). In particular, neural stem cells have the capacity to produce cells of the three main mature classes of the central nervous system: neurons, astrocytes and oligodendrocytes. Typically, human neural stem cells possess the ability for engraftment, proliferation, migration and/or differentiation in a site-specific manner. Human neural stem cells can be characterised by the expression of transcription factors Sox1 and Sox2. In addition, human neural stem cells typically express characteristic cell surface markers. Such markers include CD133, CD49f, CD29, and CD15. Human neural stem cells can therefore be isolated based on the expression of such markers or a combination of such makers, for example, CD133 positive CD24 negative to low cells. In principle, any marker specifically expressed on human neural stem cells, or any combination of markers characteristic of human neural stem cells, may be used in the isolation of human neural stem cells.

Human neural stem cells may be derived from human embryonic, fetal or adult sources, or may be derived using known techniques from other cell types, including embryonic stem (ES) cells and induced pluripotent stem (iPS) cells. Human neural stem cells may also be derived by direct reprogramming from a somatic cell population, for example fibroblasts. In some aspects of the disclosure, human neural stem cells are derived from a somatic stem cell or a pluripotent stem cell. Human neural stem cells include cells derived from the human central nervous system, including fetal spinal cord and fetal brain tissues. For example, the human neural stem cells may be human central nervous system stem cells directly isolated from human brain tissue using a method described in Uchida et al., Proc Natl Acad Sci USA, 2000, 97(26): 14720-14725.

Human neural stem cells may be cultures in adherent cultures, including monolayer cultures, on substrate-coated tissue culture plates, in suspension culture, or as self-adherent complexes of cells, forming clusters known as neurospheres.

The term "primary human neural stem cell" refers to a human neural stem cell isolated from human tissue or to the progeny of a human neural stem cell maintained in culture. A primary human neural stem cell typically retains the characteristics of the cell originally isolated from human tissue, and can be distinguished from an immortalised cell line.

The term "genome editing" refers to a type of genetic engineering in which DNA is inserted, replaced, or removed from a target DNA, *e.g*., the genome of a cell, using one or more nucleases and/or nickases. The nucleases create specific double-strand breaks (DSBs) at desired locations in the genome, and harness the cell's endogenous mechanisms to repair the induced break by homology-directed repair (HDR) (*e*.*g*., homologous recombination) or by nonhomologous end joining (NHEJ). The nickases create specific single-strand breaks at desired locations in the genome. In one non-limiting example, two nickases can be used to create two single-strand breaks on opposite strands of a target DNA, thereby generating a blunt or a sticky end. Any suitable nuclease can be introduced into a cell to induce genome editing of a target DNA sequence including, but not limited to, CRISPR-associated protein (Cas) nucleases, zinc finger nucleases (ZFNs), transcription activator-like effector nucleases (TALENs), meganucleases, other endo- or exo-nucleases, variants thereof, fragments thereof, and combinations thereof. In particular aspects of the disclosure, nuclease-mediated genome editing of a target DNA sequence (*e.g.,* a safe harbor gene) by homology-directed repair (HDR) (*e*.*g*.*,* homologous recombination) is used for generating a genetically modified human neural stem cell in accordance with the methods described herein.

The term "DNA nuclease" refers to an enzyme capable of cleaving the phosphodiester bonds between the nucleotide subunits of DNA, and may be an endonuclease or an exonuclease. According to the disclosure, the DNA nuclease may be an engineered (e.g., programmable or targetable) DNA nuclease which can be used to induce genome editing of a target DNA sequence such as a safe harbor gene. Any suitable DNA nuclease can be used including, but not limited to, CRISPR-associated protein (Cas) nucleases, zinc finger nucleases (ZFNs), transcription activator-like effector nucleases (TALENs), meganucleases, other endo- or exo-nucleases, variants thereof, fragments thereof, and combinations thereof.

The term "double strand break" or "DSB" refers to the severing or cleavage of both strands of the DNA double helix. The DSB may result in cleavage of both stands at the same position leading to "blunt ends" or staggered cleavage resulting in a region of single stranded DNA at the end of each DNA fragment, or "sticky ends". A DSB may arise from the action of one or more DNA nucleases.

The term "homology-directed repair" or "HDR" refers to a mechanism in cells to accurately and precisely repair double-strand DNA breaks using a homologous template to guide repair. The most common form of HDR is homologous recombination (HR), a type of genetic recombination in which nucleotide sequences are exchanged between two similar or identical molecules of DNA.

The term "nucleic acid," "nucleotide," or "polynucleotide" refers to deoxyribonucleic acids (DNA), ribonucleic acids (RNA) and polymers thereof in either single-, double- or multi-stranded form. The term includes, but is not limited to, single-, double- or multi-stranded DNA or RNA, genomic DNA, cDNA, DNA-RNA hybrids, or a polymer comprising purine and/or pyrimidine bases or other natural, chemically modified, biochemically modified, non-natural, synthetic or derivatised nucleotide bases. In some aspects of the disclosure, a nucleic acid can comprise a mixture of DNA, RNA and analogs thereof. Unless specifically limited, the term encompasses nucleic acids containing known analogs of natural nucleotides that have similar binding properties as the reference nucleic acid and are metabolised in a manner similar to naturally occurring nucleotides. Unless otherwise indicated, a particular nucleic acid sequence also implicitly encompasses conservatively modified variants thereof (*e*.*g*., degenerate codon substitutions), alleles, orthologs, single nucleotide polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)). The term nucleic acid is used interchangeably with gene, cDNA, and mRNA encoded by a gene.

The term "modified nucleotide" or "nucleotide analog" refers to a nucleotide that contains one or more chemical modifications (*e*.*g*., substitutions) in or on the nitrogenous base of the nucleoside (*e*.*g*., cytosine (C), thymine (T) or uracil (U)), adenine (A), or guanine (G)), the sugar moiety of the nucleoside (*e*.*g*., ribose, deoxyribose, modified ribose, modified deoxyribose, six-membered sugar analog, or open-chain sugar analog), and/or the phosphate backbone.

The term "gene" or "nucleotide sequence encoding a polypeptide" means the segment of DNA involved in producing a polypeptide chain. The DNA segment may include regions preceding and following the coding region (leader and trailer) involved in the transcription/translation of the gene product and the regulation of the transcription/translation, as well as intervening sequences (introns) between individual coding segments (exons).

The term "safe harbor gene" or "safe harbor locus," used interchangeably herein, refers to a genomic location that permits expression of integrated transgenes, *e*.*g*., exogenous DNA in the genome. In some cases, a safe harbor locus allows for stable expression of integrated DNA with minimal impact on nearby or adjacent endogenous genes, regulatory element and the like. In some cases, a safe harbor gene enables sustainable gene expression and can be targeted by engineered nuclease for gene modification in various cell types including neural stem cells, derivatives thereof, and differentiated cells thereof. Safe harbor genes according to the invention are the IL2Rγ, and HBB genes. In some cases, more than one safe harbor gene can be targeted, thereby introducing more than one transgene into the genetically modified cell.

The term "transgene" refers to exogenous DNA in the genome. A transgene may contain a marker(s) for cell purification and/or a gene of interest. Suitable markers are known in the art, including cell surface markers such as CD8, CD19 and truncated CD19 and fluorescent markers such as GFP. A gene of interest can be chosen by the skilled person and can be a transgene encoding a protein associated with a disorder of the central nervous system or a gene encoding a neuroprotective or neuroregenerative protein, a variant thereof, a fragment thereof, or a peptide mimetic thereof.

The term "cassette," in the context of a transgene cassette, refers to a combination of genetic sequence elements that may be introduced as a single element and may function together to achieve a desired result. A transgene cassette may include a transgene operably linked to a heterologous promoter. For example, an expression cassette may comprise a promoter operably linked to a second polynucleotide (*e*.*g*., a coding sequence) and can include a promoter that is heterologous to the second polynucleotide as the result of human manipulation. A cassette typically comprises polynucleotides in combinations that are not found in nature.

The term "operably linked" refers to two or more genetic elements, such as a polynucleotide coding sequence and a promoter, placed in relative positions that permit the proper biological functioning of the elements, such as the promoter directing transcription of the coding sequence.

The term "heterologous promoter" refers to a promoter that is not operably linked to a transgene in nature. The heterologous promoter may be a sequence derived from a non-human organism or a chemically synthesised, optionally modified, promoter sequence. Alternatively, the heterologous promoter may be a human sequence from a genetic locus not naturally found in conjunction with the transgene.

The term "inducible promoter system" refers to a promoter that responds to environmental factors and/or external stimuli that can be artificially controlled in order to modify the expression of, or the level of expression of, a transgene or refers to a combination of elements, for example an exogenous promoter and an additional element such as a transactivator operably linked to a separate promoter. An inducible promoter system may respond to abiotic factors such as oxygen levels or to chemical or biological molecules. In some aspects of the disclosure, the chemical or biological molecules may be molecules not naturally present in humans.

The term "isolated" with reference to a cell, refers to a cell that is in an environment different from that in which the cell naturally occurs, e.g., where the cell naturally occurs in a multicellular organism, and the cell is removed from the multicellular organism, the cell is "isolated." An isolated genetically modified cell can be present in a mixed population of genetically modified cells, or in a mixed population comprising genetically modified cells and cells that are not genetically modified. For example, an isolated genetically modified cell can be present in a mixed population of genetically modified cells *in vitro,* or in a mixed in vitro population comprising genetically modified cells and cells that are not genetically modified.

The term "cell-specific promoter" refers to a promoter that directs expression differentially in different cell or tissue types. Expression may be present or absent or may take place at different levels in different cell types according to their developmental or differentiation status. For example, a promoter may drive expression of a transgene preferentially in neural cells, or in cells of a particular neural lineage. Thus, it is possible to express a transgene specifically in neurons, astrocytes or oligodendrocytes, or even in a particular class of neurons.

The phrase "transgene encodes a protein associated with a genetic disorder of the central nervous system" refers to a gene that encodes a product, the presence or absence of which causes or contributes to a genetic disorder of the central nervous system. The genetic disorder can be an autosomal recessive genetic disorder that may be treated by expressing a wild-type (normal) allele of the corresponding mutant gene as a transgene. The genetic disorder may be caused by a mutant gene and the transgene described herein can be used to treat the disorder. The phrase "transgene encodes a neuroprotective or neuroregenerative protein" refers to a transgene that encodes a product that prevents, or helps to prevent, the development or progression of a disorder of the nervous system or that contributes to or causes the restoration, or partial restoration, of a function of the nervous system lost as a result of a disease, condition or injury. The disease, disorder, or conditions described herein include those affecting astrocytes, oligodendrocytes, glial cells, neurons, motor neurons, interneurons, retinal cells, *etc.* Such neurological disease, disorders or conditions may include Parkinson's disease, Huntington's disease, Alzheimer's disease, memory disorders, epilepsies, macular degeneration (e.g., age-related macular degeneration), retinitis pigmentosa, Leber's congenital amaurosis, retinopathies, optic neuropathies, amyotrophic lateral sclerosis, spinal muscular atrophy, myelin disease, multiple sclerosis, stroke, cerebral palsies, hereditary pediatric leukodystrophies including Pelizaeus-Merbacher disease, neuronal ceroid lipofuscinosis and Krabbe disease, metachromatic leukodystrophy, Tay-Sachs disease, spinal cord injury or trauma. The disease, disorder, or conditions described herein also include traumatic brain injury, acute inflammation of the central nervous system (CNS), chronic inflammation of the CNS, ischemia, and stroke. The protein may be a protein encoded by a gene selected from the group consisting of *GALC* (Krabbe disease), *ABCD1* (adrenoleukodystrophy), *GFAP* (Alexander disease), *CYP27A1* (cerebrotendineous xanthomatosis), *ARSA* (metachromatic leukodystrophy), *PLP1* (Pelizaeus-Merzbacher disease), *ASPA* (Canavan disease), *EIF-2B* (leukoencephalopathy with vanishing white matter), *PHYH* (Refsum disease 1), *PEX7* (Refsum disease 2), *PPT1* (infantile neuronal ceroid lipofuscinosis (NCL)), *TPP1* (late infantile NCL), *CLN3* (juvenile NCL), *CLN6* (adult NCL), *CLN5* (Finnish late infantile variant NCL), *CLN6* (late infantile variant NCL), *MSFD8* (ceroid lipofuscinosis, neuronal, 7), *CLN8* (ceroid lipofuscinosis, neuronal, 8), *CTSD* (ceroid lipofuscinosis, neuronal, 10), *UBE3A* (Angelman syndrome), *POLG* (Alpers' Disease), *TAZ* (Barth Syndrome), *GLA* (Fabry disease), *SLC20A2* (Fahr's syndrome), *PDE* (retinitis pigmentosa), *SMN1* (spinal muscular atrophy), *IKBKAP* (familial dysautonomia), *MeCP2* (Rett syndrome), *CACNA1C* (Timothy syndrome), *ATXN3* (Machado-Joseph disease), and *RPE65* (Leber congenital amaurosis), *USH2A* (retinitis pigmentosa), *RPGR* (retinitis pigmentosa), *RP2*(retinitis pigmentosa), ABCA4 (Stargardt), RS-1 (X-linked retinoschisis).

The phrase "transgene encodes a neuroprotective or neuroregenerative protein" refers to a transgene that encodes a product that prevents, or helps to prevent, the development or progression of a disorder of the nervous system or that contributes to or causes the restoration, or partial restoration, of a function of the nervous system lost as a result of a disease, condition or injury. In certain aspects of the disclosure, the neuroprotective or neuroregenerative protein is selected from the group consisting of brain-derived neurotrophic factor (BDNF), insulin-like growth factor 1 (IGF-1), insulin-like growth factor 2 (IGF-2), glial-derived neurotrophic factor (GDNF), nerve growth factor (NGF), neurotrophin-2 (NT-2), neurotrophin-3 (NT-3) neurotrophin-4/5 (NT-4/5), neurotrophin-6, conserved dopamine neurotrophic factor (CDNF), ciliary neurotrophic factor (CNTF), epidermal growth factor (EGF), a fibroblast growth factor (FGF), a bone morphogenetic protein (BMP), vascular endothelial growth factor (VEGF), granulocyte colony-stimulating factor (G-CSF), colony-stimulating factor (CSF), interferon-β (IFN-β), tumor necrosis factor-a (TNFa), tissue plasminogen activator (tPA), neurturin, persephin, artemin, neuropeptide Y (NPY), an ephrin, a semaphorin, other neuropoeitic factors, other neurotrophic factors, and a combination thereof.

The terms "polypeptide," "peptide," and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. As used herein, the terms encompass amino acid chains of any length, including full-length proteins, wherein the amino acid residues are linked by covalent peptide bonds.

The term "variant" refers to a form of an organism, strain, gene, polynucleotide, polypeptide, or characteristic that deviates from what occurs in nature.

The term "peptide mimetic" refers to an amino acid polymer in which one or more amino acid residues, comprising all or part of the polymer, is an artificial chemical mimetic of a corresponding naturally occurring amino acid. A peptide mimetic may have improved properties, such as stability or biological activity, relative to a corresponding peptide consisting of naturally occurring amino acids. A peptide mimetic may, for example, comprise one or more D-amino acids.

The term "vector" or "recombinant vector" refers a nucleic acid construct, generated recombinantly or synthetically, with a series of specified nucleic acid elements that permit transcription of a particular polynucleotide sequence in a host cell. An expression vector may be part of a plasmid, viral genome, or nucleic acid fragment. Typically, an expression vector includes a polynucleotide to be transcribed, operably linked to a promoter. "Operably linked" in this context means two or more genetic elements, such as a polynucleotide coding sequence and a promoter, placed in relative positions that permit the proper biological functioning of the elements, such as the promoter directing transcription of the coding sequence. The term "promoter" is used herein to refer to an array of nucleic acid control sequences that direct transcription of a nucleic acid. As used herein, a promoter includes necessary nucleic acid sequences near the start site of transcription, such as, in the case of a polymerase II type promoter, a TATA element. A promoter also optionally includes distal enhancer or repressor elements, which can be located as much as several thousand base pairs from the start site of transcription. Other elements that may be present in an expression vector include those that enhance transcription (*e.g.*, enhancers) and terminate transcription (*e.g*., terminators), as well as those that confer certain binding affinity or antigenicity to the recombinant protein produced from the expression vector.

"Recombinant" refers to a genetically modified polynucleotide, polypeptide, cell, tissue, or organism. For example, a recombinant polynucleotide (or a copy or complement of a recombinant polynucleotide) is one that has been manipulated using well known methods. A recombinant expression cassette comprising a promoter operably linked to a second polynucleotide (*e*.*g*., a coding sequence) can include a promoter that is heterologous to the second polynucleotide as the result of human manipulation (*e*.*g*., by methods described in Sambrook et al., Molecular Cloning - A Laboratory Manual, Cold Spring Harbor Laboratory, Cold Spring Harbor, New York, (1989) or Current Protocols in Molecular Biology Volumes 1-3, John Wiley & Sons, Inc. (1994-1998)). A recombinant expression cassette (or expression vector) typically comprises polynucleotides in combinations that are not found in nature. For instance, human manipulated restriction sites or plasmid vector sequences can flank or separate the promoter from other sequences. A recombinant protein is one that is expressed from a recombinant polynucleotide, and recombinant cells, tissues, and organisms are those that comprise recombinant sequences (polynucleotide and/or polypeptide).

The term "selectable marker" refers to a gene product that permits a cell expressing that gene product to be isolated from a mixed population of cells. Such isolation might be achieved through the selective killing of cells not expressing the selectable marker, which may be an antibiotic resistance gene. Alternatively, the selectable marker may permit isolation of cells expressing the marker as a result of the expression of a fluorescent protein such as GFP or the expression of a cell surface marker which permits isolation of cells by fluorescence-activated cell sorting (FACS), magnetic-activated cell sorting (MACS), or analogous methods. Suitable cell surface markers include CD8, CD19, and truncated CD19. Preferably, cell surface markers used for isolating desired cells are non-signaling molecules, such as subunit or truncated forms of CD1, CD2, CD8α, CD10, CD19, or CD20. Suitable markers and techniques are known in the art.

The term "detectable marker" refers to a gene product that permits a cell expressing that gene product to be identified. Such identification can be visual identification, for example by means of the expression of a fluorescent protein or by expression of a cell surface marker that can be recognised by a labelled antibody. Suitable markers and techniques are known in the art.

The term "purification marker" refers to a marker that provides for the purification of a particular cell type. The purification marker may therefore also be a selectable marker as described above, thereby permitting purification of cells expressing the marker, for example by antibiotic selection or by FACS or MACS. Suitable markers and techniques are known in the art. Markers for purifying human neural stem cells include CD133, CD49f, CD29, and CD15. Human neural stem cells can also be isolated based on the expression of a combination of makers, for example, CD133 positive CD24 negative to low cells. In principle, any marker specifically expressed on human neural stem cells, or any combination of markers characteristic of human neural stem cells, may be used in the isolation of human neural stem cells.

The terms "subject," "individual," and "patient" are used interchangeably herein to refer to a vertebrate, preferably a mammal, more preferably a human. Mammals include, but are not limited to, murines, simians, humans, farm animals, sport animals, and pets. Tissues, cells and their progeny of a biological entity obtained *in vivo* or cultured *in vitro* are also encompassed.

The term "treating" refers to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

The term "effective amount" or "sufficient amount" refers to the amount of an agent that is sufficient to effect beneficial or desired results. The therapeutically effective amount may vary depending upon one or more of: the subject and disease condition being treated, the weight and age of the subject, the severity of the disease condition, the manner of administration and the like, which can readily be determined by one of ordinary skill in the art. The specific amount may vary depending on one or more of: the particular agent chosen, the target cell type, the location of the target cell in the subject, the dosing regimen to be followed, whether it is administered in combination with other compounds, timing of administration, and the physical delivery system in which it is carried.

The term "pharmaceutically acceptable carrier" refers to a substance that aids the administration of an active agent to a cell, an organism, or a subject. "Pharmaceutically acceptable carrier" refers to a carrier or excipient that can be included in the compositions of the disclosure and that causes no significant adverse toxicological effect on the patient. Non-limiting examples of pharmaceutically acceptable carrier include water, NaCl, normal saline solutions, lactated Ringer's, normal sucrose, normal glucose, cell culture media, and the like. One of skill in the art will recognize that other pharmaceutical carriers are useful in certain aspects of the disclosure.

The term "about" in relation to a reference numerical value can include a range of values plus or minus 10% from that value. For example, the amount "about 10" includes amounts from 9 to 11, including the reference numbers of 9, 10, and 11. The term "about" in relation to a reference numerical value can also include a range of values plus or minus 10%, 9%, 8%, 7%, 6%, 5%, 4%, 3%, 2%, or 1% from that value.

### IV. Detailed Description

As used herein and in the appended claims, the singular forms "a," "or," and "the" include plural referents unless the context clearly dictates otherwise. It is understood that aspects and variations of the disclosure described herein include "consisting" and/or "consisting essentially of aspects and variations.

Aspects of the disclosure provides a method for generating a genetically modified human neural stem cell, the method comprising: introducing into an isolated human neural stem cell: a DNA nuclease or a nucleotide sequence encoding the DNA nuclease, wherein the DNA nuclease is capable of creating a double-strand break in a gene to induce end joining, thereby generating a genetically modified human neural stem cell.

Aspects of the disclosure, provide a method for generating a genetically modified human neural stem cell, the method comprising: introducing into an isolated human neural stem cell: (a) a donor template comprising: (i) a transgene cassette comprising a transgene (for example, a transgene operably linked to a promoter, for example, a heterologous promoter); and (ii) two nucleotide sequences comprising two non-overlapping, homologous portions of a safe harbor gene, wherein the nucleotide sequences are located at the 5' and 3' ends of the transgene cassette; and (b) a DNA nuclease or a nucleotide sequence encoding the DNA nuclease, wherein the DNA nuclease is capable of creating a double-strand break in the safe harbor gene to induce insertion of the transgene into the safe harbor gene, thereby generating a genetically modified human neural stem cell.

The safe harbor locus is selected from the group consisting of IL2Rγ, and HBB. In some aspects of the disclosure, the safe harbor locus is IL2Ry. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

A safe harbor locus can be a genomic location that permits expression of integrated transgenes, *e*.*g*., exogenous DNA in the genome. In some cases, the safe harbor locus allows for stable expression of integrated DNA with minimal impact on nearby or adjacent endogenous genes, regulatory element and the like. In some cases, the safe harbor gene enables sustainable gene expression and can be targeted by engineered nuclease for gene modification in various cell types include neural stem cells, derivatives thereof, and differentiated cells thereof.

In some aspects of the disclosure, there is provided a method for generating a genetically modified human neural stem cell, the method comprising: 1) culturing an isolated human neural stem cell; 2) introducing into said cultured human neural stem cell: (a) a donor template comprising: (i) a transgene cassette comprising a transgene (for example, a transgene operably linked to a promoter, for example, a heterologous promoter); and (ii) two nucleotide sequences comprising two non-overlapping, homologous portions of a safe harbor gene, wherein the nucleotide sequences are located at the 5' and 3' ends of the transgene cassette; and (b) a DNA nuclease or a nucleotide sequence encoding the DNA nuclease, wherein the DNA nuclease is capable of creating a double-strand break in the safe harbor gene to induce insertion of the transgene into the safe harbor gene, thereby generating a genetically modified human neural stem cell. The safe harbor locus is selected from the group consisting of IL2Rγ, and HBB. In some aspects of the disclosure, the safe harbor locus is IL2Ry. In some aspects of the disclosure, the neural stem cells are cultured as self-adherent complexes of cells, (e.g. by forming clusters known as neurospheres). In some aspects of the disclosure, the neural stem cells are cultured in a condition with a supplement selected from the group consisting of N2, heparin, N-acetylcysteine, fibroblast growth factor 2, epidermal growth factor, and leukemia inhibitory factor. In some aspects of the disclosure, the neural stem cells are cultured for at least about any of 5, 10, 15, 20, 25, 30, 35, 40, 45, 50, 55, 60, 65, 70, 75, 80, 85, 90, 95, or 100 days before the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages before the donor template and/or the DNA nuclease (or the nucleotide sequence encoding the DNA nuclease) is introduced into said neural stem cells. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure , there is provided a method for generating a genetically modified human neural stem cell, the method comprising: 1) isolating a population of human neural stem cells; 2) culturing said isolated human neural stem cells; 3) introducing into said cultured human neural stem cell: (a) a donor template comprising: (i) a transgene cassette comprising a transgene (for example, a transgene operably linked to a promoter, for example, a heterologous promoter); and (ii) two nucleotide sequences comprising two non-overlapping, homologous portions of a safe harbor gene, wherein the nucleotide sequences are located at the 5' and 3' ends of the transgene cassette; and (b) a DNA nuclease or a nucleotide sequence encoding the DNA nuclease, wherein the DNA nuclease is capable of creating a double-strand break in the safe harbor gene to induce insertion of the transgene into the safe harbor gene, thereby generating a genetically modified human neural stem cell. The safe harbor locus is selected from the group consisting of IL2Rγ, and HBB. In some aspects of the disclosure, the safe harbor locus is IL2Ry. In some aspects of the disclosure, the neural stem cells are cultured as self-adherent complexes of cells, (e.g. by forming clusters known as neurospheres). In some aspects of the disclosure, the neural stem cells are cultured in a condition with a supplement selected from the group consisting of N2, heparin, N-acetylcysteine, fibroblast growth factor 2, epidermal growth factor, and leukemia inhibitory factor. In some aspects of the disclosure, the neural stem cells are cultured for at least about any of 5, 10, 15, 20, 25, 30, 35, 40, 45, 50, 55, 60, 65, 70, 75, 80, 85, 90, 95, or 100 days before the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages before the donor template and/or the DNA nuclease (or the nucleotide sequence encoding the DNA nuclease) is introduced into said neural stem cells. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: 1) introducing into a population of human cells comprising human neural stem cells: (a) a donor template comprising: (i) a transgene cassette comprising a transgene (for example, a transgene operably linked to a promoter, for example, a heterologous promoter); and (ii) two nucleotide sequences comprising two non-overlapping, homologous portions of a safe harbor gene, wherein the nucleotide sequences are located at the 5' and 3' ends of the transgene cassette; and (b) a DNA nuclease or a nucleotide sequence encoding the DNA nuclease, wherein the DNA nuclease is capable of creating a double-strand break in the safe harbor gene to induce insertion of the transgene into the safe harbor gene; 2) isolating human neural stem cells from said population of human cells, thereby generating a genetically modified human neural stem cell. In some aspects of the disclosure, the method further comprises isolating genetically modified human neural stem cells from said isolated human neural stem cells. The safe harbor locus is selected from the group consisting of IL2Rγ, and HBB. In some embodiments, the safe harbor locus is IL2Rγ. In some aspects of the disclosure, the neural stem cells are cultured as self-adherent complexes of cells, (e.g. by forming clusters known as neurospheres). In some aspects of the disclosure, the neural stem cells are cultured in a condition with a supplement selected from the group consisting of N2, heparin, N-acetylcysteine, fibroblast growth factor 2, epidermal growth factor, and leukemia inhibitory factor. In some aspects of the disclosure, the method further comprises culturing isolated genetically modified human neural stem cells. In some aspects of the disclosure, the culturing time is at least about any of 5, 10, 15, 20, 25, 30, 35, 40, 45, 50, 55, 60, 65, 70, 75, 80, 85, 90, 95, or 100 days after the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages after the donor template and/or the DNA nuclease (or the nucleotide sequence encoding the DNA nuclease) is introduced into said neural stem cells. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: introducing into an isolated human neural stem cell: (a) a donor template comprising: (i) a transgene cassette comprising a transgene (for example, a transgene operably linked to a promoter, for example, a heterologous promoter); and (ii) two nucleotide sequences comprising two non-overlapping, homologous portions of a safe harbor gene, wherein the nucleotide sequences are located at the 5' and 3' ends of the transgene cassette; and (b) an RNA-guided CRISPR/Cas system (such as RNA-based CRISPR/Cas system), wherein the CRISPR/Cas system is capable of creating a double-strand break in the safe harbor gene to induce insertion of the transgene into the safe harbor gene, thereby generating a genetically modified human neural stem cell. The safe harbor locus is selected from the group consisting of IL2Rγ, and HBB. In some aspects of the disclosure, the safe harbor locus is IL2Rγ. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: 1) culturing an isolated human neural stem cell; 2) introducing into said cultured human neural stem cell: (a) a donor template comprising: (i) a transgene cassette comprising a transgene (for example, a transgene operably linked to a promoter, for example, a heterologous promoter); and (ii) two nucleotide sequences comprising two non-overlapping, homologous portions of a safe harbor gene, wherein the nucleotide sequences are located at the 5' and 3' ends of the transgene cassette; and (b) an RNA-guided CRISPR/Cas system (such as RNA-based CRISPR/Cas system), wherein the CRISPR/Cas system is capable of creating a double-strand break in the safe harbor gene to induce insertion of the transgene into the safe harbor gene, thereby generating a genetically modified human neural stem cell. The safe harbor locus is selected from the group consisting of IL2Rγ, and HBB. In some aspects of the disclosure, the safe harbor locus is IL2Ry. In some aspects of the disclosure, the neural stem cells are cultured as self-adherent complexes of cells, (e.g. by forming clusters known as neurospheres). In some aspects of the disclosure, the neural stem cells are cultured in a condition with a supplement selected from the group consisting of N2, heparin, N-acetylcysteine, fibroblast growth factor 2, epidermal growth factor, and leukemia inhibitory factor. In some aspects of the disclosure, the neural stem cells are cultured for at least about any of 5, 10, 15, 20, 25, 30, 35, 40, 45, 50, 55, 60, 65, 70, 75, 80, 85, 90, 95, or 100 days before the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages before the donor template and/or the DNA nuclease (or the nucleotide sequence encoding the DNA nuclease) is introduced into said neural stem cells. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: 1) isolating a population of human neural stem cells; 2) culturing said isolated human neural stem cells; 3) introducing into said cultured human neural stem cell: (a) a donor template comprising: (i) a transgene cassette comprising a transgene (for example, a transgene operably linked to a promoter, for example, a heterologous promoter); and (ii) two nucleotide sequences comprising two non-overlapping, homologous portions of a safe harbor gene, wherein the nucleotide sequences are located at the 5' and 3' ends of the transgene cassette; and (b) an RNA-guided CRISPR/Cas system (such as RNA-based CRISPR/Cas system), wherein the CRISPR/Cas system is capable of creating a double-strand break in the safe harbor gene to induce insertion of the transgene into the safe harbor gene, thereby generating a genetically modified human neural stem cell. The safe harbor locus is selected from the group consisting of IL2Rγ, and HBB. In some aspects of the disclosure, the safe harbor locus is IL2Ry. In some aspects of the disclosure, the neural stem cells are cultured as self-adherent complexes of cells, (e.g. by forming clusters known as neurospheres). In some aspects of the disclosure, the neural stem cells are cultured in a condition with a supplement selected from the group consisting of N2, heparin, N-acetylcysteine, fibroblast growth factor 2, epidermal growth factor, and leukemia inhibitory factor. In some aspects of the disclosure, the neural stem cells are cultured for at least about any of 5, 10, 15, 20, 25, 30, 35, 40, 45, 50, 55, 60, 65, 70, 75, 80, 85, 90, 95, or 100 days before the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages before the donor template and/or the DNA nuclease (or the nucleotide sequence encoding the DNA nuclease) is introduced into said neural stem cells. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: 1) introducing into a population of human cells comprising human neural stem cells: (a) a donor template comprising: (i) a transgene cassette comprising a transgene (for example, a transgene operably linked to a promoter, for example, a heterologous promoter); and (ii) two nucleotide sequences comprising two non-overlapping, homologous portions of a safe harbor gene, wherein the nucleotide sequences are located at the 5' and 3' ends of the transgene cassette; and (b) an RNA-guided CRISPR/Cas system (such as RNA-based CRISPR/Cas system), wherein the CRISPR/Cas system is capable of creating a double-strand break in the safe harbor gene to induce insertion of the transgene into the safe harbor gene; 2) isolating human neural stem cells from said population of human cells, thereby generating a genetically modified human neural stem cell. In some aspects of the disclosure, the method further comprises isolating genetically modified human neural stem cells from said isolated human neural stem cells. The safe harbor locus is selected from the group consisting of IL2Rγ, and HBB. In some aspects of the disclosure, the safe harbor locus is IL2Ry. In some aspects of the disclosure, the neural stem cells are cultured as self-adherent complexes of cells, (e.g. by forming clusters known as neurospheres). In some aspects of the disclosure, the neural stem cells are cultured in a condition with a supplement selected from the group consisting of N2, heparin, N-acetylcysteine, fibroblast growth factor 2, epidermal growth factor, and leukemia inhibitory factor. In some aspects of the disclosure, the neural stem cells are cultured for at least about any of 5, 10, 15, 20, 25, 30, 35, 40, 45, 50, 55, 60, 65, 70, 75, 80, 85, 90, 95, or 100 days after the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages after the donor template and/or the DNA nuclease (or the nucleotide sequence encoding the DNA nuclease) is introduced into said neural stem cells. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: introducing into an isolated human neural stem cell: (a) a donor template comprising: (i) a transgene cassette comprising a transgene (for example, a transgene operably linked to a promoter, for example, a heterologous promoter); (ii) a nucleic acid encoding a selectable marker; and (iii) two nucleotide sequences comprising two non-overlapping, homologous portions of a safe harbor gene, wherein the nucleotide sequences are located at the 5' and 3' ends of the transgene cassette; and (b) a DNA nuclease or a nucleotide sequence encoding the DNA nuclease, wherein the DNA nuclease is capable of creating a double-strand break in the safe harbor gene to induce insertion of the transgene into the safe harbor gene, thereby generating a genetically modified human neural stem cell. The safe harbor locus is selected from the group consisting of IL2Rγ, and HBB. In some aspects of the disclosure, the safe harbor locus is IL2Ry. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: 1) culturing an isolated human neural stem cell; 2) introducing into said cultured human neural stem cell: (a) a donor template comprising: (i) a transgene cassette comprising a transgene (for example, a transgene operably linked to a promoter, for example, a heterologous promoter); (ii) a nucleic acid encoding a selectable marker; and (iii) two nucleotide sequences comprising two non-overlapping, homologous portions of a safe harbor gene, wherein the nucleotide sequences are located at the 5' and 3' ends of the transgene cassette; and (b) a DNA nuclease or a nucleotide sequence encoding the DNA nuclease, wherein the DNA nuclease is capable of creating a double-strand break in the safe harbor gene to induce insertion of the transgene into the safe harbor gene, thereby generating a genetically modified human neural stem cell. In some aspects of the disclosure, the method further comprises isolating the genetically modified human neural stem cell based on the selectable marker. In some aspects of the disclosure, the selectable marker is a cell surface marker. The safe harbor locus is selected from the group consisting of IL2Rγ, and HBB. In some aspects of the disclosure, the safe harbor locus is IL2Rγ. In some aspects of the disclosure, the neural stem cells are cultured as self-adherent complexes of cells, (e.g. by forming clusters known as neurospheres). In some aspects of the disclosure, the neural stem cells are cultured in a condition with a supplement selected from the group consisting of N2, heparin, N-acetylcysteine, fibroblast growth factor 2, epidermal growth factor, and leukemia inhibitory factor. In some aspects of the disclosure, the neural stem cells are cultured for at least about any of 5, 10, 15, 20, 25, 30, 35, 40, 45, 50, 55, 60, 65, 70, 75, 80, 85, 90, 95, or 100 days before the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages before the donor template and/or the DNA nuclease (or the nucleotide sequence encoding the DNA nuclease) is introduced into said neural stem cells. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: 1) isolating a population of human neural stem cells; 2) culturing said isolated human neural stem cells; 3) introducing into said cultured human neural stem cell: (a) a donor template comprising: (i) a transgene cassette comprising a transgene (for example, a transgene operably linked to a promoter, for example, a heterologous promoter); (ii) a nucleic acid encoding a selectable marker; and (iii) two nucleotide sequences comprising two non-overlapping, homologous portions of a safe harbor gene, wherein the nucleotide sequences are located at the 5' and 3' ends of the transgene cassette; and (b) a DNA nuclease or a nucleotide sequence encoding the DNA nuclease, wherein the DNA nuclease is capable of creating a double-strand break in the safe harbor gene to induce insertion of the transgene into the safe harbor gene, thereby generating a genetically modified human neural stem cell. In some aspects of the disclosure, the method further comprises isolating the genetically modified human neural stem cell based on the selectable marker. In some aspects of the disclosure, the the selectable marker is a cell surface marker. In some aspects of the disclosure, the cell surface marker is selected from the group consisting of CD8, CD19, or a truncated fragment thereof. The safe harbor locus is selected from the group consisting of IL2Rγ, and HBB. In some aspects of the disclosure, the safe harbor locus is IL2Ry. In some aspects of the disclosure, the neural stem cells are cultured as self-adherent complexes of cells, (e.g. by forming clusters known as neurospheres). In some aspects of the disclosure, the neural stem cells are cultured in a condition with a supplement selected from the group consisting of N2, heparin, N-acetylcysteine, fibroblast growth factor 2, epidermal growth factor, and leukemia inhibitory factor. In some aspects of the disclosure, the neural stem cells are cultured for at least about any of 5, 10, 15, 20, 25, 30, 35, 40, 45, 50, 55, 60, 65, 70, 75, 80, 85, 90, 95, or 100 days before the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages before the donor template and/or the DNA nuclease (or the nucleotide sequence encoding the DNA nuclease) is introduced into said neural stem cells. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: 1) introducing into a population of human cells comprising human neural stem cells: (a) a donor template comprising: (i) a transgene cassette comprising a transgene (for example, a transgene operably linked to a promoter, for example, a heterologous promoter); (ii) a nucleic acid encoding a selectable marker; and (iii) two nucleotide sequences comprising two non-overlapping, homologous portions of a safe harbor gene, wherein the nucleotide sequences are located at the 5' and 3' ends of the transgene cassette; and (b) a DNA nuclease or a nucleotide sequence encoding the DNA nuclease, wherein the DNA nuclease is capable of creating a double-strand break in the safe harbor gene to induce insertion of the transgene into the safe harbor gene; 2) isolating human neural stem cells from said population of human cells, thereby generating a genetically modified human neural stem cell. In some aspects of the disclosure, the method further comprises isolating genetically modified human neural stem cells from said isolated human neural stem cells. In some aspects of the disclosure, the method further comprises isolating the genetically modified human neural stem cell based on the selectable marker. In some aspects of the disclosure, the -selectable marker is a cell surface marker. In some aspects of the disclosure, the cell surface marker is selected from the group consisting of CD8, CD19, or a truncated fragment thereof. The safe harbor locus is selected from the group consisting of IL2Rγ, and HBB. In some aspects of the disclosure, the safe harbor locus is IL2Ry. In some aspects of the disclosure, the neural stem cells are derived from second trimester human fetal brain. In some aspects of the disclosure, the neural stem cells are isolated based on the expression of a combination of markers, such as C133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: introducing into an isolated human neural stem cell: (a) a donor template comprising: (i) a transgene cassette comprising a transgene (for example, a transgene operably linked to a promoter, for example, a heterologous promoter); (ii) a nucleic acid encoding a selectable marker; and (iii) two nucleotide sequences comprising two non-overlapping, homologous portions of a safe harbor gene, wherein the nucleotide sequences are located at the 5' and 3' ends of the transgene cassette; and (b) an RNA-guided CRISPR/Cas system (such as RNA-based CRISPR/Cas system), wherein the CRISPR/Cas system is capable of creating a double-strand break in the safe harbor gene to induce insertion of the transgene into the safe harbor gene, thereby generating a genetically modified human neural stem cell. In some aspects of the disclosure, the method further comprises isolating genetically modified human neural stem cells from said isolated human neural stem cells. In some aspects of the disclosure, the method further comprises isolating the genetically modified human neural stem cell based on the selectable marker. In some aspects of the disclosure, the selectable marker is a cell surface marker. In some aspects of the disclosure, the cell surface marker is selected from the group consisting of CD8, CD19, or a truncated fragment thereof. The safe harbor locus is selected from the group consisting of IL2Rγ, and HBB. In some aspects of the disclosure, the safe harbor locus is IL2Ry. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: 1) culturing an isolated human neural stem cell; 2) introducing into said cultured human neural stem cell: (a) a donor template comprising: (i) a transgene cassette comprising a transgene (for example, a transgene operably linked to a promoter, for example, a heterologous promoter); (ii) a nucleic acid encoding a selectable marker; and (iii) two nucleotide sequences comprising two non-overlapping, homologous portions of a safe harbor gene, wherein the nucleotide sequences are located at the 5' and 3' ends of the transgene cassette; and (b) an RNA-guided CRISPR/Cas system (such as RNA-based CRISPR/Cas system), wherein the CRISPR/Cas system is capable of creating a double-strand break in the safe harbor gene to induce insertion of the transgene into the safe harbor gene, thereby generating a genetically modified human neural stem cell. In some aspects of the disclosure, the method further comprises isolating genetically modified human neural stem cells from said isolated human neural stem cells. In some aspects of the disclosure, the method further comprises isolating the genetically modified human neural stem cell based on the purification marker. In some aspects of the disclosure, the selectable marker is a cell surface marker. In some aspects of the disclosure, the cell surface marker is selected from the group consisting of CD8, CD19, or a truncated fragment thereof. The safe harbor locus is selected from the group consisting of IL2Rγ, and HBB. In some aspects of the disclosure, the safe harbor locus is IL2Rγ. In some aspects of the disclosure, the neural stem cells are cultured as self-adherent complexes of cells, (e.g. by forming clusters known as neurospheres). In some aspects of the disclosure, the neural stem cells are cultured in a condition with a supplement selected from the group consisting of N2, heparin, N-acetylcysteine, fibroblast growth factor 2, epidermal growth factor, and leukemia inhibitory factor. In some aspects of the disclosure, the neural stem cells are cultured for at least about any of 5, 10, 15, 20, 25, 30, 35, 40, 45, 50, 55, 60, 65, 70, 75, 80, 85, 90, 95, or 100 days before the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages before the donor template and/or the DNA nuclease (or the nucleotide sequence encoding the DNA nuclease) is introduced into said neural stem cells. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: 1) isolating a population of human neural stem cells; 2) culturing said isolated human neural stem cells; 3) introducing into said cultured human neural stem cell: (a) a donor template comprising: (i) a transgene cassette comprising a transgene (for example, a transgene operably linked to a promoter, for example, a heterologous promoter); (ii) a nucleic acid encoding a selectable marker; and (iii) two nucleotide sequences comprising two non-overlapping, homologous portions of a safe harbor gene, wherein the nucleotide sequences are located at the 5' and 3' ends of the transgene cassette; and (b) (b) an RNA-guided CRISPR/Cas system (such as RNA-based CRISPR/Cas system), wherein the CRISPR/Cas system is capable of creating a double-strand break in the safe harbor gene to induce insertion of the transgene into the safe harbor gene, thereby generating a genetically modified human neural stem cell. In some aspects of the disclosure, the method further comprises isolating genetically modified human neural stem cells from said isolated human neural stem cells. In some aspects of the disclosure, the method further comprises isolating the genetically modified human neural stem cell based on the selectable marker. In some aspects of the disclosure, the selectable marker is a cell surface marker. In some aspects of the disclosure, the cell surface marker is selected from the group consisting of CD8, CD19, or a truncated fragment thereof. The safe harbor locus is selected from the group consisting of IL2Rγ, and HBB. In some aspects of the disclosure, the safe harbor locus is IL2Rγ. In some aspects of the disclosure, the neural stem cells are cultured as self-adherent complexes of cells, (e.g. by forming clusters known as neurospheres). In some aspects of the disclosure, the neural stem cells are cultured in a condition with a supplement selected from the group consisting of N2, heparin, N-acetylcysteine, fibroblast growth factor 2, epidermal growth factor, and leukemia inhibitory factor. In some aspects of the disclosure, the neural stem cells are cultured for at least about any of 5, 10, 15, 20, 25, 30, 35, 40, 45, 50, 55, 60, 65, 70, 75, 80, 85, 90, 95, or 100 days before the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages before the donor template and/or the DNA nuclease (or the nucleotide sequence encoding the DNA nuclease) is introduced into said neural stem cells. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, there is provided a method for generating a genetically modified human neural stem cell, the method comprising: 1) introducing into a population of human cells comprising human neural stem cells: (a) a donor template comprising: (i) a transgene; (ii) a nucleic acid encoding a selectable marker; and (iii) two nucleotide sequences comprising two non-overlapping, homologous portions of a safe harbor gene, wherein the nucleotide sequences are located at the 5' and 3' ends of the transgene cassette; and (b) an RNA-guided CRISPR/Cas system (such as RNA-based CRISPR/Cas system), wherein the CRISPR/Cas system is capable of creating a double-strand break in the safe harbor gene to induce insertion of the transgene into the safe harbor gene; 2) isolating human neural stem cells from said population of human cells, thereby generating a genetically modified human neural stem cell.

In some aspects of the disclosure, there is provided a method for generating a genetically modified human neural stem cell, the method comprising: 1) introducing into a population of human cells comprising human neural stem cells: (a) a donor template comprising: (i) a transgene cassette comprising a transgene (for example, a transgene operably linked to a promoter, for example, a heterologous promoter); (ii) a nucleic acid encoding a purification marker; and (iii) two nucleotide sequences comprising two non-overlapping, homologous portions of a safe harbor gene, wherein the nucleotide sequences are located at the 5' and 3' ends of the transgene cassette; and (b) an RNA-guided CRISPR/Cas system (such as RNA-based CRISPR/Cas system), wherein the CRISPR/Cas system is capable of creating a double-strand break in the safe harbor gene to induce insertion of the transgene into the safe harbor gene; 2) isolating human neural stem cells from said population of human cells, thereby generating a genetically modified human neural stem cell. In some aspects of the disclosure, the method further comprises isolating genetically modified human neural stem cells from said isolated human neural stem cells. In some aspects of the disclosure, the method further comprises isolating genetically modified human neural stem cells from said isolated human neural stem cells. In some aspects of the disclosure, the method further comprises isolating the genetically modified human neural stem cell based on the selectable marker. In some aspects of the disclosure, the selectable marker is a cell surface marker. In some aspects of the disclosure, the cell surface marker is selected from the group consisting of CD8, CD19, or a truncated fragment thereof. The safe harbor locus is selected from the group consisting of IL2Rγ, and HBB. In some aspects of the disclosure, the safe harbor locus is IL2Rγ. In some aspects of the disclosure, the neural stem cells are cultured as self-adherent complexes of cells, (e.g. by forming clusters known as neurospheres). In some aspects of the disclosure, the neural stem cells are cultured in a condition with a supplement selected from the group consisting of N2, heparin, N-acetylcysteine, fibroblast growth factor 2, epidermal growth factor, and leukemia inhibitory factor. In some aspects of the disclosure, the neural stem cells are cultured for at least about any of 5, 10, 15, 20, 25, 30, 35, 40, 45, 50, 55, 60, 65, 70, 75, 80, 85, 90, 95, or 100 days after the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages after the donor template and/or the DNA nuclease (or the nucleotide sequence encoding the DNA nuclease) is introduced into said neural stem cells. In some aspects of the disclosure, the neural stem cells are derived from second trimester human fetal brain. In some aspects of the disclosure, the neural stem cells are isolated based on the expression of a combination of markers, such as CD133 and CD24. In some aspects of the disclosure, the neural stem cells are isolated based on the expression of a combination of two or more markers selected from the group consisting CD133, CD24, CD49f, CD29, and CD15.

In some aspects of the disclosure, multiple transgenes are introduced into multiple safe harbor loci. In some aspects of the disclosure, multiple transgenes are introduced into one single safe harbor locus. In some aspects of the disclosure, a single transgene or multiple transgenes are introduced into non-gene safe harbor locus or loci.

The transgenes described herein can encode proteins or functional nucleic acids (such as microRNA). In some aspects of the disclosure, the transgene encodes a protein associated with a genetic disorder of the central nervous system. In some aspects of the disclosure, the transgene encodes a protein selected from the group consisting of GALC (Krabbe disease), ABCD1 (adrenoleukodystrophy), GFAP (Alexander disease), CYP27A1 (cerebrotendineous xanthomatosis), ARSA (metachromatic leukodystrophy), PLP1 (Pelizaeus-Merzbacher disease), ASPA (Canavan disease), EIF-2B (leukoencephalopathy with vanishing white matter), PHYH (Refsum disease 1), PEX7 (Refsum disease 2), PPT1 (infantile neuronal ceroid lipofuscinosis (NCL)), TPP1 (late infantile NCL), CLN3 (juvenile NCL), CLN6 (adult NCL), CLN5 (Finnish late infantile variant NCL), CLN6 (late infantile variant NCL), MSFD8 (ceroid lipofuscinosis, neuronal, 7), CLN8 (ceroid lipofuscinosis, neuronal, 8), CTSD (ceroid lipofuscinosis, neuronal, 10), UBE3A (Angelman syndrome), POLG (Alpers' Disease), TAZ (Barth Syndrome), GLA (Fabry disease), SLC20A2 (Fahr's syndrome), PDE (retinitis pigmentosa), SMN1 (spinal muscular atrophy), IKBKAP (familial dysautonomia), MeCP2 (Rett syndrome), CACNA1C (Timothy syndrome), ATXN3 (Machado-Joseph disease), and RPE65 (Leber congenital amaurosis) , USH2A (retinitis pigmentosa), RPGR (retinitis pigmentosa), RP2(retinitis pigmentosa), ABCA4 (Stargardt), RS-1 (X-linked retinoschisis). In some aspects of the disclosure, the protein is secreted by the genetically modified human neural stem cells.

In some aspects of the disclosure, the transgene encodes a neuroprotective or neuroregenerative protein, a variant thereof, a fragment thereof, or a peptide mimetic thereof. In some aspects of the disclosure, the transgene encodes a neuroprotective or neurodegenerative protein which is selected from the group consisting of brain-derived neurotrophic factor (BDNF), glial-derived neurotrophic factor (GDNF), insulin-like growth factor 1 (IGF1), insulin-like growth factor 2 (IGF2), nerve growth factor (NGF), neurotrophin-2 (NT-2), neurotrophin-3 (NT-3) neurotrophin-4/5 (NT-4/5), neurotrophin-6, conserved dopamine neurotrophic factor (CDNF), ciliary neurotrophic factor (CNTF), epidermal growth factor (EGF), a fibroblast growth factor (FGF), a bone morphogenetic protein (BMP), vascular endothelial growth factor (VEGF), granulocyte colony-stimulating factor (G-CSF), colony-stimulating factor (CSF), interferon-β (IFN-β), tumor necrosis factor-α (TNFa), tissue plasminogen activator (tPA), neurturin, persephin, artemin, neuropeptide Y (NPY), an ephrin, a semaphorin, other neuropoeitic factors, other neurotrophic factors, and a combination thereof. In some aspects of the disclosure, the transgene encodes a neuroprotective or neurodegenerative protein, wherein the neuroprotective or neuroregenerative protein is a secreted protein. In some aspects of the disclosure, the transgene encodes Bcl-2. In some aspects of the disclosure, the transgene encodes a telomerase. In some aspects of the disclosure, the transgene encodes a protein that can improve survival or rejuvenate the neural stem cell to facilitate long-term survival and/or discovery of therapeutic molecules.

The transgene cassettes described herein comprises a promoter sequence, such as a heterologous promoter, which is operably linked to the transgene. In some aspects of the disclosure, the promoter and the transgene are located in the same safe harbor gene. In some aspects of the disclosure, the promoter and the transgene are located in different safe harbor genes. In some aspects of the disclosure, the promoter is a inducible promoter. In some aspects of the disclosure, the promoter is a cell-specific promoter. In some aspects of the disclosure when multiple transgenes are introduced into the human neural stem cells, the transgenes may be controlled by one or more promoters. In some aspects of the disclosure, the promoter or promoters can be a U6 RNA polymerase III promoter, a transcriptional control element, enhancer, U6 termination sequence, one or more nuclear localization signals, etc.

In some aspects of the disclosure, the transgene cassette described herein is inserted into the safe harbor locus such that the transgene is operably linked to an endogenous promoter.

In some aspects of the disclosure, the transgene and the DNA nuclease or a nucleotide sequence encoding the DNA nuclease are introduced simultaneously. In some aspects of the disclosure, the transgene and the DNA nuclease or a nucleotide sequence encoding the DNA nuclease are introduced sequentially.

In some aspects of the disclosure, the nuclease system is TALEN system. In some aspects of the disclosure, the TALEN system comprises a plasmid-based TALEN. In some aspects of the disclosure, the TALEN system comprises a RNA-based TALEN.

In some aspects of the disclosure, the nuclease system is a CRISPR/Cas9 system. In some aspects of the disclosure, the CRISPR/Cas9 system comprises a plasmid-based Cas9. In some aspects of the disclosure, the CRISPR/Cas9 system comprises a RNA-based Cas9. In some aspects of the disclosure, the CRISPR/Cas9 system comprises a Cas9 mRNA and sgRNA. In some aspects of the disclosure, the CRISPR/Cas9 system comprises a protein/RNA complex, or a plasmid/RNA complex, or a protein/plasmid complex. In some aspects of the disclosure, there are provided methods for generating genetically modified neural stem cells, which comprises introducing into said isolated neural stem cells a transgene and a DNA nuclease system, wherein the transgene is inserted into a safe harbor locus, and wherein the DNA nuclease system comprises a Cas9 mRNA and a locus-specific sgRNA. In some aspects of the disclosure, the Cas9 mRNA is modified before introduced into the neural stem cells. In some aspects of the disclosure, the sgRNA is modified before introduced into the neural stem cells.

In some aspects of the disclosure, at least 1% of the resulting genetically modified neural stem cells express the transgene. In some aspects of the disclosure, at least 0.5%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of the resulting genetically modified neural stem cells express the said transgene.

The genetically modified human neural stem cells obtained by methods described herein exhibit one or more advantageous properties, including maintaining of the "sternness" of the neural stem cells. This is particularly significant given the fact that the cells are cultured (sometimes extensively) and subjected to multiple steps of manipulations in the process of preparing genetically modified human neural stem cells. In some aspects of the disclosure, the resulting genetically modified neural stem cells are able to express SOX2. In some aspects of the disclosure, the resulting genetically modified neural stem cells are able to engraft, or migrate when transplanted into the brain of an animal model. In some aspects of the disclosure, the resulting genetically modified neural stem cells are able to differentiate into oligodendrocytes, neurons, or astrocytes. In some aspects of the disclosure, the resulting genetically modified neural stem cells are able to differentiate into neuronal lineage in the olfactory bulb. In some aspects of the disclosure, the resulting genetically modified neural stem cells are able to self-renew while differentiating into neuron, astrocyte and oligodendrocyte lineages in long-term. In some aspects of the disclosure, the resulting genetically modified neural stem cells have at least one of the above characteristics over at least 1, 2, 3, 4, 5, 6, 7, 8, 9, 10, 11, or 12 cell culture passages. In some aspects of the disclosure, the resulting genetically modified neural stem cells have at least two or more of the above characteristics. In some aspects of the disclosure, the resulting genetically modified neural stem cells have at least two or more of the above characteristics over at least 1, 2, 3, 4, 5, 6, 7, 8, 9, 10, 11, or 12 cell culture passages.

In some aspects of the disclosure, the resulting genetically modified neural stem cells are able to stably express the transgene at least after 3 passages. In some aspects of the disclosure, the resulting genetically modified neural stem cells are able to stably express the transgene over at least 1, 2, 4, 5, 6, or 7 cell culture passages. In some aspects of the disclosure, at least 1%, 5%, 10%, 20%, 30%, 40%, 50%, 55%, 60%, 65%, 70%, 75%, 80%, 85%, 90%, 95% of the resulting genetically modified neural stem cell population express the transgene.

The methods described herein in some aspects of the disclosure make use of selectable markers. In some aspects of the disclosure, the selectable marker is a protein not normally expressed on a cell of the central nervous system. In some aspects of the disclosure, the selectable marker is not expressed on the cell surface (for example GFP). In some aspects of the disclosure, the selectable marker is a cell surface marker.

In some aspects of the disclosure, the cell surface marker is CD8, CD19, CD20, or truncated fragments thereof. In some aspects of the disclosure, the cell surface marker is a CD8 alpha cell surface marker. In some aspects of the disclosure, the cell surface marker is a CD20 cell surface marker. In some aspects of the disclosure, the cell surface marker is a truncated CD8 cell surface marker. In some aspects of the disclosure, the cell surface marker is a truncated CD19 cell surface marker. In some aspects of the disclosure, the cell surface marker is a truncated CD20 cell surface marker. In some aspects of the disclosure, the two or more cell surface markers are used to facilitate selection of the genetically modified neural stem cells.

In some aspects of the disclosure, the cell surface marker is a chemokine receptor. In some aspects of the disclosure, the cell surface marker is a cytokine receptor. In some aspects of the disclosure, the cell surface marker is a cell surface enzyme (such as isomethy protease, metalloproteinase or neprilysin) and chemokine receptors.

In some aspects of the disclosure, the neural stem cells are cultured as self-adherent complexes of cells, (e.g. by forming clusters known as neurospheres). In some aspects of the disclosure, the neural stem cells are cultured in adherent cultures, such as monolayer cultures. In some aspects of the disclosure, the neural stem cells are cultured on substrate-coated tissue culture plates. In some aspects of the disclosure, the neural stem cells are cultured in suspension culture. In some aspects of the disclosure, the neural stem cells are cultured in a condition with at least one of the supplements selected from the group consisting of N2, heparin, N-acetylcysteine, fibroblast growth factor 2, epidermal growth factor, and leukemia inhibitory factor.

In some aspects of the disclosure, the neural stem cells are cultured for a period of time (such as 30 days) before the said neural stem cells are introduced the donor template and/or the DNA nuclease (or the nucleotide sequence encoding the DNA nuclease). In some aspects of the disclosure, the culturing period is at least 5, 10, 15, 20, 25, 30, 35, 40, 45, 50, 55, 60, 65, 70, 75, 80, 85, 90, 95, or 100 days before the donor template and/or the DNA nuclease (or the nucleotide sequence encoding the DNA nuclease) being introduced into said neural stem cells.

In some aspects of the disclosure, the culturing time period is long enough for the donor template to be diluted out of the proliferating neural stem cells. In some aspects of the disclosure, the neural stem cells are cultured for least about 5, 10, 15, 20, 25, 30, 35, 40, 45, 50, 55, 60, 65, 70, 75, 80, 85, 90, 95, or 100 days after the donor template and/or the DNA nuclease (or the nucleotide sequence encoding the DNA nuclease) is introduced into said neural stem cells. In some aspects of the disclosure, the neural stem cells are cultured for at least about 1, 2, 3, 4, 5, 6, 7, 8, 9, 10, 11, or 12 cell culture passages after the donor template and/or the DNA nuclease (or the nucleotide sequence encoding the DNA nuclease) is introduced into the neural stem cells.

In some aspects of the disclosure, the neural stem cells are derived from human brain or brains. In some aspects of the disclosure, the neural stem cells are derived from somatic stem cells or pluripotent stem cells, or are derived by direct reprogramming from a somatic cell population or populations. In some aspects of the disclosure, the neural stem cells are human neural stem cells. In some aspects of the disclosure, the neural stem cells are derived from second trimester human fetal brain. In some aspects of the disclosure, the neural stem cells are isolated based on the expression of a combination of markers, such as C133 and CD24. In some aspects of the disclosure, the neural stem cells are derived from CD133 positive CD24 negative to low human brain cells. In some aspects of the disclosure, the neural stem cells are isolated based on the expression of a combination of two or more markers selected from the group consisting CD133, CD24, CD49f, CD29, and CD15.

Also provided herein are genetically modified neural stem cells made by any one or more methods described herein. The genetically modified human neural stem cells are further described below in more detail.

Another aspect of the disclosure provides a population of genetically modified human neural stem cells comprising a transgene, wherein the transgene is inserted into a safe harbor locus.

In some aspects of the disclosure, the genetically modified human neural stem cells are derived from a somatic stem cell or a pluripotent stem cell, or are derived by direct reprogramming from a somatic cell population. In some aspects of the disclosure the genetically modified neural stem cells are derived from human brain or human brains. In some aspects of the disclosure, the genetically modified neural stem cells are isolated based on the expression of a combination of markers, for example, CD133 positive CD24 negative to low cells. In some aspects of the disclosure, the neural stem cells are isolated based on the expression of a combination of two or more markers selected from the group consisting CD133, CD24, CD49f, CD29, and CD15.

In some aspects of the disclosure, the genetically modified neural stem cells express SOX2 over at least 1, 2, 3, 4, 5, 6, 7, 8, 9, 10, 11, or 12 cell culture passages. In some aspects of the disclosure, the genetically modified neural stem cells are able to differentiate into oligodendrocytes. In some aspects of the disclosure, the genetically modified neural stem cells are able to differentiate into myelin-producing oligodendrocytes. In some aspects of the disclosure, the genetically modified neural stem cells are able to differentiate into neurons. In some aspects of the disclosure, the genetically modified neural stem cells are able to differentiate into astrocytes. In some aspects of the disclosure, the genetically modified neural stem cells are able to differentiate into two or more neural cell types such as neurons, astrocytes, and oligodendrocytes. In some aspects of the disclosure, the genetically modified neural stem cells are able to engraft when transplanted into the brain of an animal model. In some aspects of the disclosure, the genetically modified neural stem cells are able to migrate when transplanted into the brain of an animal model. In some aspects of the disclosure, the genetically modified neural stem cells are able to differentiate into neuronal lineage in the olfactory bulb. In some aspects of the disclosure, the resulting genetically modified neural stem cells are able to self-renew while differentiating into neuron, astrocyte and oligodendrocyte lineages in long-term. In some aspects of the disclosure, the genetically modified neural stem cells have two or more characteristics as discussed above, for example, expressing SOX2 over at least 1, 2, 3, 4, 5, 6, 7 , 8, 9, 10, 11, or 12 cell culture passages, and exhibiting at least one of the functions which comprise migrating or engrafting when transplanted into the brain of an animal model, or differentiating into at least one neural cell types such as neurons, astrocytes, and oligodendrocytes.

In some aspects of the disclosure, at least 1%, 5%, 10%, 20%, 30%, 40%, 50%, 55%, 60%, 65%, 70%, 75%, 80%, 85%, 90%, 95% of the genetically modified neural stem cell population express the transgene. In some aspects of the disclosure, the genetically modified neural stem cells are able to stably express the transgene over at least 1, 2, 3, 4, 5, 6, 7 cell culture passages.

In some aspects of the disclosure, the genetically modified neural stem cells further express a selectable marker. In some aspects of the disclosure, the selectable marker comprises a marker that is not expressed on a cell of the central nervous system. In some aspects of the disclosure, the selectable marker is not expressed on the cell surface (i.e. GFP).

In some aspects of the disclosure, the genetically modified neural stem cells express a cell surface marker, wherein the cell surface marker can be used as a selectable marker. In some aspects of the disclosure, the genetically modified neural stem cells express a CD8 cell surface marker. In some aspects of the disclosure, the genetically modified neural stem cells express a CD8 alpha cell surface marker. In some aspects of the disclosure, the genetically modified neural stem cells express a CD 19 cell surface marker. In some aspects of the disclosure, the genetically modified neural stem cells express a CD20 cell surface marker. In some aspects of the disclosure, the genetically modified neural stem cells express a truncated CD8 cell surface marker. In some aspects of the disclosure, the genetically modified neural stem cells express a truncated CD19 cell surface marker. In some aspects of the disclosure, the genetically modified neural stem cells express a truncated CD20 cell surface marker. In some aspects of the disclosure, the genetically modified neural stem cells express two or more cell surface markers.

In some aspects of the disclosure, the genetically modified neural stem cells express a cell surface marker, wherein the cell surface marker is a chemokine receptor. In some aspects of the disclosure, the genetically modified neural stem cells express a cell surface marker, wherein the cell surface marker is a cytokine receptor. In some aspects of the disclosure, the genetically modified neural stem cells express a cell surface marker, wherein the cell surface marker is a cell surface enzyme (such as isomethy protease, or metalloproteinase or neprilysin) and chemokine receptors.

In some aspects of the disclosure, the safe harbor gene is IL2Rγ. In some aspects of the disclosure, the safe harbor gene is CCR5. In some aspects of the disclosure, the safe harbor gene is HBB. In some aspects of the disclosure, two or more safe harbor genes are used to insert one or more transgenes. In some aspects of the disclosure, two or more safe harbor genes are selected from the group consisting IL2Rγ, and HBB.

In some aspects of the disclosure, the transgene comprises a promoter sequence. In some aspects of the disclosure, transgene comprises a heterologous promoter. In some aspects of the disclosure, the heterologous promoter is operably linked to a transgene. In some aspects of the disclosure, the heterologous promoter is operably linked to a transgene, wherein the heterologous promoter and the transgene are located in the same safe harbor gene. In some aspects of the disclosure, the heterologous promoter is operably linked to a transgene, wherein the heterologous promoter and the transgene are located in different safe harbor genes. In some aspects of the disclosure, the heterologous promoter of the donor template comprises an inducible promoter system. In some aspects of the disclosure, the heterologous promoter of the donor template comprises a cell-specific promoter. In some aspects of the disclosure, the genetically modified neural stem cells comprise one or more transgenes that comprise one or more promoters. In some aspects of the disclosure, the promoter or promoters can be a U6 RNA polymerase III promoter, a transcriptional control element, enhancer, U6 termination sequence, one or more nuclear localization signals, etc.

In some aspects of the disclosure, the transgene cassette described herein is inserted into the safe harbor locus such that the transgene is operably linked to an endogenous promoter.

In some aspects of the disclosure, the genetically modified neural stem cells express a transgene, wherein the transgene encodes a protein associated with a genetic disorder of the central nervous system. In some aspects of the disclosure, the genetically modified neural stem cells express a transgene selected from the group consisting of GALC (Krabbe disease), ABCD1 (adrenoleukodystrophy), GFAP (Alexander disease), CYP27A1 (cerebrotendineous xanthomatosis), ARSA (metachromatic leukodystrophy), PLP1 (Pelizaeus-Merzbacher disease), ASPA (Canavan disease), EIF-2B (leukoencephalopathy with vanishing white matter), PHYH (Refsum disease 1), PEX7 (Refsum disease 2), PPT1 (infantile neuronal ceroid lipofuscinosis (NCL)), TPP1 (late infantile NCL), CLN3 (juvenile NCL), CLN6 (adult NCL), CLN5 (Finnish late infantile variant NCL), CLN6 (late infantile variant NCL), MSFD8 (ceroid lipofuscinosis, neuronal, 7), CLN8 (ceroid lipofuscinosis, neuronal, 8), CTSD (ceroid lipofuscinosis, neuronal, 10), UBE3A (Angelman syndrome), POLG (Alpers' Disease), TAZ (Barth Syndrome), GLA (Fabry disease), SLC20A2 (Fahr's syndrome), PDE (retinitis pigmentosa), SMN1 (spinal muscular atrophy), IKBKAP (familial dysautonomia), MeCP2 (Rett syndrome), CACNA1C (Timothy syndrome), ATXN3 (Machado-Joseph disease), and RPE65 (Leber congenital amaurosis), USH2A (retinitis pigmentosa), RPGR (retinitis pigmentosa), RP2(retinitis pigmentosa), ABCA4 (Stargardt), RS-1 (X-linked retinoschisis). In some aspects of the disclosure, the protein is secreted by the genetically modified human neural stem cell.

In some aspects of the disclosure, the genetically modified neural stem cells express a transgene encoding a neuroprotective or neuroregenerative protein, a variant thereof, a fragment thereof, or a peptide mimetic thereof. In some aspects of the disclosure, the genetically modified neural stem cells express a transgene encoding a neuroprotective or neurodegenerative protein which is selected from the group consisting of brain-derived neurotrophic factor (BDNF), glial-derived neurotrophic factor (GDNF), insulin-like growth factor 1 (IGF1), insulin-like growth factor 2 (IGF2), nerve growth factor (NGF), neurotrophin-2 (NT-2), neurotrophin-3 (NT-3) neurotrophin-4/5 (NT-4/5), neurotrophin-6, conserved dopamine neurotrophic factor (CDNF), ciliary neurotrophic factor (CNTF), epidermal growth factor (EGF), a fibroblast growth factor (FGF), a bone morphogenetic protein (BMP), vascular endothelial growth factor (VEGF), granulocyte colony-stimulating factor (G-CSF), colony-stimulating factor (CSF), interferon-β (IFN-β), tumor necrosis factor-α (TNFa), tissue plasminogen activator (tPA), neurturin, persephin, artemin, neuropeptide Y (NPY), an ephrin, a semaphorin, other neuropoeitic factors, other neurotrophic factors, and a combination thereof. In some aspects of the disclosure, the genetically modified neural stem cells express a transgene encoding a neuroprotective or neurodegenerative protein, wherein the neuroprotective or neuroregenerative protein is a secreted protein. In some aspects of the disclosure, the transgene encodes Bcl-2. In some aspects of the disclosure, the transgene encodes a telomerase. In some aspects of the disclosure, the transgene encodes a protein that can improve survival or rejuvenate the neural stem cell to facilitate long-term survival and/or discovery of therapeutic molecules.

In some aspects of the disclosure, the genetically modified neural stem cells express two or more transgenes. In some aspects of the disclosure, the genetically modified neural stem cells produce a RNA that is produced from a transgene. In some aspects of the disclosure, the genetically modified neural stem cells produce an siRNA that is produced from a transgene. In some aspects of the disclosure, the genetically modified neural stem cells produce a RNA that is produced from one transgene, and express another transgene as a protein. In some aspects of the disclosure, the genetically modified neural stem cells produce an siRNA that is produced from one transgene, and express another transgene as a protein.

In some aspects of the disclosure, the genetically modified neuronal stem cells can be administered into a human subject to prevent or alleviate one or more symptoms of the neurodegenerative disease or the neurological injury. In some aspects of the disclosure, the genetically modified neuronal stem cells can be administered to the human subject to prevent or alleviate one or more symptoms of the neurodegenerative disease, wherein the neurodegenerative disease is selected from the group consisting of a leukodystrophy, neuronal ceroid lipofuscinosis, age-related macular degeneration, Alzheimer's disease, Parkinson's disease, Huntington's disease, amyotrophic lateral sclerosis, and retinal degenerative disease.

In some aspects of the disclosure, the genetically modified human neural stem cells have two or more (for example, 3, 4, 5, 6, 7, or more) characteristics discussed above.

### A. Nuclease-Mediated Genome Editing

Aspects of the disclosure includes using a DNA nuclease such as an engineered (*e*.*g*., programmable or targetable) DNA nuclease to induce genome editing of a target DNA sequence such as a safe harbor gene. Any suitable DNA nuclease can be used including, but not limited to, CRISPR-associated protein (Cas) nucleases, zinc finger nucleases (ZFNs), transcription activator-like effector nucleases (TALENs), meganucleases, other endo- or exo-nucleases, variants thereof, fragments thereof, and combinations thereof.

In some aspects of the disclosure, a nucleotide sequence encoding the DNA nuclease is present in a recombinant expression vector. In certain instances, the recombinant expression vector is a viral construct, *e*.*g*., a recombinant adeno-associated virus construct, a recombinant adenoviral construct, a recombinant lentiviral construct, *etc.* For example, viral vectors can be based on vaccinia virus, poliovirus, adenovirus, adeno-associated virus, SV40, herpes simplex virus, human immunodeficiency virus, and the like. A retroviral vector can be based on Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, mammary tumor virus, and the like. Useful expression vectors are known to those of skill in the art, and many are commercially available. The following vectors are provided by way of example for eukaryotic host cells: pXT1, pSG5, pSVK3, pBPV, pMSG, and pSVLSV40. However, any other vector may be used if it is compatible with the host cell. For example, useful expression vectors containing a nucleotide sequence encoding a Cas9 polypeptide are commercially available from, *e*.*g*., Addgene, Life Technologies, Sigma-Aldrich, and Origene.

Depending on the target cell/expression system used, any of a number of transcription and translation control elements, including promoter, transcription enhancers, transcription terminators, and the like, may be used in the expression vector. Useful promoters can be derived from viruses, or any organism, *e*.*g*., prokaryotic or eukaryotic organisms. Suitable promoters include, but are not limited to, the SV40 early promoter, mouse mammary tumor virus long terminal repeat (LTR) promoter; adenovirus major late promoter (Ad MLP); a herpes simplex virus (HSV) promoter, a cytomegalovirus (CMV) promoter such as the CMV immediate early promoter region (CMVIE), a rous sarcoma virus (RSV) promoter, a human U6 small nuclear promoter (U6), an enhanced U6 promoter, a human H1 promoter (H1), *etc.*

In other aspects of the disclosure, a nucleotide sequence encoding the DNA nuclease is present as an RNA (*e.g.,* mRNA). The RNA can be produced by any method known to one of ordinary skill in the art. As non-limiting examples, the RNA can be chemically synthesised or *in vitro* transcribed. In certain aspects of the disclosure, the RNA comprises an mRNA encoding a Cas nuclease such as a Cas9 polypeptide or a variant thereof. For example, the Cas9 mRNA can be generated through *in vitro* transcription of a template DNA sequence such as a linearised plasmid containing a Cas9 open reading frame (ORF). The Cas9 ORF can be codon optimised for expression in mammalian systems. In some instances, the Cas9 mRNA encodes a Cas9 polypeptide with an N- and/or C-terminal nuclear localization signal (NLS). In other instances, the Cas9 mRNA encodes a C-terminal HA epitope tag. In yet other instances, the Cas9 mRNA is capped, polyadenylated, and/or modified with 5-methylcytidine. Cas9 mRNA is commercially available from, *e*.*g*., TriLink BioTechnologies, Sigma-Aldrich, and Thermo Fisher Scientific.

### 1. CRISPR/Cas system

The CRISPR (Clustered Regularly Interspaced Short Palindromic Repeats)/Cas (CRISPR-associated protein) nuclease system is an engineered nuclease system based on a bacterial system that can be used for genome engineering. It is based on part of the adaptive immune response of many bacteria and archaea. When a virus or plasmid invades a bacterium, segments of the invader's DNA are converted into CRISPR RNAs (crRNA) by the "immune" response. The crRNA then associates, through a region of partial complementarity, with another type of RNA called tracrRNA to guide the Cas (*e.g.,* Cas9) nuclease to a region homologous to the crRNA in the target DNA called a "protospacer." The Cas (*e.g.,* Cas9) nuclease cleaves the DNA to generate blunt ends at the double-strand break at sites specified by a 20-nucleotide guide sequence contained within the crRNA transcript. The Cas (*e.g.,* Cas9) nuclease can require both the crRNA and the tracrRNA for site-specific DNA recognition and cleavage. This system has now been engineered such that the crRNA and tracrRNA can be combined into one molecule (the "single guide RNA" or "sgRNA"), and the crRNA equivalent portion of the single guide RNA can be engineered to guide the Cas (*e.g.,* Cas9) nuclease to target any desired sequence (*see, e.g.,* Jinek et al. (2012) Science 337:816-821; Jinek et al. (2013) eLife 2:e00471; Segal (2013) eLife 2:e00563). Thus, the CRISPR/Cas system can be engineered to create a double-strand break at a desired target in a genome of a cell, and harness the cell's endogenous mechanisms to repair the induced break by homology-directed repair (HDR) or nonhomologous end-joining (NHEJ).

In some aspects of the disclosure, the Cas nuclease has DNA cleavage activity. The Cas nuclease can direct cleavage of one or both strands at a location in a target DNA sequence. For example, the Cas nuclease can be a nickase having one or more inactivated catalytic domains that cleaves a single strand of a target DNA sequence.

Non-limiting examples of Cas nucleases include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, variants thereof, mutants thereof, and derivatives thereof. There are three main types of Cas nucleases (type I, type II, and type III), and 10 subtypes including 5 type I, 3 type II, and 2 type III proteins (*see, e.g.,* Hochstrasser and Doudna, Trends Biochem Sci, 2015:40(1):58-66). Type II Cas nucleases include Cas1, Cas2, Csn2, and Cas9. These Cas nucleases are known to those skilled in the art. For example, the amino acid sequence of the *Streptococcus pyogenes* wild-type Cas9 polypeptide is set forth, *e.g.*, in NBCI Ref. Seq. No. NP_269215, and the amino acid sequence of *Streptococcus thermophilus* wild-type Cas9 polypeptide is set forth, *e.g.,* in NBCI Ref. Seq. No. WP_011681470. CRISPR-related endonucleases that are useful in the discloure are described, *e*.*g*., in U.S. Application Publication Nos. 2014/0068797, 2014/0302563, and 2014/0356959.

Cas nucleases, *e*.*g*., Cas9 polypeptides, can be derived from a variety of bacterial species including, but not limited to, *Veillonella atypical, Fusobacterium nucleatum, Filifactor alocis, Solobacterium moorei, Coprococcus catus, Treponema denticola, Peptoniphilus duerdenii, Catenibacterium mitsuokai, Streptococcus mutans, Listeria innocua, Staphylococcus pseudintermedius, Acidaminococcus intestine, Olsenella uli, Oenococcus kitaharae, Bifidobacterium bifidum, Lactobacillus rhamnosus, Lactobacillus gasseri, Finegoldia magna, Mycoplasma mobile, Mycoplasma gallisepticum, Mycoplasma ovipneumoniae, Mycoplasma canis, Mycoplasma synoviae, Eubacterium rectale, Streptococcus thermophilus, Eubacterium dolichum, Lactobacillus coryniformis* subsp. *Torquens, Ilyobacter polytropus, Ruminococcus albus, Akkermansia muciniphila, Acidothermus cellulolyticus, Bifidobacterium longum, Bifidobacterium dentium, Corynebacterium diphtheria, Elusimicrobium minutum, Nitratifractor salsuginis, Sphaerochaeta globus, Fibrobacter succinogenes subsp. Succinogenes, Bacteroides fragilis, Capnocytophaga ochracea, Rhodopseudomonas palustris, Prevotella micans, Prevotella ruminicola, Flavobacterium columnare, Aminomonas paucivorans, Rhodospirillum rubrum, Candidatus Puniceispirillum marinum, Verminephrobacter eiseniae, Ralstonia syzygii, Dinoroseobacter shibae, Azospirillum, Nitrobacter hamburgensis, Bradyrhizobium, Wolinella succinogenes, Campylobacter jejuni* subsp. *Jejuni, Helicobacter mustelae, Bacillus cereus, Acidovorax ebreus, Clostridium perfringens, Parvibaculum lavamentivorans, Roseburia intestinalis, Neisseria meningitidis, Pasteurella multocida subsp. Multocida, Sutterella wadsworthensis, proteobacterium, Legionella pneumophila, Parasutterella excrementihominis, Wolinella succinogenes,* and *Francisella novicida.*

"Cas9" refers to an RNA-guided double-stranded DNA-binding nuclease protein or nickase protein. Wild-type Cas9 nuclease has two functional domains, e.g., RuvC and HNH, that cut different DNA strands. Cas9 can induce double-strand breaks in genomic DNA (target DNA) when both functional domains are active. The Cas9 enzyme can comprise one or more catalytic domains of a Cas9 protein derived from bacteria belonging to the group consisting of *Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor,* and *Campylobacter.* In some aspects of the disclosure, the Cas9 is a fusion protein, *e*.*g*., the two catalytic domains are derived from different bacteria species.

Useful variants of the Cas9 nuclease can include a single inactive catalytic domain, such as a RuvC⁻ or HNH⁻ enzyme or a nickase. A Cas9 nickase has only one active functional domain and can cut only one strand of the target DNA, thereby creating a single strand break or nick. In some aspects of the disclosure, the mutant Cas9 nuclease having at least a D10A mutation is a Cas9 nickase. In other aspects of the disclosure, the mutant Cas9 nuclease having at least a H840A mutation is a Cas9 nickase. Other examples of mutations present in a Cas9 nickase include, without limitation, N854A and N863A. A double-strand break can be introduced using a Cas9 nickase if at least two DNA-targeting RNAs that target opposite DNA strands are used. A double-nicked induced double-strand break can be repaired by NHEJ or HDR (Ran et al., 2013, Cell, 154:1380-1389). This gene editing strategy favors HDR and decreases the frequency of indel mutations at off-target DNA sites. Non-limiting examples of Cas9 nucleases or nickases are described in, for example, U.S. Patent No. 8,895,308; 8,889,418; and 8,865,406 and U.S. Application Publication Nos. 2014/0356959, 2014/0273226 and 2014/0186919. The Cas9 nuclease or nickase can be codon-optimised for the target cell or target organism.

In some aspects of the disclosure, the Cas nuclease can be a Cas9 polypeptide that contains two silencing mutations of the RuvC1 and HNH nuclease domains (D10A and H840A), which is referred to as dCas9 (Jinek et al., Science, 2012, 337:816-821; Qi et al., Cell, 152(5):1173-1183). In one embodiment, the dCas9 polypeptide from *Streptococcus pyogenes* comprises at least one mutation at position D10, G12, G17, E762, H840, N854, N863, H982, H983, A984, D986, A987 or any combination thereof. Descriptions of such dCas9 polypeptides and variants thereof are provided in, for example, International Patent Publication No. WO 2013/176772. The dCas9 enzyme can contain a mutation at D10, E762, H983 or D986, as well as a mutation at H840 or N863. In some instances, the dCas9 enzyme contains a D10A or D10N mutation. Also, the dCas9 enzyme can include a H840A, H840Y, or H840N. In some aspects of the disclosure, the dCas9 enzyme comprises D10A and H840A; D10A and H840Y; D10A and H840N; D10N and H840A; D10N and H840Y; or D10N and H840N substitutions. The substitutions can be conservative or non-conservative substitutions to render the Cas9 polypeptide catalytically inactive and able to bind to target DNA.

For genome editing methods, the Cas nuclease can be a Cas9 fusion protein such as a polypeptide comprising the catalytic domain of the type IIS restriction enzyme, FokI, linked to dCas9. The FokI-dCas9 fusion protein (fCas9) can use two guide RNAs to bind to a single strand of target DNA to generate a double-strand break.

In some aspects of the disclosure, the Cas nuclease can be a high-fidelity or enhanced specificity Cas9 polypeptide variant with reduced off-target effects and robust on-target cleavage. Non-limiting examples of Cas9 polypeptide variants with improved on-target specificity include the SpCas9 (K855A), SpCas9 (K810A/K1003A/R1060A) [also referred to as eSpCas9(1.0)], and SpCas9 (K848A/K1003A/R1060A) [also referred to as eSpCas9(1.1)] variants described in Slaymaker et al., Science, 351(6268):84-8 (2016), and the SpCas9 variants described in Kleinstiver et al., Nature, 529(7587):490-5 (2016) containing one, two, three, or four of the following mutations: N497A, R661A, Q695A, and Q926A *(e.g.,* SpCas9-HF1 contains all four mutations).

### 2. Zinc finger nucleases (ZFNs)

"Zinc finger nucleases" or "ZFNs" are a fusion between the cleavage domain of FokI and a DNA recognition domain containing 3 or more zinc finger motifs. The heterodimerization at a particular position in the DNA of two individual ZFNs in precise orientation and spacing leads to a double-strand break in the DNA. In some cases, ZFNs fuse a cleavage domain to the C-terminus of each zinc finger domain. In order to allow the two cleavage domains to dimerize and cleave DNA, the two individual ZFNs bind opposite strands of DNA with their C-termini at a certain distance apart. In some cases, linker sequences between the zinc finger domain and the cleavage domain requires the 5' edge of each binding site to be separated by about 5-7 bp. Exemplary ZFNs that are useful in some aspects of the- disclosure include, but are not limited to, those described in Urnov et al., Nature Reviews Genetics, 2010, 11:636-646; Gaj et al., Nat Methods, 2012, 9(8):805-7; U.S. Patent Nos. 6,534,261; 6,607,882; 6,746,838; 6,794,136; 6,824,978; 6,866,997; 6,933,113; 6,979,539; 7,013,219; 7,030,215; 7,220,719; 7,241,573; 7,241,574; 7,585,849; 7,595,376; 6,903,185; 6,479,626; and U.S. Application Publication Nos. 2003/0232410 and 2009/0203140.

ZFNs can generate a double-strand break in a target DNA, resulting in DNA break repair which allows for the introduction of gene modification. DNA break repair can occur via non-homologous end joining (NHEJ) or homology-directed repair (HDR). In HDR, a donor DNA repair template that contains homology arms flanking sites of the target DNA can be provided.

In some aspects of the disclosure, a ZFN is a zinc finger nickase which can be an engineered ZFN that induces site-specific single-strand DNA breaks or nicks, thus resulting in HDR. Descriptions of zinc finger nickases are found, *e.g.,* in Ramirez et al., Nucl Acids Res, 2012, 40(12):5560-8; Kim et al., Genome Res, 2012, 22(7):1327-33.

### 3. TALENs

"TALENs" or "TAL-effector nucleases" are engineered transcription activator-like effector nucleases that contain a central domain of DNA-binding tandem repeats, a nuclear localization signal, and a C-terminal transcriptional activation domain. In some instances, a DNA-binding tandem repeat comprises 33-35 amino acids in length and contains two hypervariable amino acid residues at positions 12 and 13 that can recognize one or more specific DNA base pairs. TALENs can be produced by fusing a TAL effector DNA binding domain to a DNA cleavage domain. For instance, a TALE protein may be fused to a nuclease such as a wild-type or mutated FokI endonuclease or the catalytic domain of FokI. Several mutations to FokI have been made for its use in TALENs, which, for example, improve cleavage specificity or activity. Such TALENs can be engineered to bind any desired DNA sequence.

TALENs can be used to generate gene modifications by creating a double-strand break in a target DNA sequence, which in turn, undergoes NHEJ or HDR. In some cases, a single-stranded donor DNA repair template is provided to promote HDR.

Detailed descriptions of TALENs and their uses for gene editing are found, *e.g.,* in U.S. Patent Nos. 8,440,431; 8,440,432; 8,450,471; 8,586,363; and 8,697,853; Scharenberg et al., Curr Gene Ther, 2013, 13(4):291-303; Gaj et al., Nat Methods, 2012, 9(8):805-7; Beurdeley et al., Nat Commun, 2013, 4:1762; and Joung and Sander, Nat Rev Mol Cell Biol, 2013, 14(1):49-55.

### 4. Meganucleases

"Meganucleases" are rare-cutting endonucleases or homing endonucleases that can be highly specific, recognizing DNA target sites ranging from at least 12 base pairs in length, *e.g.*, from 12 to 40 base pairs or 12 to 60 base pairs in length. Meganucleases can be modular DNA-binding nucleases such as any fusion protein comprising at least one catalytic domain of an endonuclease and at least one DNA binding domain or protein specifying a nucleic acid target sequence. The DNA-binding domain can contain at least one motif that recognizes single- or double-stranded DNA. The meganuclease can be monomeric or dimeric.

In some instances, the meganuclease is naturally-occurring (found in nature) or wild-type, and in other instances, the meganuclease is non-natural, artificial, engineered, synthetic, rationally designed, or man-made. In certain aspects of the disclosure, the meganuclease includes an I-CreI meganuclease, I-CeuI meganuclease, I-MsoI meganuclease, I-SceI meganuclease, variants thereof, mutants thereof, and derivatives thereof.

Detailed descriptions of useful meganucleases and their application in gene editing are found, *e.g.,* in Silva et al., Curr Gene Ther, 2011, 11(1):11-27; Zaslavoskiy et al., BMC Bioinformatics, 2014, 15:191; Takeuchi et al., Proc Natl Acad Sci USA, 2014, 111(11):4061-4066, and U.S. Patent Nos. 7,842,489; 7,897,372; 8,021,867; 8,163,514; 8,133,697; 8,021,867; 8,119,361; 8,119,381; 8,124,36; and 8,129,134.

### B. Donor Template for HDR

Provided herein is a donor template *(e.g.,* a recombinant donor repair template) comprising: (i) a transgene cassette comprising a transgene (for example, a transgene operably linked to a promoter, for example, a heterologous promoter); and (ii) two homology arms that flank the transgene cassette and are homologous to portions of a safe harbor gene at either side of a DNA nuclease (*e.g*., Cas9 nuclease) cleavage site. The donor template can further comprise a selectable marker, a detectable marker, and/or a purification marker.

In some aspects of the disclosure, the homology arms are the same length. In other aspects of the disclosure, the homology arms are different lengths. The homology arms can be at least about 10 base pairs (bp), *e.g.,* at least about 10 bp, 15 bp, 20 bp, 25 bp, 30 bp, 35 bp, 45 bp, 55 bp, 65 bp, 75 bp, 85 bp, 95 bp, 100 bp, 150 bp, 200 bp, 250 bp, 300 bp, 350 bp, 400 bp, 450 bp, 500 bp, 550 bp, 600 bp, 650 bp, 700 bp, 750 bp, 800 bp, 850 bp, 900 bp, 950 bp, 1000 bp, 1.1 kilobases (kb), 1.2 kb, 1.3 kb, 1.4 kb, 1.5 kb, 1.6 kb, 1.7 kb, 1.8 kb, 1.9 kb, 2.0 kb, 2.1 kb, 2.2 kb, 2.3 kb, 2.4 kb, 2.5 kb, 2.6 kb, 2.7 kb, 2.8 kb, 2.9 kb, 3.0 kb, 3.1 kb, 3.2 kb, 3.3 kb, 3.4 kb, 3.5 kb, 3.6 kb, 3.7 kb, 3.8 kb, 3.9 kb, 4.0 kb, or longer. The homology arms can be about 10 bp to about 4 kb, *e.g.,* about 10 bp to about 20 bp, about 10 bp to about 50 bp, about 10 bp to about 100 bp, about 10 bp to about 200 bp, about 10 bp to about 500 bp, about 10 bp to about 1 kb, about 10 bp to about 2 kb, about 10 bp to about 4 kb, about 100 bp to about 200 bp, about 100 bp to about 500 bp, about 100 bp to about 1 kb, about 100 bp to about 2 kb, about 100 bp to about 4 kb, about 500 bp to about 1 kb, about 500 bp to about 2 kb, about 500 bp to about 4 kb, about 1 kb to about 2 kb, about 1 kb to about 2 kb, about 1 kb to about 4 kb, or about 2 kb to about 4 kb.

The donor template can be cloned into an expression vector. Conventional viral and non-viral based expression vectors known to those of ordinary skill in the art can be used.

### C. DNA-targeting RNA

In some aspects of the disclosure, the methods further comprise introducing into a human neural stem cell a guide nucleic acid, *e.g.,* DNA-targeting RNA *(e.g.,* a single guide RNA (sgRNA) or a double guide nucleic acid) or a nucleotide sequence encoding the guide nucleic acid (*e.g*., DNA-targeting RNA).

The DNA-targeting RNA (*e.g.,* sgRNA) can comprise a first nucleotide sequence that is complementary to a specific sequence within a target DNA (*e.g.,* a guide sequence) and a second nucleotide sequence comprising a protein-binding sequence that interacts with a DNA nuclease (*e.g.,* Cas9 nuclease) or a variant thereof (*e.g.,* a scaffold sequence or tracrRNA). The guide sequence ("first nucleotide sequence") of a DNA-targeting RNA can comprise about 10 to about 2000 nucleic acids, for example, about 10 to about 100 nucleic acids, about 10 to about 500 nucleic acids, about 10 to about 1000 nucleic acids, about 10 to about 1500 nucleic acids, about 10 to about 2000 nucleic acids, about 50 to about 100 nucleic acids, about 50 to about 500 nucleic acids, about 50 to about 1000 nucleic acids, about 50 to about 1500 nucleic acids, about 50 to about 2000 nucleic acids, about 100 to about 500 nucleic acids, about 100 to about 1000 nucleic acids, about 100 to about 1500 nucleic acids, about 100 to about 2000 nucleic acids, about 500 to about 1000 nucleic acids, about 500 to about 1500 nucleic acids, about 500 to about 2000 nucleic acids, about 1000 to about 1500 nucleic acids, about 1000 to about 2000 nucleic acids, or about 1500 to about 2000 nucleic acids at the 5' end that can direct the DNA nuclease (*e.g*., Cas9 nuclease) to the target DNA site (*e.g.,* safe harbor gene sequence) using RNA-DNA complementarity base pairing. In some aspects of the disclosure, the guide sequence of a DNA-targeting RNA comprises about 100 nucleic acids at the 5' end that can direct the DNA nuclease (*e.g*., Cas9 nuclease) to the target DNA site (*e.g.,* safe harbor gene sequence) using RNA-DNA complementarity base pairing. In some aspects of the disclosure, the guide sequence comprises 20 nucleic acids at the 5' end that can direct the DNA nuclease (*e.g*., Cas9 nuclease) to the target DNA site (*e.g.,* safe harbor gene sequence) using RNA-DNA complementarity base pairing. In other aspects of the disclosure, the guide sequence comprises less than 20, *e.g.,* 19, 18, 17, 16, 15 or less, nucleic acids that are complementary to the target DNA site (*e.g.,* safe harbor gene sequence). The guide sequence can include 17 nucleic acids that can direct the DNA nuclease (*e.g.,* Cas9 nuclease) to the target DNA site (*e.g.,* safe harbor gene sequence). In some instances, the guide sequence contains about 1 to about 10 nucleic acid mismatches in the complementarity region at the 5' end of the targeting region. In other instances, the guide sequence contains no mismatches in the complementarity region at the last about 5 to about 12 nucleic acids at the 3' end of the targeting region.

The protein-binding scaffold sequence ("second nucleotide sequence") of the DNA-targeting RNA can comprise two complementary stretches of nucleotides that hybridize to one another to form a double-stranded RNA duplex (dsRNA duplex). The protein-binding scaffold sequence can be between about 30 nucleic acids to about 200 nucleic acids, *e.g*., about 40 nucleic acids to about 200 nucleic acids, about 50 nucleic acids to about 200 nucleic acids, about 60 nucleic acids to about 200 nucleic acids, about 70 nucleic acids to about 200 nucleic acids, about 80 nucleic acids to about 200 nucleic acids, about 90 nucleic acids to about 200 nucleic acids, about 100 nucleic acids to about 200 nucleic acids, about 110 nucleic acids to about 200 nucleic acids, about 120 nucleic acids to about 200 nucleic acids, about 130 nucleic acids to about 200 nucleic acids, about 140 nucleic acids to about 200 nucleic acids, about 150 nucleic acids to about 200 nucleic acids, about 160 nucleic acids to about 200 nucleic acids, about 170 nucleic acids to about 200 nucleic acids, about 180 nucleic acids to about 200 nucleic acids, or about 190 nucleic acids to about 200 nucleic acids. In certain aspects, the protein-binding sequence can be between about 30 nucleic acids to about 190 nucleic acids, *e.g*., about 30 nucleic acids to about 180 nucleic acids, about 30 nucleic acids to about 170 nucleic acids, about 30 nucleic acids to about 160 nucleic acids, about 30 nucleic acids to about 150 nucleic acids, about 30 nucleic acids to about 140 nucleic acids, about 30 nucleic acids to about 130 nucleic acids, about 30 nucleic acids to about 120 nucleic acids, about 30 nucleic acids to about 110 nucleic acids, about 30 nucleic acids to about 100 nucleic acids, about 30 nucleic acids to about 90 nucleic acids, about 30 nucleic acids to about 80 nucleic acids, about 30 nucleic acids to about 70 nucleic acids, about 30 nucleic acids to about 60 nucleic acids, about 30 nucleic acids to about 50 nucleic acids, or about 30 nucleic acids to about 40 nucleic acids.

In some aspects of the disclosure, the DNA-targeting RNA (*e.g.,* sgRNA) is a truncated form thereof comprising a guide sequence having a shorter region of complementarity to a target DNA sequence (*e.g*., less than 20 nucleotides in length). In certain instances, the truncated DNA-targeting RNA (*e.g.,* sgRNA) provides improved DNA nuclease (*e.g.,* Cas9 nuclease) specificity by reducing off-target effects. For example, a truncated sgRNA can comprise a guide sequence having 17, 18, or 19 complementary nucleotides to a target DNA sequence (*e.g.,* 17-18, 17-19, or 18-19 complementary nucleotides). *See, e.g.,* Fu et al., Nat. Biotechnol., 32(3): 279-284 (2014).

The DNA-targeting RNA can be selected using any of the web-based software described above. As a non-limiting example, considerations for selecting a DNA-targeting RNA can include the PAM sequence for the Cas9 nuclease to be used, and strategies for minimizing off-target modifications. Tools, such as the CRISPR Design Tool, can provide sequences for preparing the DNA-targeting RNA, for assessing target modification efficiency, and/or assessing cleavage at off-target sites.

The DNA-targeting RNA can be produced by any method known to one of ordinary skill in the art. In some aspects of the disclosure, a nucleotide sequence encoding the DNA-targeting RNA is cloned into an expression cassette or an expression vector. In certain aspects of the disclosure, the nucleotide sequence is produced by PCR and contained in an expression cassette. For instance, the nucleotide sequence encoding the DNA-targeting RNA can be PCR amplified and appended to a promoter sequence, *e.g.,* a U6 RNA polymerase III promoter sequence. In other aspects of the disclosure, the nucleotide sequence encoding the DNA-targeting RNA is cloned into an expression vector that contains a promoter, *e.g.,* a U6 RNA polymerase III promoter, and a transcriptional control element, enhancer, U6 termination sequence, one or more nuclear localization signals, *etc.* In some aspects of the disclosure, the expression vector is multicistronic or bicistronic and can also include a nucleotide sequence encoding a fluorescent protein, an epitope tag and/or an antibiotic resistance marker. In certain instances of the bicistronic expression vector, the first nucleotide sequence encoding, for example, a fluorescent protein, is linked to a second nucleotide sequence encoding, for example, an antibiotic resistance marker using the sequence encoding a self-cleaving peptide, such as a viral 2A peptide. Viral 2A peptides including foot-and-mouth disease virus 2A (F2A); equine rhinitis A virus 2A (E2A); porcine teschovirus-1 2A (P2A) and *Thoseaasigna* virus 2A (T2A) have high cleavage efficiency such that two proteins can be expressed simultaneously yet separately from the same RNA transcript.

Suitable expression vectors for expressing the DNA-targeting RNA are commercially available from Addgene, Sigma-Aldrich, and Life Technologies. The expression vector can be pLQ1651 (Addgene Catalog No. 51024) which includes the fluorescent protein mCherry. Non-limiting examples of other expression vectors include pX330, pSpCas9, pSpCas9n, pSpCas9-2A-Puro, pSpCas9-2A-GFP, pSpCas9n-2A-Puro, the GeneArt^{®} CRISPR Nuclease OFP vector, the GeneArt^{®} CRISPR Nuclease OFP vector, and the like.

In certain aspects of the disclosure, the DNA-targeting RNA (*e.g.,* sgRNA) is chemically synthesised. DNA-targeting RNAs can be synthesised using 2'-O-thionocarbamate-protected nucleoside phosphoramidites. Methods are described in, *e.g.,* Dellinger et al., J. American Chemical Society 133, 11540-11556 (2011); Threlfall et al., Organic & Biomolecular Chemistry 10, 746-754 (2012); and Dellinger et al., J. American Chemical Society 125, 940-950 (2003).

In particular aspects of the disclosure, the DNA-targeting RNA (*e.g.,* sgRNA) is chemically modified. As a non-limiting example, the DNA-targeting RNA is a modified sgRNA comprising a first nucleotide sequence complementary to a portion of a safe harbor gene sequence (*e.g.,* a guide sequence or crRNA) and a second nucleotide sequence that interacts with a Cas polypeptide (*e.g.,* a scaffold sequence or tracrRNA).

Without being bound by any particular theory, sgRNAs containing one or more chemical modifications can increase the activity, stability, and specificity and/or decrease the toxicity of the modified sgRNA compared to a corresponding unmodified sgRNA when used for CRISPR-based genome editing, *e.g*., homologous recombination. Non-limiting advantages of modified sgRNAs include greater ease of delivery into target cells, increased stability, increased duration of activity, and reduced toxicity. The modified sgRNAs described herein as part of a CRISPR/Cas9 system provide higher frequencies of on-target genome editing (*e.g.,* homologous recombination), improved activity, and/or specificity compared to their unmodified sequence equivalents.

One or more nucleotides of the guide sequence and/or one or more nucleotides of the scaffold sequence can be a modified nucleotide. For instance, a guide sequence that is about 20 nucleotides in length may have 1 or more, *e.g.,* 1, 2, 3, 4, 5, 6, 7, 8, 9, 10, 11, 12, 13, 14, 15, 16, 17, 18, 19, or 20 modified nucleotides. In some cases, the guide sequence includes at least 2, 3, 4, 5, 6, 7, 8, 9, 10, or more modified nucleotides. In other cases, the guide sequence includes at least 2, 3, 4, 5, 6, 7, 8, 9, 10, 11, 12, 13, 14, 15, 16, 17, 19, 20, or more modified nucleotides. The modified nucleotide can be located at any nucleic acid position of the guide sequence. In other words, the modified nucleotides can be at or near the first and/or last nucleotide of the guide sequence, and/or at any position in between. For example, for a guide sequence that is 20 nucleotides in length, the one or more modified nucleotides can be located at nucleic acid position 1, position 2, position 3, position 4, position 5, position 6, position 7, position 8, position 9, position 10, position 11, position 12, position 13, position 14, position 15, position 16, position 17, position 18, position 19, and/or position 20 of the guide sequence. In certain instances, from about 10% to about 30%, *e.g.,* about 10% to about 25%, about 10% to about 20%, about 10% to about 15%, about 15% to about 30%, about 20% to about 30%, or about 25% to about 30% of the guide sequence can comprise modified nucleotides. In other instances, from about 10% to about 30%, *e.g.,* about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, or about 30% of the guide sequence can comprise modified nucleotides.

In certain aspects of the disclosure, the modified nucleotides are located at the 5'-end (*e.g.,* the terminal nucleotide at the 5'-end) or near the 5'-end (*e.g.,* within 1, 2, 3, 4, or 5 nucleotides of the terminal nucleotide at the 5'-end) of the guide sequence and/or at internal positions within the guide sequence.

In some aspects of the disclosure, the scaffold sequence of the modified sgRNA contains one or more modified nucleotides. For example, a scaffold sequence that is about 80 nucleotides in length may have 1 or more, *e.g.,* 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 76, 77, 78, 79, or 80 modified nucleotides. In some instances, the scaffold sequence includes at least 2, 3, 4, 5, 6, 7, 8, 9, 10, or more modified nucleotides. In other instances, the scaffold sequence includes at least 2, 3, 4, 5, 6, 7, 8, 9, 10, 11, 12, 13, 14, 15, 16, 17, 19, 20, or more modified nucleotides. The modified nucleotides can be located at any nucleic acid position of the scaffold sequence. For example, the modified nucleotides can be at or near the first and/or last nucleotide of the scaffold sequence, and/or at any position in between. For example, for a scaffold sequence that is about 80 nucleotides in length, the one or more modified nucleotides can be located at nucleic acid position 1, position 2, position 3, position 4, position 5, position 6, position 7, position 8, position 9, position 10, position 11, position 12, position 13, position 14, position 15, position 16, position 17, position 18, position 19, position 20, position 21, position 22, position 23, position 24, position 25, position 26, position 27, position 28, position 29, position 30, position 31, position 32, position 33, position 34, position 35, position 36, position 37, position 38, position 39, position 40, position 41, position 42, position 43, position 44, position 45, position 46, position 47, position 48, position 49, position 50, position 51, position 52, position 53, position 54, position 55, position 56, position 57, position 58, position 59, position 60, position 61, position 62, position 63, position 64, position 65, position 66, position 67, position 68, position 69, position 70, position 71, position 72, position 73, position 74, position 75, position 76, position 77, position 78, position 79, and/or position 80 of the sequence. In some instances, from about 1% to about 10%, *e.g.,* about 1% to about 8%, about 1% to about 5%, about 5% to about 10%, or about 3% to about 7% of the scaffold sequence can comprise modified nucleotides. In other instances, from about 1% to about 10%, *e.g.,* about 1%, about 2%, about 3%, about 4%, about 5%, about 6%, about 7%, about 8%, about 9%, or about 10% of the scaffold sequence can comprise modified nucleotides.

In certain aspects of the disclosure, the modified nucleotides are located at the 3'-end (*e.g.,* the terminal nucleotide at the 3'-end) or near the 3'-end (*e.g.,* within 1, 2, 3, 4, or 5 nucleotides of the 3'-end) of the scaffold sequence and/or at internal positions within the scaffold sequence.

In some aspects of the disclosure, the modified sgRNA comprises one, two, or three consecutive or non-consecutive modified nucleotides starting at the 5'-end (*e.g.,* the terminal nucleotide at the 5'-end) or near the 5'-end (*e.g.,* within 1, 2, 3, 4, or 5 nucleotides of the terminal nucleotide at the 5'-end) of the guide sequence and one, two, or three consecutive or non-consecutive modified nucleotides starting at the 3'-end (*e.g.,* the terminal nucleotide at the 3'-end) or near the 3'-end (*e.g*., within 1, 2, 3, 4, or 5 nucleotides of the 3'-end) of the scaffold sequence.

In some instances, the modified sgRNA comprises one modified nucleotide at the 5'-end (*e.g.,* the terminal nucleotide at the 5'-end) or near the 5'-end (*e.g.,* within 1, 2, 3, 4, or 5 nucleotides of the terminal nucleotide at the 5'-end) of the guide sequence and one modified nucleotide at the 3'-end *(e.g.,* the terminal nucleotide at the 3'-end) or near the 3'- end (*e.g*., within 1, 2, 3, 4, or 5 nucleotides of the 3'-end) of the scaffold sequence.

In other instances, the modified sgRNA comprises two consecutive or non-consecutive modified nucleotides starting at the 5'-end (*e.g.,* the terminal nucleotide at the 5'-end) or near the 5'-end (*e.g*., within 1, 2, 3, 4, or 5 nucleotides of the terminal nucleotide at the 5'-end) of the guide sequence and two consecutive or non-consecutive modified nucleotides starting at the 3'-end (*e.g.,* the terminal nucleotide at the 3'-end) or near the 3'-end (*e.g.,* within 1, 2, 3, 4, or 5 nucleotides of the 3'-end) of the scaffold sequence.

In yet other instances, the modified sgRNA comprises three consecutive or non-consecutive modified nucleotides starting at the 5'-end (*e.g.,* the terminal nucleotide at the 5'-end) or near the 5'-end (*e.g*., within 1, 2, 3, 4, or 5 nucleotides of the terminal nucleotide at the 5'-end) of the guide sequence and three consecutive or non-consecutive modified nucleotides starting at the 3'-end (*e.g.,* the terminal nucleotide at the 3'-end) or near the 3'-end (*e.g.,* within 1, 2, 3, 4, or 5 nucleotides of the 3'-end) of the scaffold sequence.

In particular aspects of the disclosure, the modified sgRNA comprises three consecutive modified nucleotides at the 5'-end of the guide sequence and three consecutive modified nucleotides at the 3'-end of the scaffold sequence.

The modified nucleotides of the sgRNA can include a modification in the ribose (*e.g.,* sugar) group, phosphate group, nucleobase, or any combination thereof. In some aspects of the disclosure, the modification in the ribose group comprises a modification at the 2' position of the ribose.

In some aspects of the disclosure, the modified nucleotide includes a 2'fluoro-arabino nucleic acid, tricycle-DNA (tc-DNA), peptide nucleic acid, cyclohexene nucleic acid (CeNA), locked nucleic acid (LNA), ethylene-bridged nucleic acid (ENA), a phosphodiamidate morpholino, or a combination thereof.

Modified nucleotides or nucleotide analogues can include sugar- and/or backbone-modified ribonucleotides (*i.e.,* include modifications to the phosphate-sugar backbone). For example, the phosphodiester linkages of a native or natural RNA may be modified to include at least one of a nitrogen or sulfur heteroatom. In some backbone-modified ribonucleotides, the phosphoester group connecting to adjacent ribonucleotides may be replaced by a modified group, *e.g.,* a phosphothioate group. In preferred sugar-modified ribonucleotides, the 2' moiety is a group selected from H, OR, R, halo, SH, SR, NH₂, NHR, NR₂ or ON, wherein R is C₁-C₆ alkyl, alkenyl or alkynyl and halo is F, Cl, Br or I.

In some aspects of the disclosure, the modified nucleotide contains a sugar modification. Non-limiting examples of sugar modifications include 2'-deoxy-2'-fluoro-oligoribonucleotide (2'-fluoro-2'-deoxycytidine-5'-triphosphate, 2'-fluoro-2'-deoxyuridine-5'-triphosphate), 2'-deoxy-2'-deamine oligoribonucleotide (2'-amino-2'-deoxycytidine-5'-triphosphate, 2'-amino-2'-deoxyuridine-5-triphosphate), 2'-O-alkyl oligoribonucleotide, 2'-deoxy-2'-C-alkyl oligoribonucleotide (2'-O-methylcytidine-5'-triphosphate, 2'-methyluridine-5'-triphosphate), 2'-C-alkyl oligoribonucleotide, and isomers thereof (2'-aracytidine-5'-triphosphate, 2'-arauridine-5'-triphosphate), azidotriphosphate (2'-azido-2'-deoxycytidine-5'-triphosphate, 2'-azido-2'-deoxyuridine-5'-triphosphate), and combinations thereof.

In some aspects of the disclosure, the modified sgRNA contains one or more 2'-fluoro, 2'-amino and/or 2'-thio modifications. In some instances, the modification is a 2'-fluoro-cytidine, 2'-fluoro-uridine, 2'-fluoro-adenosine, 2'-fluoro-guanosine, 2'-amino-cytidine, 2'-amino-uridine, 2'-amino-adenosine, 2'-amino-guanosine, 2,6-diaminopurine, 4-thio-uridine, 5-amino-allyl-uridine, 5-bromo-uridine, 5-iodo-uridine, 5-methyl-cytidine, ribo-thymidine, 2-aminopurine, 2'-amino-butyryl-pyrene-uridine, 5-fluoro-cytidine, and/or 5-fluoro-uridine.

There are more than 96 naturally occurring nucleoside modifications found on mammalian RNA. *See, e.g.,* Limbach et al., Nucleic Acids Research, 22(12):2183-2196 (1994). The preparation of nucleotides and modified nucleotides and nucleosides are well-known in the art, *e.g.,* from U.S. Pat. Nos. 4,373,071, 4,458,066, 4,500,707, 4,668,777, 4,973,679, 5,047,524, 5,132,418, 5,153,319, 5,262,530, and 5,700,642. Numerous modified nucleosides and modified nucleotides that are suitable for use as described herein are commercially available. The nucleoside can be an analogue of a naturally occurring nucleoside. In some cases, the analogue is dihydrouridine, methyladenosine, methylcytidine, methyluridine, methylpseudouridine, thiouridine, deoxycytodine, and deoxyuridine.

In some cases, the modified sgRNA described herein includes a nucleobase-modified ribonucleotide, *i.e.,* a ribonucleotide containing at least one non-naturally occurring nucleobase instead of a naturally occurring nucleobase. Non-limiting examples of modified nucleobases which can be incorporated into modified nucleosides and modified nucleotides include m5C (5-methylcytidine), m5U (5-methyluridine), m6A (N6-methyladenosine), s2U (2-thiouridine), Um (2'-0-methyluridine), m1A (1-methyl adenosine), m2A (2-methyladenosine), Am (2-1-O-methyladenosine), ms2m6A (2-methylthio-N6-methyladenosine), i6A (N6-isopentenyl adenosine), ms2i6A (2-methylthio-N6isopentenyladenosine), io6A (N6-(cis-hydroxyisopentenyl) adenosine), ms2io6A (2-methylthio-N6-(cis-hydroxyisopentenyl)adenosine), g6A (N6-glycinylcarbamoyladenosine), t6A (N6-threonyl carbamoyladenosine), ms2t6A (2-methylthio-N6-threonyl carbamoyladenosine), m6t6A (N6-methyl-N6-threonylcarbamoyladenosine), hn6A(N6.-hydroxynorvalylcarbamoyl adenosine), ms2hn6A (2-methylthio-N6-hydroxynorvalyl carbamoyladenosine), Ar(p) (2'-O-ribosyladenosine(phosphate)), I (inosine), m11 (1-methylinosine), m'Im (1,2'-O-dimethylinosine), m3C (3-methylcytidine), Cm (2T-O-methylcytidine), s2C (2-thiocytidine), ac4C (N4-acetylcytidine), f5C (5-fonnylcytidine), m5Cm (5,2-O-dimethylcytidine), ac4Cm (N4acetyl2TOmethylcytidine), k2C (lysidine), m1G (1-methylguanosine), m2G (N2-methylguanosine), m7G (7-methylguanosine), Gm (2'-O-methylguanosine), m22G (N2,N2-dimethylguanosine), m2Gm (N2,2'-O-dimethylguanosine), m22Gm (N2,N2,2'-O-trimethylguanosine), Gr(p) (2'-O-ribosylguanosine(phosphate)), yW (wybutosine), o2yW (peroxywybutosine), OHyW (hydroxywybutosine), OHyW^{∗} (undermodified hydroxywybutosine), imG (wyosine), mimG (methylguanosine), Q (queuosine), oQ (epoxyqueuosine), galQ (galtactosyl-queuosine), manQ (mannosyl-queuosine), preQo (7-cyano-7-deazaguanosine), preQi (7-aminomethyl-7-deazaguanosine), G (archaeosine), D (dihydrouridine), m5Um (5,2'-O-dimethyluridine), s4U (4-thiouridine), m5s2U (5-methyl-2-thiouridine), s2Um (2-thio-2'-O-methyluridine), acp3U (3-(3-amino-3-carboxypropyl)uridine), ho5U (5-hydroxyuridine), mo5U (5-methoxyuridine), cmo5U (uridine 5-oxyacetic acid), mcmo5U (uridine 5-oxyacetic acid methyl ester), chm5U (5-(carboxyhydroxymethyl)uridine)), mchm5U (5-(carboxyhydroxymethyl)uridine methyl ester), mcm5U (5-methoxycarbonyl methyluridine), mcm5Um (S-methoxycarbonylmethyl-2-O-methyluridine), mcm5s2U (5-methoxycarbonylmethyl-2-thiouridine), nm5s2U (5-aminomethyl-2-thiouridine), mnm5U (5-methylaminomethyluridine), mnm5s2U (5-methylaminomethyl-2-thiouridine), mnm5se2U (5-methylaminomethyl-2-selenouridine), ncm5U (5-carbamoylmethyl uridine), ncm5Um (5-carbamoylmethyl-2'-O-methyluridine), cmnm5U (5-carboxymethylaminomethyluridine), cnmm5Um (5-carboxymethylaminomethyl-2-L-Omethyluridine), cmnm5s2U (5-carboxymethylaminomethyl-2-thiouridine), m62A (N6,N6-dimethyladenosine), Tm (2'-O-methylinosine), m4C (N4-methylcytidine), m4Cm (N4,2-O-dimethylcytidine), hm5C (5-hydroxymethylcytidine), m3U (3-methyluridine), cm5U (5-carboxymethyluridine), m6Am (N6,T-O-dimethyladenosine), rn62Am (N6,N6,O-2-trimethyladenosine), m2'7G (N2,7-dimethylguanosine), m2'2'7G (N2,N2,7-trimethylguanosine), m3Um (3,2T-O-dimethyluridine), m5D (5-methyldihydrouridine), f5Cm (5-formyl-2'-O-methylcytidine), m1Gm (1,2'-O-dimethylguanosine), m'Am (1,2-0-dimethyl adenosine)irinomethyluridine), tm5s2U (S-taurinomethyl-2-thiouridine)), imG-14 (4-demethyl guanosine), imG2 (isoguanosine), or ac6A (N6-acetyladenosine), hypoxanthine, inosine, 8-oxo-adenine, 7-substituted derivatives thereof, dihydrouracil, pseudouracil, 2-thiouracil, 4-thiouracil, 5-aminouracil, 5-(C₁-C₆)-alkyluracil, 5-methyluracil, 5-(C₂-C₆)-alkenyluracil, 5-(C₂-C₆)-alkynyluracil, 5-(hydroxymethyl)uracil, 5-chlorouracil, 5-fluorouracil, 5-bromouracil, 5-hydroxycytosine, 5-(C₁-C₆)-alkylcytosine, 5-methylcytosine, 5-(C₂-C₆)-alkenylcytosine, 5-(C₂-C₆)-alkynylcytosine, 5-chlorocytosine, 5-fluorocytosine, 5-bromocytosine, N²-dimethylguanine, 7-deazaguanine, 8-azaguanine, 7-deaza-7-substituted guanine, 7-deaza-7-(C2-C6)alkynylguanine, 7-deaza-8-substituted guanine, 8-hydroxyguanine, 6-thioguanine, 8-oxoguanine, 2-aminopurine, 2-amino-6-chloropurine, 2,4-diaminopurine, 2,6-diaminopurine, 8-azapurine, substituted 7-deazapurine, 7-deaza-7-substituted purine, 7-deaza-8-substituted purine, and combinations thereof.

In some aspects of the disclosure, the phosphate backbone of the modified sgRNA is altered. The modified sgRNA can include one or more phosphorothioate, phosphoramidate (*e.g*., N3'-P5'-phosphoramidate (NP)), 2'-O-methoxy-ethyl (2'MOE), 2'-O-methyl-ethyl (2'ME), and/or methylphosphonate linkages. In certain instances, the phosphate group is changed to a phosphothioate, 2'-O-methoxy-ethyl (2'MOE), 2'-O-methyl-ethyl (2'ME), N3'-P5'-phosphoramidate (NP), and the like.

In particular aspects of the disclosure, the modified nucleotide comprises a 2'-O-methyl nucleotide (M), a 2'-O-methyl, 3'-phosphorothioate nucleotide (MS), a 2'-O-methyl, 3'thioPACE nucleotide (MSP), or a combination thereof.

In some instances, the modified sgRNA includes one or more MS nucleotides. In other instances, the modified sgRNA includes one or more MSP nucleotides. In yet other instances, the modified sgRNA includes one or more MS nucleotides and one or more MSP nucleotides. In further instances, the modified sgRNA does not include M nucleotides. In certain instances, the modified sgRNA includes one or more MS nucleotides and/or one or more MSP nucleotides, and further includes one or more M nucleotides. In certain other instances, MS nucleotides and/or MSP nucleotides are the only modified nucleotides present in the modified sgRNA.

It should be noted that any of the modifications described herein may be combined and incorporated in the guide sequence and/or the scaffold sequence of the modified sgRNA.

In some cases, the modified sgRNAs also include a structural modification such as a stem loop, *e.g.,* M2 stem loop or tetraloop.

The chemically modified sgRNAs can be used with any CRISPR-associated or RNA-guided technology. As described herein, the modified sgRNAs can serve as a guide for any Cas9 polypeptide or variant thereof, including any engineered or man-made Cas9 polypeptide. The modified sgRNAs can target DNA and/or RNA molecules in isolated cells or *in vivo* (*e.g.,* in an animal).

### D. Transgene Cassettes for Producing Gene Modified Neural Stem Cells

Transgene cassettes for producing gene modified neural stem cells can be as described herein in respect of any aspect of the disclosure. The cassette may contain one or more of a promoter, *e.g.,* a U6 RNA polymerase III promoter, a transcriptional control element, enhancer, U6 termination sequence, one or more nuclear localization signals, *etc.* In some aspects of the disclosure, the cassette is multicistronic or bicistronic and can also include a nucleotide sequence encoding a fluorescent protein, an epitope tag, and/or an antibiotic resistance marker. In certain instances of the bicistronic cassette, the first nucleotide sequence encoding, for example, a fluorescent protein, is linked to a second nucleotide sequence encoding, for example, an antibiotic resistance marker using a sequence encoding a self-cleaving peptide, such as a viral 2A peptide. Viral 2A peptides including foot-and-mouth disease virus 2A (F2A); equine rhinitis A virus 2A (E2A); porcine teschovirus-1 2A (P2A) and *Thoseaasigna* virus 2A (T2A) have high cleavage efficiency such that two proteins can be expressed simultaneously yet separately from the same RNA transcript. Exemplary cassettes include a reporter cassette containing the ubiquitin C promoter driving GFP expression. Alternatively, bicistronic cassette constructs separated by a self-cleaving 2A peptide have been generated, thereby providing high cleavage efficiency between the two transgenes located upstream and downstream of the 2A peptide. These constructs include: (1) UbC-GFP-2A-CD8 (CD8 alpha cell surface marker for purification of genetically modified human neural stem cells); (2) UbC-GFP-2A-tCD19 (truncated cell surface marker for purification of genetically modified human neural stem cells); (3) UbC-GalC-2A-tCD19 (therapeutic enzyme construct for Krabbe disease with truncated CD19) or UbC-GalC-2A-eGFP; and (4) UbC-TPP1-2A-CD8 (therapeutic enzyme construct for LINCL with CD8 alpha).

### E. Methods for Isolating and Purifying Gene Modified Neural Stem Cells

Selectable markers, detectable markers, cell surface markers, and purification markers, alone or in combination, can be used to isolate and/or purify gene modified human neural stem cells of the disclosure. For example, expression of a selectable marker gene encoding an antibiotic resistance factor provides for preferential survival of gene modified cells in the presence of the corresponding antibiotic, whereas other cells present in the culture will be selectively killed. Alternatively, expression of a fluorescent protein such as GFP or expression of a cell surface marker not normally expressed on neural stem cells may permit gene modified neural stem cells to be identified, purified or isolated by fluorescence-activated cell sorting (FACS), magnetic-activated cell sorting (MACS), or analogous methods. Suitable cell surface markers used in the following example are CD8, truncated CD8, CD19, and truncated CD19, although other cell surface markers can also fulfill the same function.

Methods for isolating or purifying the genetically modified cells are described herein and are known in the art. In specific examples, FACS or MACS methods were used to human neural stem cells expressing the cell surface markers CD8 or CD19.

Methods for culturing or expanding the genetically modified cells are known in the art. Methods for culturing neural stem cells and their progeny are known, and suitable culture media, supplements, growth factors, and the like are both known and commercially available. Typically human neural stem cells are maintained and expanded in serum-free conditions. One suitable medium is Ex Vivo 15 (BioWhittaker) with N2 supplement (GIBCO), fibroblast growth factor (20 ng/ml), epidermal growth factor (20 ng/ml), lymphocyte inhibitory factor (10 ng/ml), neural survival factor-1 (Clonetics, San Diego) and N-acetylcysteine (60 µg/ml; Sigma). Alternative media, supplements and growth factors and/or alternative concentrations can readily be determined by the skilled person and are extensively described in the literature.

In some aspects of the disclosure, the isolated or purified genetically modified cells can be expanded *in vitro* according to standard methods known to those of ordinary skill in the art.

### F. Introducing Components of Nuclease-Mediated Genome Editing into Cells

Methods for introducing polypeptides and nucleic acids into a target cell (host cell) are known in the art, and any known method can be used to introduce a nuclease or a nucleic acid (*e.g.,* a nucleotide sequence encoding the nuclease, a DNA-targeting RNA (*e.g.,* single guide RNA), a donor template for homology-directed repair (HDR), *etc.*) into a human neural stem cell. Non-limiting examples of suitable methods include electroporation (*e.g*., nucleofection), viral or bacteriophage infection, transfection, conjugation, protoplast fusion, lipofection, calcium phosphate precipitation, polyethyleneimine (PEI)-mediated transfection, DEAE-dextran mediated transfection, liposome-mediated transfection, particle gun technology, calcium phosphate precipitation, direct microinjection, nanoparticle-mediated nucleic acid delivery, and the like.

In some aspects of the disclosure, a DNA nuclease system (such as CRISPR or TALEN) is introduced into neural stem cells. In some aspects of the disclosure, the CRISPR/Cas9 system comprises a plasmid-based Cas9. In some aspects of the disclosure, the CRISPR/Cas9 system comprises a Cas9 mRNA and sgRNA. In some aspects of the disclosure, the Cas9 mRNA, or sgRNA, or both Cas9 mRNA and sgRNA is modified before introduced into the neural stem cells. In some aspects of the disclosure, the nuclease system is TALEN system. In some aspects of the disclosure, the TALEN system comprises a plasmid-based TALEN. In some aspects of the disclosure, the TALEN system comprises a RNA-based TALEN.

In some aspects of the disclosure, the components of nuclease-mediated genome editing can be introduced into a target cell using a delivery system. In certain instances, the delivery system comprises a nanoparticle, a microparticle (*e.g*., a polymer micropolymer), a liposome, a micelle, a virosome, a viral particle, a nucleic acid complex, a transfection agent, an electroporation agent (*e.g*., using a NEON transfection system), a nucleofection agent, a lipofection agent, and/or a buffer system that includes a nuclease component (as a polypeptide or encoded by an expression construct) and one or more nucleic acid components such as a DNA-targeting RNA and/or a donor template. For instance, the components can be mixed with a lipofection agent such that they are encapsulated or packaged into cationic submicron oil-in-water emulsions. Alternatively, the components can be delivered without a delivery system, *e.g*., as an aqueous solution.

Methods of preparing liposomes and encapsulating polypeptides and nucleic acids in liposomes are described in, *e.g.,* Methods and Protocols, Volume 1: Pharmaceutical Nanocarriers: Methods and Protocols. (ed. Weissig). Humana Press, 2009 and Heyes et al. (2005) J Controlled Release 107:276-87. Methods of preparing microparticles and encapsulating polypeptides and nucleic acids are described in, *e.g*., Functional Polymer Colloids and Microparticles volume 4 (Microspheres, microcapsules & liposomes). (eds. Arshady & Guyot). Citus Books, 2002 and Microparticulate Systems for the Delivery of Proteins and Vaccines. (eds. Cohen & Bernstein). CRC Press, 1996.

### G. Human Neural Stem Cells

Human neural stem cells may be neural stem cells directly isolated from human brain tissue using a method described in Uchida et al., Proc Natl Acad Sci USA, 2000, 97(26):14720-14725 in which cell surface markers were used to isolate CD133+ CD24-/lo NSCs. Other markers can also be used including CD49f, CD29 and CD15. Human neural stem cells may be derived from second trimester human fetal brain. In principle any marker specifically expressed on human neural stem cells, or any combination of markers characteristic of human neural stem cells, may be used in the isolation of human neural stem cells.

Human neural stem cells may be derived from human embryonic (not part of the invention), fetal or adult sources, or may be derived using known techniques from other cell types, including embryonic stem (ES) cells and induced pluripotent stem (iPS) cells. Human neural stem cells may also be derived by direct reprogramming from a somatic cell population, for example fibroblasts. In some aspects of the disclosure, human neural stem cells are derived from a somatic stem cell or a pluripotent stem cell. Human neural stem cells include cells derived from the human central nervous system, including fetal spinal cord and fetal brain tissues. The use of primary neural stem cells obtained from a donor may be preferred in order to provide cells immediately suited for the treatment of CNS disorders and directly transplantable to the CNS of a patient.

Human neural stem cells of the disclosure may be autologous or allogeneic human neural stem cells. Autologous stem cells are derived from the patient to be treated and thus would not be expected to provoke an immune response from the patient. However, autologous stem cells might need to be genetically modified or corrected to eliminate an inherent disease or disorder in addition to any further modification according to the aspects of the disclosure. In contrast, allogeneic, *i.e.,* donor-derived, cells from a healthy subject can be isolated, and purified to be ready for use without modification other than the modification provided by aspects of the disclosure. Such allogeneic human neural stem cells can be genetically modified according to the disclosure and expanded using large-scale culture methods to provide a validated, safe and consistent product that can be used off-the-shelf without costly de novo derivation, modification and expansion of cells on an individual basis. Such allogeneic cells can be prepared and banked, *e.g*., using standard cryopreservation methods, to provide a resource ready for use upon identification of a suitable patient.

Neural stem cells (NSCs) can be derived directly from embryos (not part of the invention), from adult tissue, from fetal tissue, from multipotent stem cells, or from pluripotent stem cells including embryonic stem cells (ESCs) or induced pluripotent stem cells (iPSCs). In some cases, the pluripotent stem cells are either wild-type or genetically modified.

In some aspects of the disclosure, the NSCs are obtained or derived from tissue of the nervous system of a subject, *e.g.,* a human subject and cultured *in vitro.* The nervous tissue can be normal or diseased. In some aspects of the disclosure, the nervous tissue is obtained from an individual with a genetic disorder of the central nervous system. In other aspects of the disclosure, the nervous tissue is from an individual with or suspected of having a neurodegenerative disease. In yet other aspects of the disclosure, the nervous tissue is from an individual with or suspected of having a neurological injury.

In other cases, the NSCs of the disclosure are from a population of symmetrically dividing neural stem cells that has previously been maintained in culture.

### H. Transgenes for Treating a Genetic Disorder of the CNS,

### Neurodegenerative Disease or Neural Injury

In some aspects of the disclosure, the transgene of the donor template encodes a protein associated with a genetic disorder of the central nervous system. The genetic disorder can be an autosomal recessive genetic disorder than may be treated by expressing a wild-type (normal) allele of the corresponding mutant gene as a transgene. The genetic disorder is caused by a mutant gene and the transgene described herein can be used to treat the disorder. In some aspects of the disclosure, the NSCs carrying the mutant gene can be genetically modified to express a wild-type allele of the mutant gene via the transgene. The transgene will typically belong to one of three categories: (1) secreted proteins; (2) cell surface enzymes, for example, a metallo-endopeptidase (*e.g*., Neprilysin) to cleave or digest unwanted protein and/or scar tissue; and (3) intracellular proteins such as regulatable BCL2.

Exemplary transgenes for use herein include *GALC* (Krabbe disease), *ABCD1* (adrenoleukodystrophy), *GFAP* (Alexander disease), *CYP27A1* (cerebrotendineous xanthomatosis), ARSA (metachromatic leukodystrophy), *PLP1* (Pelizaeus-Merzbacher disease), ASPA (Canavan disease), *EIF-2B* (leukoencephalopathy with vanishing white matter), *PHYH* (Refsum disease 1), *PEX7* (Refsum disease 2), *PPT1* (infantile neuronal ceroid lipofuscinosis (NCL)), *TPP1* (late infantile NCL), *CLN3* (juvenile NCL), *CLN6* (adult NCL), *CLN5* (Finnish late infantile variant NCL), *CLN6* (late infantile variant NCL), *MSFD8* (ceroid lipofuscinosis, neuronal, 7), *CLN8* (ceroid lipofuscinosis, neuronal, 8), *CTSD* (ceroid lipofuscinosis, neuronal, 10), *UBE3A* (Angelman syndrome), *POLG* (Alpers' Disease), TAZ (Barth Syndrome), *GLA* (Fabry disease), *SLC20A2* (Fahr's syndrome), *PDE* (retinitis pigmentosa), *SMN1* (spinal muscular atrophy), *IKBKAP* (familial dysautonomia), *MeCP2* (Rett syndrome), *CACNA1C* (Timothy syndrome), *ATXN3* (Machado-Joseph disease), and *RPE65* (Leber congenital amaurosis), USH2A (retinitis pigmentosa), RPGR (retinitis pigmentosa), RP2(retinitis pigmentosa), ABCA4 (Stargardt), RS-1 (X-linked retinoschisis). In some aspects of the disclosure, the protein is secreted by the genetically modified human neural stem cell.

### I. Genetic Diseases of the Central Nervous System, Neurodegenerative

### Diseases, Neurological Injury, and retinal degenerative disease.

The diseases, disorders, and conditions described herein include those affecting astrocytes, oligodendrocytes, glial cells, neurons, motor neurons, interneurons, retinal cells, *etc.* Such neurological diseases, disorders, and conditions that can be treated using the compositions, kits, and methods described herein include Parkinson's disease, Huntington's disease, Alzheimer's disease, memory disorders, epilepsies, macular degeneration (e.g., age-related macular degeneration), retinitis pigmentosa, Leber's congenital amaurosis, retinopathies, optic neuropathies, amyotrophic lateral sclerosis, spinal muscular atrophy, myelin disease, multiple sclerosis, stroke, cerebral palsies, hereditary pediatric leukodystrophies including Pelizaeus-Merbacher disease, neuronal ceroid lipofuscinosis and Krabbe disease, metachromatic leukodystrophy, Tay-Sachs disease, spinal cord injury or trauma. The diseases, disorders, and conditions described herein also include traumatic brain injury, acute inflammation of the central nervous system (CNS), chronic inflammation of the CNS, ischemia, and stroke. The diseases, disorders, and conditions described herein also include retinal degenerative disease.

### J. Methods for using GM-NSCs

The GM-NSCs described herein are useful for treating diseases. In some aspects of the disclosure, there is provided a method of treating neurodegenerative diseases comprising administering to a subject a plurality of genetically modified neural stem cells comprising a transgene, wherein the transgene is inserted into a safe harbor locus (such as any of the GM-NSC cells described herein).

The GM-NSCs described herein are useful for preventing diseases. In some aspects of the disclosure, there is provided a method of preventing neurodegenerative diseases comprising administering to a subject a plurality of genetically modified neural stem cells comprising a transgene, wherein the transgene is inserted into a safe harbor locus (such as any of the GM-NSC cells described herein).

The genetically modified human neural stem cells can be administered into a human subject to prevent or alleviate one or more symptoms of the neurodegenerative disease or the neurological injury. In some aspects of the disclosure, the generated genetically modified neuronal stem cells can be administered to the human subject to prevent or alleviate one or more symptoms of the neurodegenerative disease, wherein the neurodegenerative disease is selected from the group consisting of a leukodystrophy, neuronal ceroid lipofuscinosis, age-related macular degeneration, Alzheimer's disease, Parkinson's disease, Huntington's disease, amyotrophic lateral sclerosis, and retinal degenerative disease.

The neurodegenerative diseases or neurological injury described herein include those affecting astrocytes, oligodendrocytes, glial cells, neurons, motor neurons, interneurons, retinal cells, etc. Such neurodegenerative diseases or neurological injury include Parkinson's disease, Huntington's disease, Alzheimer's disease, memory disorders, epilepsies, macular degeneration (e.g., age-related macular degeneration), retinitis pigmentosa, Leber's congenital amaurosis, retinopathies, optic neuropathies, amyotrophic lateral sclerosis, spinal muscular atrophy, myelin disease, multiple sclerosis, stroke, cerebral palsies, hereditary pediatric leukodystrophies including Pelizaeus-Merbacher disease, neuronal ceroid lipofuscinosis and Krabbe disease, metachromatic leukodystrophy, Tay-Sachs disease, spinal cord injury or trauma. The neurodegenerative diseases or neurological injury described herein also include traumatic brain injury, acute inflammation of the central nervous system (CNS), chronic inflammation of the CNS, ischemia, and stroke. The neurodegenerative diseases or neurological injury described herein also include retinal degenerative disease.

In some aspects of the disclosure, the transgene encodes a neuroprotective or neurodegenerative protein which is selected from the group consisting of brain-derived neurotrophic factor (BDNF), glial-derived neurotrophic factor (GDNF), insulin-like growth factor 1 (IGF1), insulin-like growth factor 2 (IGF2), nerve growth factor (NGF), neurotrophin-2 (NT-2), neurotrophin-3 (NT-3) neurotrophin-4/5 (NT-4/5), neurotrophin-6, conserved dopamine neurotrophic factor (CDNF), ciliary neurotrophic factor (CNTF), epidermal growth factor (EGF), a fibroblast growth factor (FGF), a bone morphogenetic protein (BMP), vascular endothelial growth factor (VEGF), granulocyte colony-stimulating factor (G-CSF), colony-stimulating factor (CSF), interferon-β (IFN-β), tumor necrosis factor-α (TNFa), tissue plasminogen activator (tPA), neurturin, persephin, artemin, neuropeptide Y (NPY), an ephrin, a semaphorin, other neuropoeitic factors, other neurotrophic factors, and a combination thereof. In some aspects of the disclosure, the transgene encodes a neuroprotective or neurodegenerative protein, wherein the neuroprotective or neuroregenerative protein is a secreted protein. In some aspects of the disclosure, the transgene encodes Bcl-2. In some aspects of the disclosure, the transgene encodes a telomerase. In some aspects of the disclosure, the transgene encodes a protein that can improve survival or rejuvenate the neural stem cell to facilitate long-term survival and/or discovery of therapeutic molecules.

In some aspects of the disclosure, the genetically modified neural stem cells are derived from the same subject to be treated or administered. In some aspects of the disclosure, the genetically modified neural stem cells are derived from another subject or subjects. For example, allogeneic, i.e., donor-derived, cells from a healthy subject or subjects can be genetically modified and administered into a patient subject.

In some aspects of the disclosure, there is provided a method of treating neurodegenerative diseases comprising 1) isolating allogeneic human neural stem cells from multiple subjects; 2) genetically modifying isolated human neural stem cells by inserting a transgene into a safe harbor locus; 3) expanding genetically modified isolated human neural stem cells; 4) administering to a subject a plurality of said genetically modified neural stem cells. In some aspects of the disclosure, such allogeneic human neural stem cells can be genetically modified and expanded using large-scale culture methods to provide a validated, safe and consistent product that can be used off-the-shelf without costly de novo derivation, modification and expansion of cells on an individual basis. In some aspects of the disclosure, such allogeneic cells can be prepared and banked, e.g., using standard cryopreservation methods, to provide a resource ready for use upon identification of a suitable patient.

In some aspects of the disclosure, there is provided a method of treating neurodegenerative diseases comprising 1) isolating human neural stem cells from a patient; 2) genetically modifying isolated human neural stem cells by inserting a transgene into a safe harbor locus; 3) administering to said patient a plurality of said genetically modified neural stem cells. In some aspects of the disclosure, the method further comprises expanding genetically modified isolated human neural stem cells before administering into said patient.

The number of the genetically modified neural stem cells administered, and the route of administration varies depending on different disease targets. For example, to treat neuronal ceroid lipofuscinosis (NCL), typically 500-1000 million neural stem cells are administered with direct injection into brain (i.e., subcortical sites or lateral ventricles). *See* Selden et al, J Neurosurg Pediatr. 2013 Jun;11(6):643-52 for additional details. For another example, to treat Pelizaeus-Merzbacher disease (PMD), 300 million neural stem cells are administered with direct injection into the brain. *See* Gupta et al, Sci Transl Med. 2012 Oct 10;4(155): 155ra137 for more details. For another example, to treat NCL orPMD, 20-40 million neural stem cells are directly injected into spinal cord. For another example, to treat age-related macular degeneration, 0.1-2 million neural stem cells are administered via subretinal injection or intravitreal injection.

Methods of administration typically involve transplantation of gene modified human neural stem cells of the disclosure into an immune suppressed subject. Immune suppression to avoid an immune response to transplanted cells, tissues or organs is well-known in the fields of transplant surgery and cell therapy and suitable agents and methods are widely available. Immunosuppressive drugs may include glucocorticoids such as prednisone and prednisolone, cytostatics such as methotrexate, azathioprine and mercaptopurine, antibodies such as anti-IL-2 receptor antibodies basiliximab and daclizumab, drugs acting on immunophilins such as ciclosporin and other drugs including opioids, TNF binding proteins and mycophenolate. Gene modified human neural stem cells are then administered to a site appropriate to the disease or condition to be treated, typically a predetermined region of the brain or the spinal cord.

Pharmaceutical compositions are provided comprising a neural stem cell according to the disclosure and a pharmaceutical carrier.

Pharmaceutically acceptable carriers suitable in the context of the disclosure are known and include substance that aids the administration of an active agent to a cell, an organism, or a subject. "Pharmaceutically acceptable carrier" refers to a carrier or excipient that can be included in the compositions of the disclosure and that causes no significant adverse toxicological effect on the patient. Non-limiting examples of pharmaceutically acceptable carrier include water, NaCl, normal saline solutions, lactated Ringer's, normal sucrose, normal glucose, cell culture media, and the like.

Neural stem cells of the disclosure and pharmaceutical compositions comprising such cells may be administered as a single dose or as multiple doses, for example two doses administered at an interval of about one month, about two months, about three months, about six months or about 12 months. Other suitable dosage schedules can be determined by a medical practitioner.

Also provided herein are methods of using GM-NSC produced by the methods described herein to identify or develop a potential therapeutic molecule. For example, in some aspects of the disclosure, there is provided a method of identifying a therapeutic molecule having an effect on the GM-NSC comprising contacting the GM-NSCs with the therapeutic molecule and determining effects of the therapeutic moelecule on the GM-NSCs. A therapeutic molecule can be, for example, a drug to enhance self-renewal, migration, or myelination. A therapeutic molecule can also be a drug to treat psychiatric or neurodegeneration as well as retinal degeneration. Non-limiting examples of therapeutic molecules include retinoic acid, sodium butyrate, amitriptyline, fluoxetine, sertraline, carbamazepine, valproate, KHS101, oxadiazol compounds, phosphoserine, P7C3, atorvastatin, metformin. The methods may also comprise the screening of a small molecules or biological molecules to identify a potential therapeutic molecule that involve in specific signal pathways. Non-limiting examples of signal pathways include self-renewal, migration, myelination, and differentiation.

### K. Kits

Aspects of the disclosure also provides a kit comprising: (a) a donor template comprising: (i) a transgene cassette comprising a transgene (for example, a transgene operably linked to a promoter, for example, a heterologous promoter); and (ii) two nucleotide sequences comprising two non-overlapping, homologous portions of a safe harbor gene, wherein the nucleotide sequences are located at the 5' and 3' ends of the transgene cassette; (b) a DNA nuclease or a nucleotide sequence encoding the DNA nuclease; and (c) an isolated human neural stem cell. The isolated neural stem cell may be a NSC directly isolated from human brain tissue using a method described in Uchida et al., Proc Natl Acad Sci USA, 2000, 97(26): 14720-14725, in which cell surface markers were used to isolate CD133+ CD24-/lo NSCs.

The DNA nuclease is as defined herein in respect of other aspects of the disclosure, *i.e.,* the methods, cells, and/or pharmaceutical compositions of the disclosure.

Electroporation agents or other agents for introducing components of nuclease-mediated genome editing into cells may also be included in the kits of the disclosure. Suitable agents are known in the art and are widely available commercially. Methods for delivery of the components to cells, *e.g*., electroporation, are described herein and are widely known.

### L. EXEMPLARY EMBODIMENTS

One aspect of the disclosure provides a method for generating a genetically modified human neural stem cell, the method comprising: introducing into an isolated human neural stem cell: (a) a donor template comprising: (i) a transgene cassette comprising a transgene; and (ii) two nucleotide sequences comprising two non-overlapping, homologous portions of a safe harbor locus, wherein the nucleotide sequences are located at the 5' and 3' ends of the transgene cassette; and (b) a DNA nuclease or a nucleotide sequence encoding the DNA nuclease, wherein the DNA nuclease is capable of creating a double-strand break in the safe harbor locus to induce insertion of the transgene into the safe harbor locus, thereby generating a genetically modified human neural stem cell.

In some aspects of the disclosure according to the method described above, the DNA nuclease is selected from the group consisting of a CRISPR-associated protein (Cas) polypeptide, a zinc finger nuclease (ZFN), a transcription activator-like effector nuclease (TALEN), a meganuclease, a variant thereof, a fragment thereof, and a combination thereof.

In some aspects of the disclosure according to any one of the methods described above, the isolated human neural stem cell comprises a primary neural stem cell.

In some aspects of the disclosure according to any one of the methods described above, the isolated human neural stem cell is derived from a somatic stem cell or a pluripotent stem cell, or is derived by direct reprogramming from a somatic cell population.

In some aspects of the disclosure according to any one of the methods described above, the transgene encodes a protein associated with a genetic disorder of the central nervous system. In some aspects of the disclosure, the protein is encoded by a gene selected from the group consisting of GALC (Krabbe disease), ABCD1 (adrenoleukodystrophy), GFAP (Alexander disease), CYP27A1 (cerebrotendineous xanthomatosis), ARSA (metachromatic leukodystrophy), PLP1 (Pelizaeus-Merzbacher disease), ASPA (Canavan disease), EIF-2B (leukoencephalopathy with vanishing white matter), PHYH (Refsum disease 1), PEX7 (Refsum disease 2), PPT1 (infantile neuronal ceroid lipofuscinosis (NCL)), TPP1 (late infantile NCL), CLN3 (juvenile NCL), CLN6 (adult NCL), CLN5 (Finnish late infantile variant NCL), CLN6 (late infantile variant NCL), MSFD8 (ceroid lipofuscinosis, neuronal, 7), CLN8 (ceroid lipofuscinosis, neuronal, 8), CTSD (ceroid lipofuscinosis, neuronal, 10), UBE3A (Angelman syndrome), POLG (Alpers' Disease), TAZ (Barth Syndrome), GLA (Fabry disease), SLC20A2 (Fahr's syndrome), PDE (retinitis pigmentosa), SMN1 (spinal muscular atrophy), IKBKAP (familial dysautonomia), MeCP2 (Rett syndrome), CACNA1C (Timothy syndrome), ATXN3 (Machado-Joseph disease), and RPE65 (Leber congenital amaurosis), USH2A (retinitis pigmentosa), RPGR (retinitis pigmentosa), RP2(retinitis pigmentosa), ABCA4 (Stargardt), RS-1 (X-linked retinoschisis). In some aspects of the disclosure, the protein is secreted by the genetically modified human neural stem cell.

In some aspects of the disclosure according to any one of the methods described above, the transgene encodes a neuroprotective or neuroregenerative protein, a variant thereof, a fragment thereof, or a peptide mimetic thereof. In some aspects of the disclosure, the neuroprotective or neuroregenerative protein is selected from the group consisting of brain-derived neurotrophic factor (BDNF), insulin-like growth factor 1 (IGF-1), insulin-like growth factor 2 (IGF-2), glial-derived neurotrophic factor (GDNF), nerve growth factor (NGF), neurotrophin-2 (NT-2), neurotrophin-3 (NT-3) neurotrophin-4/5 (NT-4/5), neurotrophin-6, conserved dopamine neurotrophic factor (CDNF), ciliary neurotrophic factor (CNTF), epidermal growth factor (EGF), a fibroblast growth factor (FGF), a bone morphogenetic protein (BMP), vascular endothelial growth factor (VEGF), granulocyte colony-stimulating factor (G-CSF), colony-stimulating factor (CSF), interferon-β (IFN-β), tumor necrosis factor-a (TNFa), tissue plasminogen activator (tPA), neurturin, persephin, artemin, neuropeptide Y (NPY), an ephrin, a semaphorin, other neuropoeitic factors, other neurotrophic factors, and a combination thereof. In some aspects of the disclosure, the neuroprotective or neuroregenerative protein is a secreted protein. In some aspects of the disclosure, the transgene encodes Bcl-2. In some aspects of the disclosure, the transgene encodes a telomerase. In some aspects of the disclosure, the transgene encodes a protein that can improve survival or rejuvenate the neural stem cell to facilitate long-term survival and/or discovery of therapeutic molecules.

In some aspects of the disclosure according to any one of the methods described above, the donor template comprises a heterologous promoter. In some aspects of the disclosure, the heterologous promoter of the donor template comprises an inducible promoter or a cell-specific promoter.

In some aspects of the disclosure according to any one of the methods described above, the nucleotide sequence encoding the DNA nuclease comprises RNA.

In some aspects of the disclosure according to any one of the methods described above, the method further comprising introducing into the human neural stem cell a DNA-targeting RNA, a truncated DNA-targeting RNA, or a nucleotide sequence encoding the DNA-targeting RNA or truncated DNA-targeting RNA. In some aspects of the disclosure, the DNA nuclease comprises a Cas polypeptide or a nucleotide sequence encoding the Cas polypeptide, and wherein the DNA-targeting RNA comprises a single guide RNA (sgRNA) or a truncated sgRNA comprising a first nucleotide sequence complementary to a portion of the safe harbor locus and a second nucleotide sequence that interacts with the Cas polypeptide. In some aspects of the disclosure, the Cas polypeptide comprises a Cas9 polypeptide, a variant thereof, or a fragment thereof. In some aspects of the disclosure, the Cas polypeptide variant comprises a high-fidelity or enhanced specificity Cas9 polypeptide variant. In some aspects of the disclosure, the sgRNA or truncated sgRNA comprises one or more modified nucleotides. In some aspects of the disclosure, the one or more modified nucleotides comprise a modification in the ribose group, phosphate group, nucleobase, or a combination thereof. In some aspects of the disclosure, the modification in the ribose group comprises a modification at the 2' position of the ribose group. In some aspects of the disclosure, the modification at the 2' position of the ribose group is selected from the group consisting of 2'-O-methyl, 2'-fluoro, 2'-deoxy, 2'-O-(2-methoxyethyl), and a combination thereof. In some aspects of the disclosure, the modification in the phosphate group comprises a phosphorothioate modification. In some aspects of the disclosure, the one or more modified nucleotides are selected from the group consisting of a 2'-O-methyl nucleotide (M), a 2'-O-methyl, 3'-phosphorothioate nucleotide (MS), a 2'-O-methyl, 3'-thioPACE nucleotide (MSP), and a combination thereof. In some aspects of the disclosure, at least two, three, four, five, six, seven, eight, nine, ten, or more of the nucleotides in the first nucleotide sequence are modified nucleotides and/or at least two, three, four, five, six, seven, eight, nine, ten, or more of the nucleotides in the second nucleotide sequence are modified nucleotides. In some aspects of the disclosure, from about 10% to about 30% of the nucleotides in the first nucleotide sequence are modified nucleotides and/or from about 1% to about 10% of the nucleotides in the second nucleotide sequence are modified nucleotides.

In some aspects of the disclosure according to any one of the methods described above, the safe harbor locus is the IL2Rγ or HBB gene.

In some aspects of the disclosure according to any one of the methods described above, introducing comprises electroporation.

In some aspects of the disclosure according to any one of the methods described above, the donor template further comprises a selectable marker. In some aspects of the disclosure, the selectable marker comprises a marker that is not expressed on a cell of the central nervous system. In some aspects of the disclosure, the selectable marker is a cell surface protein. In some aspects of the disclosure, the cell surface protein is selected from the group consisting of CD1, CD2, CD4, CD8α, CD 10, CD19, CD20, a variant thereof, a fragment thereof, a derivative thereof, and a combination thereof.

In some aspects of the disclosure according to any one of the methods described above, the method further comprises purifying the genetically modified human neural stem cell based on expression of the selectable marker by the genetically modified human neural stem cell. In some aspects of the disclosure, the method further comprises expanding the purified genetically modified human neural stem cell.

Another aspect of the disclosure provides a genetically modified human neural stem cell produced by the methods described above.

Another aspect of the disclosure provides a pharmaceutical composition comprising the genetically modified human neural stem cells described above and a pharmaceutically acceptable carrier.

Another aspect of the disclosure provides a method for preventing or treating a neurodegenerative disease or a neurological injury in a human subject in need thereof, the method comprising: administering to the human subject an effective amount of the pharmaceutical composition described above.

In some aspects of the disclosure according to the method for preventing or treating a neurodegenerative disease or a neurological injury in a human subject described above, the method is for treatment of a neurodegenerative disease selected from the group consisting of a leukodystrophy, neuronal ceroid lipofuscinosis, age-related macular degeneration, Alzheimer's disease, Parkinson's disease, Huntington's disease, amyotrophic lateral sclerosis, and retinal degenerative disease. In some aspects of the disclosure, the method is for treatment of a leukodystrophy selected from the group consisting of Krabbe disease, Pelizaeus-Merzbacher disease, adrenomyeloneuropathy, Alexander disease, cerebrotendineous xanthomatosis, metachromatic leukodystrophy, Canavan disease, leukoencephalopathy with vanishing white matter, adrenoleukodystrophy, Refsum disease, and xenobefantosis. In some aspects of the disclosure, the method is for treatment of a neurological injury selected from the group consisting of spinal cord injury, traumatic brain injury, acute inflammation of the central nervous system (CNS), chronic inflammation of the CNS, ischemia, and stroke.

In some aspects of the disclosure according to any one of the methods for preventing or treating a neurodegenerative disease or a neurological injury in a human subject described above, the genetically modified human neural stem cell is autologous to the subject. In some aspects of the disclosure, the genetically modified human neural stem cell is allogeneic to the subject.

In some aspects of the disclosure according to any one of the methods for preventing or treating a neurodegenerative disease or a neurological injury in a human subject described above, administering comprises administering by injection or surgical transplantation.

Another aspect of the disclosure provides a kit comprising: (a) a donor template comprising: (i) a transgene cassette comprising a transgene; and (ii) two nucleotide sequences comprising two non-overlapping, homologous portions of a safe harbor locus, wherein the nucleotide sequences are located at the 5' and 3' ends of the transgene cassette; (b) a DNA nuclease or a nucleotide sequence encoding the DNA nuclease; and (c) an isolated human neural stem cell.

In some aspects of the disclosure according to the kit described above, the DNA nuclease is selected from the group consisting of a CRISPR-associated protein (Cas) polypeptide, a zinc finger nuclease (ZFN), a transcription activator-like effector nuclease (TALEN), a meganuclease, a variant thereof, a fragment thereof, and a combination thereof.

In some aspects of the disclosure according to any one of the kits described above, the isolated human neural stem cell comprises a primary neural stem cell.

In some aspects of the disclosure according to any one of the kits described above, the isolated human neural stem cell is derived from a somatic stem cell or a pluripotent stem cell, or is derived by direct reprogramming from a somatic cell population.

In some aspects of the disclosure according to any one of the kits described above, the transgene encodes a protein associated with a genetic disorder of the central nervous system. In some aspects of the disclosure, the transgene comprises a gene encoding a neuroprotective or neuroregenerative protein, a variant thereof, a fragment thereof, or a peptide mimetic thereof.

In some aspects of the disclosure according to any one of the kits described above, the nucleotide sequence encoding the DNA nuclease comprises RNA.

In some aspects of the disclosure according to any one of the kits described above, the kit further comprises a DNA-targeting RNA, a truncated DNA-targeting RNA, or a nucleotide sequence encoding the DNA-targeting RNA or truncated DNA-targeting RNA. In some aspects of the disclosure, the DNA nuclease comprises a Cas polypeptide or a nucleotide sequence encoding the Cas polypeptide, and wherein the DNA-targeting RNA comprises a single guide RNA (sgRNA) or a truncated sgRNA comprising a first nucleotide sequence complementary to a portion of the safe harbor locus and a second nucleotide sequence that interacts with the Cas polypeptide. In some aspects of the disclosure, the Cas polypeptide comprises a Cas9 polypeptide, a variant thereof, or a fragment thereof. In some aspects of the disclosure, the Cas polypeptide variant comprises a high-fidelity or enhanced specificity Cas9 polypeptide variant. In some aspects of the disclosure, the sgRNA or truncated sgRNA comprises one or more modified nucleotides. In some aspects of the disclosure, the one or more modified nucleotides are selected from the group consisting of a 2'-O-methyl nucleotide (M), a 2'-O-methyl, 3'-phosphorothioate nucleotide (MS), a 2'-O-methyl, 3'-thioPACE nucleotide (MSP), and a combination thereof.

In some aspects of the disclosure according to any one of the kits described above, the safe harbor locus is the IL2Rγ, or HBB gene.

In some aspects of the disclosure according to any one of the kits described above, the donor template further comprises a selectable marker.

In some aspects of the disclosure according to any one of the kits described above, the kit further comprises instructions for generating a genetically modified human neural stem cell.

Another aspect of the disclosure provides a genetically modified human neural stem cell comprising a transgene cassette comprising a transgene, wherein the transgene cassette is located within a safe harbor locus.

Another aspect of the disclosure provides use of any one of the genetically modified human neural stem cell described above or the genetically modified human neural stem cell produced by any one of the methods described above in a method of identifying or developing a potential therapeutic molecule. In some aspects of the disclosure, the potential therapeutic molecule is a molecule that promotes the proliferation and/or viability of a human neural stem cell and/or the differentiated progeny of a human neural stem cell.

Another aspect of the disclosure provides use of any one of the genetically modified human neural stem cell described above or the genetically modified human neural stem cell produced by any one of the methods described above in research.

### V. Examples

The following examples are offered to illustrate, but not to limit, certain aspects of the disclosure

These examples show the generation of genetically modified human neural stem cells (GM-NSCs) using engineered nucleases such as TALENs and CRISPR/Cas9. Using homologous recombination into a safe harbor locus, an exogenous transgene containing a marker(s) for cell purification and a gene of interest was introduced into the neural stem cells. This example describes methods for generating GM-NSCs and methods for purifying the engineered cells. These examples also show that the purified GM-NSCs engrafted, migrated, and differentiated into neural cell types when transplanted into the brain of an animal model.

Three human gene loci were chosen for homologous recombination (HR) for safe harbors in HuCNS-SC^{®} (human neural stem cells grown as neurospheres). These loci have been extensively characterised for hematologic disorder for HR using TALEN or CRISPR/Cas 9 systems: (i) *IL2RG,* can harbor mutations responsible for severe combined immunodeficiency (SCID)-X1, (ii) *HBB* can harbor mutations responsible for sickle cell anemia and thalassemia, and (iii) *CCR5* encodes a co-receptor of HIV and is currently being investigated as a target for therapeutic gene editing in anti-HIV clinical trials. These loci can serve as "safe-harbors" permitting stable gene expression of exogenous transgenes into the genome of HuCNS-SC^{®} cells without adverse effects.

### A. Methods

To construct the TALEN-mediated genome editing assembly, IL2RG TALENs were synthesised (GenScript) using the Δ152 N-terminal domain and the +63 C-terminal domain as previously described (Miller et al., Nature Biotechnology, 2011, 29:143-148 ) and fused to the FokI nuclease domain and cloned into pcDNA3.1 (Invitrogen) as described (Hendel et al., Cell Report, 2014, dx.doi.org/10.1016/j.celrep.2014.02.040).

To construct the CRISPR/Cas9-mediated genome editing assembly, sgRNA expression vectors were constructed by cloning of 20 bp oligonucleotide target sequences into px330 (Addgene plasmid #42230) containing a human codon-optimised SpCas9 expression cassette and a human U6 promoter driving the expression of the chimeric sgRNA. sgRNA synthesis for the CRISPR/Cas9 system is described in detail in described Hendel et al., Cell Report, 2014, dx.doi.org/10.1016/j.celrep.2014.02.040.

For the targeting vectors, three plasmid targeting vectors carrying about 2 × 800 bp arms of homology were generated by PCR amplification of the corresponding loci using genomic DNA isolated from K562 cells. The homology arms were then cloned into an about 2,900 base pair vector based on pBluescript SK⁺ using standard cloning methods. *See,* Hendel *et al*., *supra*; and Hendel et al., Nature Biotechnology, 2015, 33:985-989 for additional details. Between the homology arms, the donor template included a reporter cassette containing the ubiquitin C promoter driving GFP expression (FIG. 2A). Alternatively, bicistronic cassette constructs separated by a self-cleaving 2A peptide were generated, thereby providing high cleavage efficiency between the two transgenes located upstream and downstream of the 2A peptide (FIG. 8). The constructs included: (1) UbC-GFP-2A-CD8 (CD8 alpha cell surface marker for purification of GM-HuCNS-SC cells; FIG. 11A and 13A); (2) UbC-GFP-2A-tCD19 (truncated cell surface marker for purification of GM-HuCNS-SC cells); (3) UbC-GalC-2A-tCD19 (therapeutic enzyme construct for Krabbe disease with truncated CD19; FIG. 12A) or UbC-GalC-2A-eGFP (FIG. 9A); and (4) UbC-TPP1-2A-CD8 (therapeutic enzyme construct for LINCL with CD8 alpha).

HuCNS-SC^{®} cells were directly isolated from human brain tissue using a method described in Uchida et al., Proc Natl Acad Sci USA, 2000, 97(26): 14720-14725. HuCNS-SC^{®} cells were generated under non-GMP conditions. Cells were cultured at a density of 1×10⁵ per ml in X-VIVO 15 medium (Lonza) supplemented with N2, heparin, N-acetylcysteine, fibroblast growth factor 2, epidermal growth factor (20 ng/ml), and leukemia inhibitory factor (10 ng/ml). Neurospheres were passaged by liberase or benzyme, i.e., highly purified collagenase (Roche) treatment and replated in the same medium. Cell surface markers were used to isolate CD133+ CD24-/lo HuCNS-SC^{®} cells.

The gene targeting donor templates were introduced into the isolated HuCNS-SC^{®}s using nucleofection. Single suspension of 5×10⁵ HuCNS-SC^{®} cells were transfected with 1 µg TALEN-encoding plasmids and 1-2 µg donor plasmid (unless otherwise indicated) by nucleofection (Lonza) with an Amaxa 4D Nucleofector (program CA137) with the P3 Primary Cell Nucleofector Kit (V4XP-3032) with and following by 20 µL 16-well Nucleocuvette strip with manufacturer's instructions. After nucleofection, HuCNS-SC^{®} cells were placed into flasks and continue to culture for multiple passages. In some cases, the cultured HuCNS-SC^{®} cells were used for genome editing upon dissociation with collagenase described in Uchida et al., supra.

An AflII restriction enzyme site was introduced between the 800bp homology arms, thereby creating a Restriction Fragment Length Polymorphism (RFLP) homologous recombination donor. Cas9 (under the CMV promoter) and sgRNA (under the U6 promoter) were delivered in a px330 plasmid construct (1 µg). TALEN pairs were constructed with the golden gate system and delivered via plasmid construct (0.5 µg of each). 500,000 neural stem cells were nucleofected with either 1 µg of the HR donor template or 1 µg of the HR donor and an engineered nuclease. Cells were allowed to grow in culture for 7 days. Genomic DNA was harvested, and In-Out PCR was performed. PCR products were isolated, and digested with AflII overnight. The digestion products were run on 10% PAGE gels and visualised for band intensities. RFLP analysis confirmed insertion of the GFP transgene of the donor template into the IL2Rγ locus.

Flow cytometry was used to monitor homologous recombination. HuCNS-SC^{®} cells were analysed at each passage after nucleofection. GFP expression was measured on an Accuri C6 flow cytometer (BD Biosciences, San Jose, CA, USA). In some cases, expression of transgene CD8 or CD19 was assessed by flow cytometry at each passage. At passage, neurospheres were dissociated as described above and single cell suspension were immunostained with anti-CD8-APC or anti-CD19-APC (Miltneyi Biotec) with following manufacture's instruction.

To purify GM-HuCNS-SC cells carrying a GFP transgene insertion, fluorescence activated cell sorting (FACS) was used. Neurospheres were dissociated as described above and GFP+ cells were sorted on a FACS Aria II (BD Bioscience).

GM-HuCNS-SCs were also purified by magnetic activated cell sorting (MACS). To purify GM-HuCNS-SC cells expressing either a CD8 or CD19 transgene cell surface marker, either human CD8 Microbeads or CD19 MicroBeads (Milenyi Biotech) were used according to the manufacturer's instructions.

To test for engraftment, migration, neuroprotection and retinal preservation of purified GM-HuCNS-SC cells, the cells were transplanted into various immunodeficient mice. A suspension of HuCNS-SC^{®} cells (1×10⁵ cells in 1 µL per site) was prepared and transplanted bilaterally into the corpus callosum, SVZ or cerebellar white matter of neonatal or juvenile shiverer-immunodeficient (Shi-id ) mice.

### B. IL2RG cDNA-UbC-GFP donor template

FIG. 2A shows a schematic of the IL2RG cDNA-UbC-GFP homologous recombination donor template that homologously recombined into the IL2RG locus. Each flanking arm included about 800 bps of exon 1 of the IL2RG gene. HuCNS-SC^{®} cells were nucleofected with 1 µg HR donor or with 1 µg HR donor and a nuclease. Cells were grown for 70 days and FACS every ~10 days until episomal HR donor diluted out of proliferating cells to confirm stable expression of integrated GFP cassette into IL2RG locus. The data confirms that IL2RG is a suitable locus for HR using engineered nucleases in HuCNS-SC^{®} cells.

CRISPR/Cas9 stimulated higher frequencies of gene editing at the IL2Rγ locus. FIGS. 1A and 1B show that that both CRISPR/Cas9 and TALEN systems mediated homologous recombination (HR) at a frequency of about 1-6%. The CRISPR/Cas9 system mediated 2-fold more HR events than the TALEN system.

The level of long-term stable GFP expression (2-4% GFP) was consistent with the percentage of homologous recombination determined in the RFLP analysis (1.7-3% HR frequency) (FIGS. 2B and 2C). An image of GFP⁺ HuCNS-SC^{®} cells that were gene edited using CRISPR/Cas9 technology is provided in FIG. 3.

FIG. 4A shows that HuCNS-SC^{®} cells carrying the IL2RG cDNA-UbC-GFP donor template engrafted into the brain of shiverer mice after transplantation. FIG. 4B shows that the GFP-positive HuCNS-SC^{®} cells developed into myelin producing GM-oligodendrocytes.

### C. Increasing homologous recombination frequency using RNA-based CRISPR/Cas9 system

The gene editing efficiency of HuCNS-SC^{®} cells using a plasmid-based versus RNA-based CRISPR/Cas9 systems was compared. Cas9 was delivered as an mRNA and included chemical modifications comprising 2'-*O*-methyl (M), 2'-*O*-methyl 3'phosphorothioate (MS), or 2'-*O*-methyl 3'thioPACE (MSP) incorporated at three terminal nucleotides at both the 5' and 3' ends. HuCNS-SCs were nucleofected with the plasmid-based and RNA-based nucleases. After 7 days in culture, genomic DNA was harvested and PCR was performed to amplify the targeted region. FIG. 5 shows that the RNA based CRISPR/Cas9 system induced more double stranded breaks (INDELS) compared to the plasmid based system.

HR frequency was also assessed by FACS. HuCNS-SC^{®} cells were nucleofected with the HR donor alone or the HR donor plus engineered nuclease (plasmid (FIG. 6A) or MS modified RNA or MSP MS modified RNA (FIG. 6B)). Cells were analysed every 10 days for a total of 30 days via FACS to allow episomal HR donor to dilute out. The data also shows that the MSP-modified RNA based CRISPR/Cas9 increased HR frequency by about 2-fold compared to plasmid delivery of Cas9. The MS modification did not enhance HR activity even though it did increase INDEL efficiencies.

### D. IL2RG cDNA-UbC-GalC-2A-GFP donor template

FIG. 7 shows a schematic of an exemplary IL2RG cDNA-UbC-GalC-2A-GFP donor template. An engineered nuclease can induce a double-strand break (DSB) that can be repaired by homology-directed repair via a donor template. By co-delivering the nuclease with the donor template (ubiquitin C promoter-galactosylceramidase transgene-2A-GFP transgene) having locus-specific homology arms (IL2RG-specific flanking arms), the donor template can be inserted into the IL2RG locus.

In this experiment, isolated HuCNS-SCs were nucleofected with 1 µg of IL2RG cDNA-UbC-GalC-2A-GFP donor template alone, or a combination of 1 µg of IL2RG cDNA-UbC-GalC-2A-GFP donor template and an engineered nuclease (CRISPR/Cas9 or TALEN). Cells were grown for 70 days and FACS every about 10 days until the episomal donor template was diluted out of the proliferating cells and stable expression of the integrated GFP cassette into the IL2RG locus was confirmed. Cells were cultured for 56 days and then sorted to select GFP positive cells. These cells were then sorted for an additional 140 days.

FIG. 8 shows that the transgenes were stably expressed in the purified GM-HuCNS-SC cells and that GalC and GFP expression was maintained in long-term cultures (about 168 days post nucleofection). The data confirms that after 30 days to allow the episomal HR donor template to dilute out, sorting of GFP-positive cells enriches a pure population of gene edited HuCNS-SC^{®} cells.

PCR analysis was performed to confirm targeted integration of the GalC gene into the IL2RG locus. The forward primer is situated in the GalC transgene and the reverse primer is located outside the 3' homology arm and is not present in the donor template. As such, PCR should only amplify the targeted integration and generated an expected amplicon of 2355 bases in length. Isolated HuCNS-SC cells were nucleofected with 1 µg of IL2RG cDNA-UbC-GalC-2A-GFP donor template and either a TALEN or CRISPR/Cas9 nuclease. The cells were cultured for 7 days and genomic DNA was harvested. In-Out PCR was performed and the PCR products were run on a 1% agarose gel. FIG. 9B confirms engineered nuclease-mediated homology-directed repair with the GalC donor.

GFP⁺ cells were purified by flow cytometry and passaged for long-term culturing/expansion (over 160 days). Upon purification, GFP expression and Sox2 expression were monitored. For the CRISPR/Cas9 engineered cells, FIG. 10A shows that GFP expression was stable after expansion and that the sorted and expanded cells were Sox2 positive. FIG. 10B shows similar results for the TALEN engineered cells.

### E. IL2RG cDNA-UbC-GalC-2A-CD8 and IL2RG cDNA-UbC-GalC-2A-tCD19 donor templates

The GFP transgene described above was replaced with either a CD8 or CD19 (or truncated CD19) cell surface marker. Cells expressing these markers can be selectively purified using, for example CD8 or CD19 selection microbeads. FIG. 11A and 13A show schematics of an exemplary IL2RG cDNA-UbC-GalC-2A-CD8 donor template. FIG. 12A shows a schematic of an exemplary IL2RG cDNA-UbC-GalC-2A-CD8 donor template.

Isolated HuCNS-SCs were nucleofected with 1 µg of IL2RG cDNA-UbC-GalC-2A-CD8 donor template or IL2RG cDNA-UbC-GalC-2A-tCD19 donor template, and either a TALEN or CRISPR/Cas9 nuclease.

Flow cytometry analysis of cells expressing the IL2RG cDNA-UbC-GALC-2A-CD8 transgene is shown in FIGS. 11B (pre-MACS selection) and 11C (post-MACS selection). The differential potential of the GM-HuCNS-SC cells was evaluated. For the in vitro neuronal differentiation assay, the GM-HuCNS-SC cells were cultured without mitogen and supplemented with BDNF and GDNF for 10 days. The GM-HuCNS-SCs were multipotent and capable of differentiating into neuronal (doublecortin), astrocyte (GFAP) and oligodendrocyte (O4, day 14; FIG. 11D) lineages.

Another population of cells expressing the IL2RG cDNA-UbC-GalC-2A-CD8 transgene was analysed for expression of the cell surface marker CD8 and GFP. FIGS. 13B (pre-MACS selection) and 13C (post-MACS selection) shows an increase in CD8⁺,GFP⁺ cells after selection. FIG. 13D shows that cells that passed through the CD8 selection column were CD8⁻,GFP⁻ cells. FIG. 13E shows that a high percentage of the CD8 selected cells are also GFP+. The GM-HuCNS-SC cells were expanded and GFP was stable in these cells (FIG. 13F). Single cell suspensions were prepared for transplantation into neonatal shiverer mice. 12 weeks post transplantation, brain sections of the mice were analysed. Robust engraftment and migration of the GM-HuCNS-SC cells was observed (FIGS. 14B and 14C).

Flow cytometry analysis of cells expressing the IL2RG cDNA-UbC-GALC-2A-tCD19 transgene is shown in FIGS. 12C (pre-MACS selection) and 12D (post-MACS selection). Upon purification, the GM-HuCNS-SC cells retained transgene expression during long-term culturing/expansion (FIG. 12B).

### F. IL2RG cDNA-UbC-GalC-2A-tCD19 donor template with RNA-based CRISPR/Cas9 system

In this experiment, isolated HuCNS-SC cells were nucleofected with 1 µg of IL2RG cDNA-UbC-GalC-2A-tCD19 donor template and either a plasmid-based sgRNA/Cas9 nuclease or a MSP-modified sgRNA with Cas9 mRNA. The cells were purified using the cell surface marker tCD19, and the selected cells were further expanded. Using the purification strategy, a pure population of CD19 positive cells was generated. After expansion, the purified population maintained cell surface expression of CD19. The data shows that MSP modification increased HDR frequency compared to using a plasmid-based Cas9. *See,* FIGS. 15A-15F.

### G. TALEN or CRISPR/Cas9-mediated homologous recombination (HR) in NSCs

We first compared genome-editing efficiencies at the NSCs "safe harbor" locus, interleukin-2 receptor gamma chain (*IL2Rγ*), in NSCs using *IL2Rγ*-specific TALEN pairs and the CRISPR/Cas9 system. Neural stem cell cell-specific safe harbors for gene addition were classified on two criteria: 1) no known or reported biological function(s) in NSCs and 2) sufficient gene expression in the endogenous locus with an exogenous promoter, both of which are true for *IL2R*γin NSCs. To characterize the frequency of DSBs induced, we electroporated plasmids encoding either optimised *IL2Rγ*-specific TALEN pairs or CRISPR components (Cas9 and sgRNA), cultured cells for 7 days, isolated genomic DNA (gDNA) and then analysed analysed INDEL frequencies. The CRISPR system generated an average of 15% INDELs compared to 5% using the TALEN (FIG. 33A). We next compared mRNA delivery of *IL2Rγ* TALEN pairs to mRNA delivery of Cas9 along with chemically modified sgRNAs ("All RNA"). Electroporating NSCs with the All RNA CRISPR system induced 5 fold more INDELs than mRNA delivery of TALENs (FIG. 33B). Since both engineered nuclease systems were inducing DSBs (albeit at different frequencies), we targeted NSCs for HR with a plasmid encoding a homologous *IL2Rγ*cDNA-UbC-GFP donor cassette (FIG. 16), or a plasmid that introduced single nucleotide changes. Targeted NSCs were cultured for at least 30 days to allow episomal donor expression to dilute out to allow GFP FACS analysis of HR of stably integrated cassettes. While plasmid-based delivery of both engineered nuclease platforms mediated stable GFP expression in an average of 3.5% of NSCs (FIG. 33C), the CRISPR system consistency modified more *IL2Rγ* alleles (FIG. 33D). These data suggested that the CRISPR system was superior to TALENs, and therefore, was the nuclease used for the remainder of the studies described herein.

We next tested whether the CRISPR system could edit two other NSCs "safe harbor" loci, the chemokine (C-C motif) receptor 5 (*CCR5*) and β-globin (*HBB*) genes, which neither have been reported to function during human neurogenesis (and that is why we consider them be safe loci to target in NSCs). Plasmids encoding Cas9 and sgRNAs specific to *IL2Rγ*, *HBB,* and *CCR5* were electroporated into NSCs and 7 days later, gDNA was extracted and alleles were analysed for INDEL frequencies. The CRISPR-Cas9 system induced an average of 12%, 6%, and 36% INDELs at the *IL2Rγ*, *HBB,* and *CCR5* loci, respectively (FIG. 18). Consistent with our previous reports, delivery of Cas9 as mRNA and chemically modified sgRNAs induced more INDELs than plasmid delivery at all three loci tested (FIG. 18). To see if DSB formation promoted HR at all 3 loci, NSCs were co-electroporated with homologous DNA donor templates encoding GFP, and corresponding CRISPR components delivered as plasmid or All RNA. Our results showed that all 3 loci were amenable to HR in NSCs using plasmid or All RNA delivery of CRISPR-Cas9 components (FIG. 19 and FIGS. 17A-17B). Targeting NSCs at *IL2Rγ*, *HBB,* and *CCR5* loci with plasmid delivery of CRISPR, resulted in an average of 2.8%, 4.1%, and 2.4% GFP⁺ NSCs after at least 30 days in culture, respectively (FIG. 20). Interestingly, ALL RNA delivery of *IL2Rγ* CRISPR resulted in 2-fold more GFP⁺ NSCs than plasmid delivery. To determine if HR occurred at the intended loci, gDNA was analysed for on-target integration of the donor construct by In-Out PCR (where one primer binds outside the homology arm of the donor construct and the other primer binds inside the donor cassette), thus only on-target integration events can be amplified. While PCR amplification was not detected with mock or donor only samples, on-target integration was evident when NSCs were co-electroporated with CRISPR and a homologous donor, confirming HR at the intended locus (FIG. 21). Because Cas9 can create DSBs at unintended genomic locations, we investigated off-target DSB activity at 3 predicted *IL2Rγ* off-target sites that we have previously assayed by next generation sequencing (NGS) in K562 cells. While we detected 28% DSB activity at *IL2Rγ*, less than 0.7% cleavage was seen at any of the off-target sites investigated (Table 1). Collectively, these studies demonstrated that CRISPR-Cas9 can mediate on-target HR at multiple "safe harbor" loci in NSCs with minimal off-target cleavage.

**Table 1: Low off-target activity of IL2RγCRISPR/Cas9 system in human NSCs. Targeted deep sequencing of IL2Rγ alleles and three previously predicted off-target sites was performed on unedited NSCs and GM-NSCs. Each column represents an independent targeting experiment from GM-NSCs that were unselected, CD8 selected, or tCD19 selected. All numbers are subtracted from background INDEL frequencies in an unedited NSCS sample.**

| | Unselected | CD8 Selected | CD8 Selected | tCD19 Selected |
|---|---|---|---|---|
| IL2R*γ* | 28.35 | 75.85 | 59.11 | 71.27 |
| Off Target 1 | 0 | 0 | 0.01 | 0 |
| Off Target 2 | 0 | 0.28 | 0.07 | 0 |
| Off Target 3 | 0 | 0 | 0 | 0.62 |

### Methods

Three human gene loci were chosen for homologous recombination (HR) for safe harbors in NSCs (human neural stem cells grown as neurospheres). These loci have been extensively characterised for hematologic disorder for HR using TALEN or CRISPR/Cas 9 systems: (i) *IL2Rγ*, can harbor mutations responsible for severe combined immunodeficiency (SCID)-X1, (ii) *HBB* can harbor mutations responsible for sickle cell anemia and thalassemia, and (iii) *CCR5* encodes a co-receptor of HIV and is currently being investigated as a target for therapeutic gene editing in anti-HIV clinical trials. These loci can serve as "safe-harbors" permitting stable gene expression of exogenous transgenes into the genome of NSCs cells without adverse effects.

To construct the TALEN-mediated genome editing assembly, IL2RG TALENs were synthesised (GenScript) using the Δ152 N-terminal domain and the +63 C-terminal domain as previously described (Miller et al., Nature Biotechnology, 2011, 29:143-148 ) and fused to the FokI nuclease domain and cloned into pcDNA3.1 (Invitrogen) as described (Hendel et al., Cell Report, 2014, dx.doi.org/10.1016/j.celrep.2014.02.040).

To construct the CRISPR/Cas9-mediated genome editing assembly, sgRNA expression vectors were constructed by cloning of 20 bp oligonucleotide target sequences into px330 (Addgene plasmid #42230) containing a human codon-optimised SpCas9 expression cassette and a human U6 promoter driving the expression of the chimeric sgRNA. sgRNA synthesis for the CRISPR/Cas9 system is described in detail in described Hendel et al., Cell Report, 2014, dx.doi.org/10.1016/j.celrep.2014.02.040.

To chemically modify sgRNAs, Cas9 was delivered as an mRNA and the chemical modifications comprising 2'-*O*-methyl (M), 2'-*O*-methyl 3'phosphorothioate (MS), or 2'-*O*-methyl 3'thioPACE (MSP) were incorporated at three terminal nucleotides at both the 5'_and 3'_ends, and thus represents the "All RNA" delivery of the CRISPR system.

For the targeting vectors, three plasmid targeting vectors (such as CCR5 and IL2RG plasmid targeting vectors) carrying about 2 × 800 bp arms of homology were generated by PCR amplification of the corresponding loci using genomic DNA isolated from K562 cells. HBB had homology arms of 540bp and 420bp. The homology arms were then cloned into an about 2,900 base pair vector based on pBluescript SK⁺ using standard cloning methods. *See,* Hendel *et al*., *supra*; and Hendel et al., Nature Biotechnology, 2015, 33:985-989 for additional details. Between the homology arms, the donor template included a reporter cassette containing the ubiquitin C promoter driving GFP expression (FIG. 2A). Alternatively, bicistronic cassette constructs separated by a self-cleaving 2A peptide were generated, thereby providing high cleavage efficiency between the two transgenes located upstream and downstream of the 2A peptide (FIG. 8). The constructs included: (1) UbC-GFP-2A-CD8 (CD8 alpha cell surface marker for purification of GM-NSCs; FIG. 11A and 13A); (2) UbC-GFP-2A-tCD19 (truncated cell surface marker for purification of GM-NSCs); (3) UbC-GalC-2A-tCD19 (therapeutic enzyme construct for Krabbe disease with truncated CD19; FIG. 12A) or UbC-GalC-2A-eGFP (FIG. 9A); and (4) UbC-TPP1-2A-CD8 (therapeutic enzyme construct for LINCL with CD8 alpha).

NSCs were directly isolated from human brain tissue using a method described in Uchida et al., Proc Natl Acad Sci USA, 2000, 97(26):14720-14725. NSCs were generated under non-GMP conditions. Cells were cultured at a density of 1×10⁵ per ml in X-VIVO 15 medium (Lonza) supplemented with N2, heparin, N-acetylcysteine, fibroblast growth factor 2, epidermal growth factor (20 ng/ml), and leukemia inhibitory factor (10 ng/ml). Neurospheres were passaged by liberase or benzyme, i.e., highly purified collagenase (Roche) treatment and replated in the same medium. Cell surface markers were used to isolate CD133+ CD24-/lo NSCs.

The gene targeting donor templates were introduced into the isolated NSCs using nucleofection. Single suspension of 5×10⁵ NSCs cells were transfected with 1 µg TALEN-encoding plasmids and 1-2 µg donor plasmid (unless otherwise indicated) by nucleofection (Lonza) with an Amaxa 4D Nucleofector (program CA137) with the P3 Primary Cell Nucleofector Kit (V4XP-3032) with and following by 20 µL 16-well Nucleocuvette strip with manufacturer's instructions. Alternatively, 2.5E6 GM-NSCs were transfected with 5 µg TALEN-encoding plasmids and 5-10 µg donor plasmid (unless otherwise indicated) by nucleofection (Lonza) with an Amaxa 4D Nucleofector (program CA137) with the P3 Primary Cell Nucleofector Kit (V4XP-3024) by 100 microL Nucelocuvette with following manufacturer's instructions. After nucleofection, NSCs were placed into flasks and continue to culture for multiple passages. In some cases, the cultured NSCs were used for genome editing upon dissociation with collagenase described in Uchida et al., supra.

An AflII restriction enzyme site was introduced between the 800bp homology arms, thereby creating a Restriction Fragment Length Polymorphism (RFLP) homologous recombination donor. Cas9 (under the CMV promoter) and sgRNA (under the U6 promoter) were delivered in a px330 plasmid construct (1 µg). TALEN pairs were constructed with the golden gate system and delivered via plasmid construct (0.5 µg of each). 500,000 neural stem cells were nucleofected with either 1 µg of the HR donor template or 1 µg of the HR donor and an engineered nuclease. Cells were allowed to grow in culture for 7 days. Genomic DNA was harvested, and In-Out PCR was performed. PCR products were isolated, and digested with AflII overnight. The digestion products were run on 10% PAGE gels and visualised for band intensities. RFLP analysis confirmed insertion of the GFP transgene of the donor template into the IL2Rγ locus. The amount of modified IL2RG alleles was quantified by dividing the density of cut alleles (800bp) by total alleles (unmodified (1.6kb) and modified).

Targeted amplicon library generation for MiSeq runsIL2RG (ON) and the top three in silico predicted off target (OFF1-3) Cas9-sgRNA amplicons were PCR-amplified with sequencing primers utilised in deep sequencing MiSeq runs as previously reported. Amplicons were gel purified and then subjected to a second round of PCR to add adapters and unique 8bp barcodes to distinguish experiments. Barcoded amplicons were then purified and pooled in equimolar concentrations. The purified library was sequenced on an illumina MiSeq DNA sequencer at 2 × 200 cycles with indexing at the Protein and Nucleic Acid (PAN) Stanford Core Facility. Sequences were aligned to the human genome and INDELs were calculated as described below.

MiSeq analyses of in silico predicted IL2RG sgRNA off-target sites

IL2RG (ON) and top three in silico predicted OFF-target Cas9-sgRNA sites were quantified by mapping reads from each samples to the four amplicon sequence target regions (IL2RG, OFF1-3) and measuring number of mapped reads with a gap in the neighborhood of the cut site. Specifically, each read was first assigned to one of the target regions by finding a perfect match between the first 70 bases of each read and the amplicon sequence. If the first 70 base pairs of a read do not perfectly map to any amplicon sequence the read is discarded. Each read was then aligned with its corresponding target amplicon sequence using EMBOSS version 6.5.7.0 needle with default parameters. To quantify the number of reads with INDELs, each read was marked as modified if at least one insertion or deletion (a "-" in the alignment of either read or the amplicon) occurs within 5bp up or downstream of the CRISPR cut site (between bases 17 and 18 of the guide RNA sequence). The overall INDEL percentage at a given target site was reported as the number of reads mapped to the locus that are modified over the total number of reads mapped to the locus, minus the background INDEL percentage in an non-electroporated sample.

### H. Enrichment of genetically modified NSCs by magnetic activated cell sorting (MACS) selection

Enrichment and expansion of GM-NSCs to clinically relevant transplantable cell numbers would greatly increase their translatable potential. We devised an enrichment paradigm by establishing alpha chain of CD8 (CD8α) cell surface marker expression following successful HR at *IL2RG* coupled with a one-step magnetic activated cell sorting (MACS) enrichment. CD8α complex requires dimerization of alpha and beta chains to transduce intercellular signals in cytotoxic T cells. Of note, CD8α has not been reported to be expressed or functional and expression of alpha chain alone should influence in NSCs, thereby solely serving as a selection transgene. We created an *IL2RG* HR donor construct (*IL2RG* cDNA-UbC-GFP-T2A- CD8α) that upon successful targeting, would express a multigene mRNA, but two distinct GFP and CD8α proteins. NSCs were electroporated with a plasmid encoding Cas9 and sgRNA specific to *IL2RG* and also the CD8α HR donor cassette described above. Six cell passages post electroporation, 4.67% of NSCs were stably expressing GFP (FIG. 22A, left), similar to what we found with the GFP construct (FIG. 19). Then NSCs were subjected to a one-step CD8α magnetic bead enrichment protocol that resulted in >90% of cells expressing GFP (FIG. 22A, right), and these cells were shown to expand and continually express GFP for multiple passages post-selection, confirming they were a population of GM-NSCs (FIG. 22B).

In addition to CD8α, we also evaluated CD19 cell surface protein for our MACS-based enrichment protocol. Unlike CD8 complex, CD19 is a single chain molecule to transduce signal (ref). Therefore we created a truncated form of the CD19 cell surface protein, where the intracellular signaling domain is removed, thus making CD19 solely serve the purpose of selection. Again, CD19 has no known role in neurogenesis or gliogenesis. We therefore created an *IL2RG* HR donor construct (*IL2RG* cDNA-UbC-GFP-T2A-tCD19), and targeted NSCs for HR at the *IL2RG* locus. At passage four-post electroporation, 3.78% of NSCs were stably expressing GFP and cells were then subjected to MACS-based enrichment of tCD19 (FIG. 23A, left). tCD19 selection resulted in enrichment and expansion of >90% of GM-NSCs, similar to CD8 selection of GM-NSCs (FIG. 22B, FIG. 23B, and FIG. 24). Because we are enriching cells with HR, we next wanted to investigate whether we were also selecting GM-NSCs with a higher frequency off-target INDELs. While we observed a 3-fold enrichment of *IL2RG* INDELs (because cells are female with 2 x chromosomes), off-target INDELs were all less than 1% in CD8 and tCD19 selected GM-NSCs (Table 1).

To determine if GM-NSCs maintained their NSCS characteristics, we enriched *IL2RG* targeted NSCs to over 95% (FIG. 24) via the tCD19 selection scheme and then analysed the expression of the two quintessential NSCs markers: the cell surface gene CD133 (FIG. 25A), and the intracellular NSCS-maintaining transcription factor, SOX2 (FIG. 25B). Following 4 weeks of expansion post-enrichment, greater than 95% of NSCs were CD133/CD19/GFP/SOX2 positive, highlighting that targeted neurospheres may fully possess NSC potential. These data detail a simplified one-step MACS-based protocol for enriching and then expanding >90% of GM-NSCs for transplantation.

### Methods

Flow cytometry was used to monitor homologous recombination. NSCs cells were analysed at each passage after nucleofection. GFP expression was measured on an Accuri C6 flow cytometer (BD Biosciences, San Jose, CA, USA). In some cases, expression of transgene CD8 or CD19 was assessed by flow cytometry at each passage. At passage, neurospheres were dissociated as described above and single cell suspension were immunostained with anti-CD8-APC or anti-CD19-APC (Miltneyi Biotec) with following manufacture's instruction.

To purify GM-NSCs carrying a GFP transgene insertion, fluorescence activated cell sorting (FACS) was used. Neurospheres were dissociated as described above and GFP+ cells were sorted on a FACS Aria II (BD Bioscience).

GM-NSCs were also purified by magnetic activated cell sorting (MACS). To purify GM-NSCs expressing either a CD8 or CD19 transgene cell surface marker, either human CD8 Microbeads or CD19 MicroBeads (Milenyi Biotech) were used according to the manufacturer's instructions.

### I. GM-NSCs migrate and differentiate into neurons, astrocytes and oligodendrocytes in vivo

While we have shown that we can target NSCs for HR using the CRISPR/Cas9 system, we also investigated if GM-NSCs still retain their NSC biological activity as previously shown for non-edited NSCs. Specifically, we targeted the *IL2RG* locus in NSCs with a GFP cassette (FIG. 16), expanded GM-NSCs and then transplanted them into immunodeficient mice. CD8α -purified GM-NSCs were transplanted bilaterally into the subventricular zone (SVZ) of neonatal Shi-id mice and then after 12 weeks, sibling forebrain sections from same animal were analysed for engraftment of human modified cells by immunohistochemistry (FIGS. 14B-14C). Immunoperoxidase staining with the human-specific mAb SC121 (FIG. 14B) and GFP (FIG. 14C) detected engraftment of GM-NSCs in the cortex, corpus collusum and importantly, showed migration of cells from the SVZ to the olfactory bulb along the rostral migratory stream (RMS) (FIGS. 14B-14C). It is important to note that transgene GFP expression is very similar to human cells engraftment detected by SC121. Hence, GM-NSCs can engraft and migrate in the SVZ/Olfactory system, comparable to non-edited NSCs we reported previously.

The hallmark function of human NSCs is their ability to self-renew while differentiating into neuron, astrocyte and oligodendrocyte lineages in long-term. Twenty four weeks after transplant into Shi/+ heterozygous-id mice, GFP+ cells were detected in the hippocampus, suggesting that GM-NSCs maintain robust migratory capacity in long-term. In addition, some of these cells expressed GM-NSC transcription factor SOX2 in the subgranular layer of dentate gyrus of the hippocampus, which is neurogenic site in adult rodent besides SVZ (FIG. 26A arrows). These data suggests that GM-NSCs migrated into the neurogenic site and maintain NSC self-renewal potentials after expansion *in vivo.* To confirm GM-NSCs were able to differentiate into astrocytic or neuronal lineages *in vivo,* we performed confocal microscopy on brain sections for the astrocytic, neuronal markers, glial fibrillary acidic protein (GFAP) and doublecortin (DCX), respectively. We observed GFP positive cells costained with GFAP in the corpus collasum or white matter bundle of the striatum (FIG. 26B). Furthermore, GM-NSCs migrated into the RMS and differentiated into DCX + neuronal lineage in the olfactory bulb (FIG. 26B), revealing that migration and differentiation of GM-NSC into astrocytic and neuronal lineages in site-appropriate manner.

Differentiation potential of GM-NSC into oligodendrocytes was evaluated oligodendrocyte mutant shiverer-immunodeficient mice (shi/shi-id) as described previously (Uchida STM). The shi/shi-id mouse has deletion in major basic protein (MBP) and mAb against MBP does not stain shi/shi mouse brains (ref). Eight weeks post transplantation, GM-NSCs migrated extensively within the white matter tract of the cerebellum (FIG. 27A) and express transgene GFP (FIG. 27B). Immunofluorescence staining reveals that the engrafted white matter display co-expression of GFP and MBP (FIG. 27C). In a higher magnification of a different mouse brain, we observed human GFP+ cells extended their processes and MBP expression was localised at the tip of their process, indicative of mature myelinating oligodendroctyes (FIG. 4B). To put all together, GM-NSCs maintain potential to engraft, migrate, self-renew and differentiate into 3 CNS lineages in site appropriate manner, comparable to unedited NSCs as previous reported. In addition progeny of GM-NSCs persist to express transgene mediated by Crispr/Cas9, including mature myelinating oligodendrocytes.

### Methods

To test for engraftment, migration, neuroprotection and retinal preservation of purified GM-NSCs, the cells were transplanted into various immunodeficient mice. A suspension of NSCs (1×10⁵ cells in 1 µL per site) was prepared and transplanted bilaterally into the corpus callosum, SVZ or cerebellar white matter of neonatal or juvenile shiverer-immunodeficient (Shi-id ) mice.

### J. GALC lysosomal enzyme overexpressing NSCs engraft and produce myelin in vivo

Lysosomal storage disorders (LSDs) are a group of more than fifty inherited monogenic metabolic disorders that result from the lysosomal inefficiencies. Among them, Globoid cell leukodystrophy, or Krabbe disease, is a type of LSD that mainly manifests in the central and peripheral nervous systems due to the loss of galactosylceramidase (*GALC*)*,* which cause death of myelin producing, oligodendrocytes and Schwann cells, respectively. NSCs have been transplanted into brains of NCL patients to deliver missing enzyme and into Pelizaeus Merzbacher Disease (PMD) patients to provide myelin-producing cells. Since we show that GM-NSCs produced myelin comparable to unedited NSCs, we believed that GM-NSCs overexpressing *GALC* would be superior in terms of producing enough GALC to enable "cross-correction" of damaged myelin-producing cells moreover, these cells could also replace already lost oligodendrocytes with 'normal' GALC expressing oligodendrocytes. Therefore, we created a HR donor to knock-in *GALC* (*IL2RG* cDNA-UbC-GALC-T2A-tCD19) into the *IL2RG* locus and also a tCD19 selection cassette to enable robust MACS-based enrichment (FIG. 28). NSCs were electroporated with the "All RNA" *IL2RG* CRISPR/Cas9 platform and the *GALC* HR donor with 5.17% CD19 positive NSCs after episomal donor DNA was diluted out at least four passages post-targeting (FIG. 29A). Consistently, tCD19 MACS beads enriched >95% of GM-NSCs (FIG. 29B) and 'In-Out' PCR confirmed on-target integration of the *GALC* cassette into the *IL2RG* locus (FIG. 30). To confirm that GALC NSCs were overexpressing functional enzyme, we performed an *in vitro* GALC enzyme assay. Accordingly, GM-NSCs expressed 3-5 fold more GalC enzyme than unedited NSCs and 10 fold more than fibroblasts from a Krabbe disease patient (FIG. 31). We next tested if *GALC* GM-NSCs retained their NSCS biological characteristics; therefore, we transplanted *GALC* GM-NSCs into the cerebellum of juvenile Shi-id mice. Immunohistochemical analyses showed engraftment and myelin production of *GALC* GM-NSCs (FIGS. 32A-32B). These data highly suggest that *GALC* GM-NSCs retain their NSCS biological characteristics and have high therapeutic potential for the treatment of Krabbe disease and other demyelinating central nervous system disorders.

### Methods

GALC enzyme assay was performed (Wiederschain et al. Clin Chim Acta 1992) as described in R&D System (https://www.rndsystems.com/products/recombinant-human-galactosylceramidase-galc-protein-cf_7310-gh). Briefly, protein extractions from unedited NSC, fibroblasts from Krabbe patient (GM4372 Fibroblast, heterozygous for a 30 kb deletion beginning near the middle of intron 10 in the GALC gene, Coriell) and two different GE-NSCs modified with IL2RG cDNA-UbC-GALC-T2A-tCD19 donor template (Exp1) or IL2RG cDNA-UbC-GALC-T2A-GFP (Exp2). To perform GALC enzyme assays, protein extracts from these cells were prepared by harvesting cells, wash once with PBS, and treat with M-PER protein extraction reagent (Thermo Scientific) according manufacture's instruction. Protein concentration was measured by Bradford assay kit with BSA standard curve, ranging 0.25-2.0 mg/ml (Thermo Scientific).

The GALC assay was performed by missing 250 ug or 500 ug protein extract (50 uL) with 50 uL of 1 mM 4-methylumbelliferyl-beta -D-galactopyranoside substrate (Sigma) resuspended in 50 mM Sodium Citrate, 125 mM NaCl, 0.1% Triton^{®} X-100,pH 4.5. Reactions were incubated 20 min at 37 °C and then stopped with 0.5 M Glycine, 0.3 M NaOH pH 10.0. Fluorescence of GALC enzyme cleavage was measured by BioTek with Gen5 software at excitation and emission wavelengths of 365 nm and 445 nm (top read), respectively, in endpoint mode. Standard curve for GALC enzyme assay was established by recombinant human Galactosylceramidase (rhGALC) (R&D Systems, # 7310-GH), ranging from 0.0375 ng-1.2 ng of rhGALC per reaction with linear range. All assays were done in triplicate using three independent samples. GALC enzyme activity were normalised with unedited NSCs.

Although the foregoing disclosure has been described in some detail by way of illustration and example for purposes of clarity of understanding, one of skill in the art will appreciate that certain changes and modifications may be practiced within the scope of the appended claims.

## Claims

1. A method for generating a genetically modified human neural stem cell, the method comprising:
introducing into an isolated human neural stem cell:
(a) a donor template comprising: (i) a transgene cassette comprising a transgene; and (ii) two nucleotide sequences comprising two non-overlapping, homologous portions of a safe harbor locus, wherein the nucleotide sequences are located at the 5' and 3' ends of the transgene cassette; and
(b) a DNA nuclease or a nucleotide sequence encoding the DNA nuclease, wherein the DNA nuclease is a CRISPR-associated protein (Cas) polypeptide capable of creating a double-strand break in the safe harbor locus to induce insertion of the transgene into the safe harbor locus, thereby generating a genetically modified human neural stem cell,
wherein the safe harbor locus is the IL2Rγ or HBB gene.

2. The method of claim 1, wherein the isolated human neural stem cell comprises a primary neural stem cell or is derived a) from a somatic stem cell, b) from a pluripotent stem cell, or c) by direct reprogramming from a somatic cell population.

3. The method of claim 1 or 2, wherein the transgene encodes a protein associated with a genetic disorder of the central nervous system, optionally the protein is encoded by a gene selected from the group consisting of *GALC* (Krabbe disease), *ABCD1* (adrenoleukodystrophy), *GFAP* (Alexander disease), *CYP27A1* (cerebrotendineous xanthomatosis), *ARSA* (metachromatic leukodystrophy), *PLP1* (Pelizaeus-Merzbacher disease), *ASPA* (Canavan disease), *EIF-2B* (leukoencephalopathy with vanishing white matter), *PHYH* (Refsum disease 1), *PEX7* (Refsum disease 2), *PPT1* (infantile neuronal ceroid lipofuscinosis (NCL)), *TPP1* (late infantile NCL), *CLN3* (juvenile NCL), *CLN6* (adult NCL), *CLN5* (Finnish late infantile variant NCL), *CLN6* (late infantile variant NCL), *MSFD8* (ceroid lipofuscinosis, neuronal, 7), *CLN8* (ceroid lipofuscinosis, neuronal, 8), *CTSD* (ceroid lipofuscinosis, neuronal, 10), *UBE3A* (Angelman syndrome), *POLG* (Alpers' Disease), *TAZ* (Barth Syndrome), *GLA* (Fabry disease), *SLC20A2* (Fahr's syndrome), *PDE* (retinitis pigmentosa), *SMN1* (spinal muscular atrophy), *IKBKAP* (familial dysautonomia), *MeCP2* (Rett syndrome), *CACNA1C* (Timothy syndrome), *ATXN3* (Machado-Joseph disease), and *RPE65* (Leber congenital amaurosis), optionally wherein the protein is selected by the genetically modified human neural stem cell.

4. The method of claim 1 or 2, wherein the transgene encodes a neuroprotective or neuroregenerative protein, a variant thereof, a fragment thereof, or a peptide mimetic thereof, optionally wherein the neuroprotective or neuroregenerative protein is selected from the group consisting of brain-derived neurotrophic factor (BDNF), insulin-like growth factor 1 (IGF-1), insulin-like growth factor 2 (IGF-2), glial-derived neurotrophic factor (GDNF), nerve growth factor (NGF), neurotrophin-2 (NT-2), neurotrophin-3 (NT-3) neurotrophin-4/5 (NT-4/5), neurotrophin-6, conserved dopamine neurotrophic factor (CDNF), ciliary neurotrophic factor (CNTF), epidermal growth factor (EGF), a fibroblast growth factor (FGF), a bone morphogenetic protein (BMP), vascular endothelial growth factor (VEGF), granulocyte colony-stimulating factor (G-CSF), colony-stimulating factor (CSF), interferon-β (IFN-β), tumor necrosis factor-a (TNFa), tissue plasminogen activator (tPA), neurturin, persephin, artemin, neuropeptide Y (NPY), an ephrin, a semaphorin, other neuropoeitic factors, other neurotrophic factors, and a combination thereof, optionally wherein the neuroprotective or neuroregenerative protein is a secreted protein.

5. The method of any one of claims 1 to 4, wherein the donor template comprises a heterologous promoter, optionally wherein the heterologous promoter of the donor template comprises an inducible promoter or a cell-specific promoter.

6. The method of any one of claims 1 to 5, wherein the nucleotide sequence encoding the DNA nuclease comprises RNA.

7. The method of any one of claims 1 to 6, further comprising introducing into the human neural stem cell a DNA-targeting RNA, a truncated DNA-targeting RNA, or a nucleotide sequence encoding the DNA-targeting RNA or truncated DNA-targeting RNA.

8. The method of claim 7, wherein the DNA nuclease comprises a Cas polypeptide or a nucleotide sequence encoding the Cas polypeptide, and wherein the DNA-targeting RNA comprises a single guide RNA (sgRNA) or a truncated sgRNA comprising a first nucleotide sequence complementary to a portion of the safe harbor locus and a second nucleotide sequence that interacts with the Cas polypeptide, optionally wherein the Cas polypeptide comprises a Cas9 polypeptide, a variant thereof, or a fragment thereof, optionally wherein the Cas polypeptide variant comprises a high-fidelity or enhanced specificity Cas9 polypeptide variant.

9. The method of claim 8, wherein the sgRNA or truncated sgRNA comprises one or more modified nucleotides, and optionally:
(a) wherein the one or more modified nucleotides comprise a modification in the ribose group (optionally a modification at the 2' position of the ribose group, optionally selected from the group consisting of 2'-O-methyl, 2'-fluoro, 2'-deoxy, 2'-O-(2-methoxyethyl), and a combination thereof), a modification in the phosphate group, (optionally a phosphorothioate modification), a modification in the nucleobase, or a combination thereof, or
(b) wherein the one or more modified nucleotides are selected from the group consisting of a 2'-O-methyl nucleotide (M), a 2'-O-methyl, 3'-phosphorothioate nucleotide (MS), a 2'-O-methyl, 3'-thioPACE nucleotide (MSP), and a combination thereof.

10. The method of claim 9, wherein:
from about 10% to about 30% of the nucleotides in the first nucleotide sequence are modified nucleotides and/or from about 1% to about 10% of the nucleotides in the second nucleotide sequence are modified nucleotides.

11. The method of any one of claims 1 to 10, wherein the donor template further comprises a selectable marker, optionally wherein the selectable marker comprises a marker that is not expressed on a cell of the central nervous system, optionally wherein the selectable marker is a cell surface protein, optionally wherein the cell surface protein is selected from the group consisting of CD1, CD2, CD4, CD8α, CD10, CD19, CD20, a variant thereof, a fragment thereof, a derivative thereof, and a combination thereof.

12. The method of claim 11, further comprising purifying the genetically modified human neural stem cell based on expression of the selectable marker by the genetically modified human neural stem cell, optionally further comprising expanding the purified genetically modified human neural stem cell.

13. The method of any one of claims 1-12, wherein the isolated human neural stem cell expresses a combination of two or more markers selected from the group consisting of CD133, CD24, CD49f, CD29, and CD15.

14. A genetically modified human neural stem cell comprising a transgene cassette comprising a transgene, wherein the transgene cassette is located within a safe harbor locus, wherein the safe harbor locus is the IL2Rγ or HBB gene.

15. A composition for use in a method of preventing or treating a neurodegenerative disease or a neurological injury in an individual, comprising genetically modified human neural stem cells comprising a transgene cassette comprising a transgene wherein the transgene cassette is located within a safe harbor locus, wherein the safe harbor locus is the IL2Rγ or HBB gene.

## Patentansprüche

1. Verfahren zur Erzeugung einer genetisch modifizierten menschlichen neuralen Stammzelle, wobei das Verfahren Folgendes umfasst:
das Einbringen in eine isolierte menschliche neurale Stammzelle:
(a) einer Donormatrize, die Folgendes umfasst: (i) eine Transgen-Kassette, die ein Transgen umfasst; und (ii) zwei Nucleotidsequenzen, die zwei nicht überlappende, homologe Abschnitte eines Safe-Harbor-Locus umfassen, wobei sich die Nucleotidsequenzen an dem 5'- und 3'-Ende der Transgen-Kassette befinden; und
(b) einer DNA-Nuclease oder einer Nucleotidsequenz, die für die DNA-Nuclease kodiert, wobei die DNA-Nuclease ein Polypeptid eines CRISPR-assoziierten Proteins (Cas) ist, das zur Erzeugung eines Doppelstrangbruchs in dem Safe-Harbor-Locus in der Lage ist, um die Insertion des Transgens in den Safe-Harbor-Locus zu induzieren, wodurch eine genetisch modifizierte menschliche neurale Stammzelle erzeugt wird,
wobei der Safe-Harbor-Locus das IL2Rγ- oder das HBB-Gen ist.

2. Verfahren nach Anspruch 1, wobei die isolierte menschliche neurale Stammzelle eine primäre neurale Stammzelle umfasst oder a) von einer somatischen Stammzelle, b) von einer pluripotenten Stammzelle oder c) durch eine direkte Umprogrammierung von einer somatischen Zellpopulation abgeleitet ist.

3. Verfahren nach Anspruch 1 oder 2, wobei das Transgen für ein Protein kodiert, das mit einer genetischen Störung des zentralen Nervensystems in Zusammenhang steht, wobei das Protein gegebenenfalls von einem Gen kodiert wird, das aus der aus GALC (Morbus Krabbe), ABCD1 (Adrenoleukodystrophie), GFAP (Alexander-Krankheit), CYP27A1 (zerebrotendinöse Xanthomatose), ARSA (metachromatische Leukodystrophie), PLP1 (Pelizaeus-Merzbacher-Krankheit), ASPA (Morbus Canavan), EIF-2B (CACH-Syndrom), PHYH (Refsum-Krankheit 1), PEX7 (Refsum-Krankheit 2), PPT1 (infantile neuronale Ceroidlipofuszinose (NCL)), TPP1 (spät infantile NCL), CLN3 (juvenile NCL), CLN6 (adulte NCL), CLN5 (finnische spät infantile NCL-Variante), CLN6 (spät infantile NCL-Variante), MSFD8 (Ceroidlipofuszinose, neuronal, 7), CLN8 (Ceroidlipofuszinose, neuronal, 8), CTSD (Ceroidlipofuszinose, neuronal, 10), UBE3A (Angelman-Syndrom), POLG (Alpers-Syndrom), TAZ (Barth-Syndrom), GLA (Morbus Fabry), SLC20A2 (Morbus Fahr), PDE (Retinitis pigmentosa), SMN1 (spinale Muskelatrophie), IKBKAP (familiäre Dysautonomie), MeCP2 (Rett-Syndrom), CACNA1C (Timothy-Syndrom), ATXN3 (Machado-Joseph-Krankheit) und RPE65 (Lebersche kongenitale Amaurose) bestehenden Gruppe ausgewählt ist, wobei das Protein gegebenenfalls von der genetisch modifizierten menschlichen neuralen Stammzelle ausgewählt wird.

4. Verfahren nach Anspruch 1 oder 2, wobei das Transgen für ein neuroprotektives oder neurogeneratives Protein, eine Variante davon, ein Fragment davon oder ein Peptidmimetikum davon kodiert, wobei das neuroprotektive oder neurogenerative Protein gegebenenfalls aus der aus Gehirn-abgeleitetem neurotrophem Faktor (BDNF), Insulin-ähnlichem Wachstumsfaktor 1 (IGF-1), Insulin-ähnlichem Wachstumsfaktor 2 (IGF-2), Glia-abgeleitetem neurotrophem Faktor (GDNF), Nervenwachstumsfaktor (NGF), Neurotrophin-2 (NT-2), Neurotrophin-3 (NT-3), Neurotrophin-4/5 (NT-4/5), Neurotrophin-6, konserviertem-Dopaminneurotrophem-Faktor (CDNF), ziliärem neurotrophem Faktor (CNTF), epidermalem Wachstumsfaktor (EGF), Fibroblasten-Wachstumsfaktor (FGF), knochenmorphogenetischem Protein (BMP), Gefäßendothel-Wachstumsfaktor (VEGF), Granulozyten-Kolonie-stimulierendem Faktor (G-CSF), Kolonie-stimulierendem Faktor (CSF), Interferon-β (IFN-β), Tumor-Nekrose-Faktor-a (TNFa), Gewebe-Plasminogen-Aktivator (tPA), Neurturin, Persephin, Artemin, Neuropeptid Y (NPY), einem Ephrin, einem Semaphorin, anderen neuropoetischen Faktoren, anderen neurotrophen Faktoren und einer Kombination daraus bestehenden Gruppe ausgewählt ist, wobei das neuroprotektive oder neurogenerative Protein gegebenenfalls ein sekretiertes Protein ist.

5. Verfahren nach einem der Ansprüche 1 bis 4, wobei die Donormatrize einen heterologen Promotor umfasst, wobei der heterologe Promotor der Donormatrize gegebenenfalls einen induzierbaren Promotor oder einen zellspezifischen Promotor umfasst.

6. Verfahren nach einem der Ansprüche 1 bis 5, wobei die Nucleotidsequenz, die für die DNA-Nuclease kodiert, RNA umfasst.

7. Verfahren nach einem der Ansprüche 1 bis 6, das außerdem das Einbringen einer auf DNA abzielenden RNA, einer auf DNA abzielenden trunkierten RNA oder einer Nucleotidsequenz, die für die auf DNA abzielende RNA oder auf DNA abzielende trunkierte RNA kodiert, in die menschliche neurale Stammzelle umfasst.

8. Verfahren nach Anspruch 7, wobei die DNA-Nuclease ein Cas-Polypeptid oder eine Nucleotidsequenz, die für ein Cas-Polypeptid kodiert, umfasst und wobei die auf DNA abzielende RNA eine Single-Guide-RNA (sgRNA) oder eine trunkierte sgRNA umfasst, die eine erste Nucleotidsequenz, die komplementär zu einem Abschnitt des Safe-Harbor-Locus ist, und eine zweite Nucleotidsequenz umfasst, die mit dem Cas-Polypeptid wechselwirkt, wobei das Cas-Polypeptid gegebenenfalls ein Cas9-Polypeptid, eine Variante davon oder ein Fragment davon umfasst, wobei die Cas-Polypeptid-Variante gegebenenfalls eine Cas9-Polypeptidvariante mit hoher Genauigkeit oder verstärkter Spezifität umfasst.

9. Verfahren nach Anspruch 8, wobei die sgRNA oder trunkierte sgRNA ein oder mehrere modifizierte Nucleotide umfasst und wobei gegebenenfalls:
(a) das eine oder die mehreren modifizierten Nucleotide eine Modifikation in der Ribosegruppe (gegebenenfalls eine Modifikation an der 2'-Position der Ribosegruppe, gegebenenfalls aus der aus 2'-O-Methyl, 2'-Fluor, 2'-Desoxy, 2'-O-(2-Methoxyethyl) und einer Kombination daraus bestehenden Gruppe ausgewählt), eine Modifikation in der Phosphatgruppe (gegebenenfalls eine Thiophosphatmodifikation), eine Modifikation in der Nucleobase oder eine Kombination daraus umfasst oder
(b) das eine oder die mehreren modifizierten Nucleotide aus der aus einem 2'-O-Methylnucleotid (M), einem 2'-O-Methyl-3'-thiophosphatnucleotid (MS), einem 2'-O-Methyl-3'-thioPACE-Nucleotid (MSP) und einer Kombination daraus bestehenden Gruppe ausgewählt sind.

10. Verfahren nach Anspruch 9, wobei etwa 10 % bis etwa 30 % der Nucleotide in der ersten Nucleotidsequenz modifizierte Nucleotide sind und/oder etwa 1 % bis etwa 10 % der Nucleotide in der zweiten Nucleotidsequenz modifizierte Nucleotide sind.

11. Verfahren nach einem der Ansprüche 1 bis 10, wobei die Donormatrize außerdem einen selektierbaren Marker umfasst, wobei der selektierbare Marker gegebenenfalls einen Marker umfasst, der nicht auf einer Zelle der zentralen Nervensystems exprimiert wird, wobei der selektierbare Marker gegebenenfalls ein Zelloberflächenprotein ist, wobei das Zelloberflächenprotein gegebenenfalls aus der aus CD1, CD2, CD4, CD8a, CD10, CD19, CD20, einer Variante davon, einem Fragment davon, einem Derivat davon und einer Kombination daraus bestehenden Gruppe ausgewählt ist.

12. Verfahren nach Anspruch 11, das außerdem das Reinigen der genetisch modifizierten menschlichen neuralen Stammzelle basierend auf der Expression des selektierbaren Markers durch die genetisch modifizierte menschliche neurale Stammzelle umfasst und gegebenenfalls außerdem das Verlängern der gereinigten genetisch modifizierten menschlichen neuralen Stammzelle umfasst.

13. Verfahren nach einem der Ansprüche 1 bis 12, wobei die isolierte menschliche neurale Stammzelle eine Kombination aus zwei oder mehr Markern exprimiert, die aus der aus CD133, CD24, CD49f, CD29 und CD15 bestehenden Gruppe ausgewählt sind.

14. Genetisch modifizierte menschliche neurale Stammzelle, die eine Transgen-Kassette umfasst, die ein Transgen umfasst, wobei sich die Transgen-Kassette in einem Safe-Harbor-Locus befindet, wobei der Safe-Harbor-Locus das IL2Rγ- oder das HBB-Gen ist.

15. Zusammensetzung zur Verwendung in einem Verfahren zur Prävention oder Behandlung einer neurodegenerativen Erkrankung oder einer neurologischen Verletzung bei einem Individuum, die genetisch modifizierte menschliche neurale Stammzellen umfasst, die eine Transgen-Kassette mit einem Transgen umfasst, wobei sich die Transgen-Kassette in einem Safe-Harbor-Locus befindet, wobei der Safe-Harbor-Locus das IL2Rγ- oder das HBB-Gen ist.

## Revendications

1. Procédé de génération d'une cellule souche neurale humaine génétiquement modifiée, le procédé comprenant :
l'introduction dans une cellule souche neurale humaine isolée :
(a) d'une matrice donneuse comprenant: (i) une cassette transgénique comprenant un transgène ; et (ii) deux séquences nucléotidiques comprenant deux parties homologues non chevauchantes d'un locus refuge sûr, dans lequel les séquences nucléotidiques sont situées aux extrémités 5' et 3' de la cassette transgénique ; et
(b) d'une ADN nucléase ou d'une séquence nucléotidique codant pour l'ADN nucléase, dans lequel l'ADN nucléase est un polypeptide de la protéine associée à CRISPR (Cas) capable de créer une cassure double brin dans le locus refuge sûr pour induire l'insertion du transgène dans le locus refuge sûr, générant ainsi une cellule souche neurale humaine génétiquement modifiée,
dans lequel le locus refuge sûr est le gène IL2Rγ ou HBB.

2. Procédé selon la revendication 1, dans lequel la cellule souche neurale humaine isolée comprend une cellule souche neurale primaire ou est dérivée a) d'une cellule souche somatique, b) d'une cellule souche pluripotente, ou c) par reprogrammation directe à partir d'une population de cellules somatiques.

3. Procédé selon la revendication 1 ou 2, dans lequel le transgène code pour une protéine associée à une maladie génétique du système nerveux central, facultativement la protéine est codée par un gène choisi dans le groupe constitué de *GALC* (maladie de Krabbe), *ABCD1* (adrénoleucodystrophie), *GFAP* (maladie d'Alexander), *CYP27A1* (xanthomatose cérébrotendineuse), *ARSA* (leucodystrophie métachromatique), *PLP1* (maladie de Pelizaeus-Merzbacher), *ASPA* (maladie de Canavan), *EIF-2B* (leucoencéphalopathie avec disparition de la substance blanche), *PHYH* (maladie de Refsum 1), *PEX7* (maladie de Refsum 2), *PPT1* (céroïde-lipofuscinose neuronale (NCL) infantile), *TPP1* (NCL infantile tardive), *CLN3* (NCL juvénile), *CLN6* (NCL adulte), *CLN5* (NCL variante infantile finlandaise tardive), *CLN6* (NCL variante infantile tardive), *MSFD8* (céroïde-lipofuscinose, neuronale, 7), *CLN8* (céroïde-lipofuscinose, neuronale, 8), *CTSD* (céroïde-lipofuscinose, neuronale, 10), *UBE3A* (syndrome d'Angelman), *POLG* (maladie d'Alpers), *TAZ* (syndrome de Barth), *GLA* (maladie de Fabry), *SLC20A2* (syndrome de Fahr), *PDE* (rétinite pigmentaire), *SMN1* (amyotrophie spinale), *IKBKAP* (dysautonomie familiale), *MeCP2* (syndrome de Rett), *CACNA1C* (syndrome de Timothy), *ATXN3* (maladie de Machado-Joseph), et *RPE65* (amaurose congénitale de Leber), facultativement dans lequel la protéine est sélectionnée par la cellule souche neurale humaine génétiquement modifiée.

4. Procédé selon la revendication 1 ou 2, dans lequel le transgène code pour une protéine neuroprotectrice ou neurorégénérative, un variant de celle-ci, un fragment de celle-ci ou un peptide mimétique de celle-ci, facultativement dans lequel la protéine neuroprotectrice ou neurorégénérative est choisie dans le groupe constitué des facteur neurotrophique dérivé du cerveau (BDNF), facteur de croissance analogue à l'insuline 1 (IGF-1), facteur de croissance analogue à l'insuline 2 (IGF-2), facteur neurotrophique dérivé des cellules gliales (GDNF), facteur de croissance nerveuse (NGF), neurotrophine-2 (NT-2), neurotrophine-3 (NT-3), neurotrophine-4/5 (NT-4/5), neurotrophine-6, facteur neurotrophique dopaminergique conservé (CDNF), facteur neurotrophique ciliaire (CNTF), facteur de croissance épidermique (EGF), facteur de croissance des fibroblastes (FGF), protéine morphogénétique osseuse (BMP), facteur de croissance de l'endothélium vasculaire (VEGF), facteur de stimulation des colonies de granulocytes (G-CSF), facteur de stimulation des colonies (CSF), interféron-β (IFN-β), facteur de nécrose tumorale-α (TNFα), activateur tissulaire du plasminogène (tPA), neurturine, perséphine, artémine, neuropeptide Y (NPY), éphrine, sémaphorine, autres facteurs neuropoïétiques, autres facteurs neurotrophiques, et combinaison de ceux-ci, facultativement dans lequel la protéine neuroprotectrice ou neurorégénérative est une protéine sécrétée.

5. Procédé selon l'une quelconque des revendications 1 à 4, dans lequel la matrice donneuse comprend un promoteur hétérologue, facultativement dans lequel le promoteur hétérologue de la matrice donneuse comprend un promoteur inductible ou un promoteur spécifique aux cellules.

6. Procédé selon l'une quelconque des revendications 1 à 5, dans lequel la séquence nucléotidique codant pour l'ADN nucléase comprend de l'ARN.

7. Procédé selon l'une quelconque des revendications 1 à 6, comprenant en outre l'introduction dans la cellule souche neurale humaine d'un ARN ciblant l'ADN, d'un ARN ciblant l'ADN tronqué, ou d'une séquence nucléotidique codant pour l'ARN ciblant l'ADN ou l'ARN ciblant l'ADN tronqué.

8. Procédé selon la revendication 7, dans lequel l'ADN nucléase comprend un polypeptide Cas ou une séquence nucléotidique codant pour le polypeptide Cas, et dans lequel l'ARN ciblant l'ADN comprend un ARN guide unique (ARNsg) ou un ARNsg tronqué comprenant une première séquence nucléotidique complémentaire d'une partie du locus refuge sûr et une seconde séquence nucléotidique qui interagit avec le polypeptide Cas, facultativement dans lequel le polypeptide Cas comprend un polypeptide Cas9, un variant de celui-ci, ou un fragment de celui-ci, facultativement dans lequel le variant du polypeptide Cas comprend un variant du polypeptide Cas9 de haute-fidélité ou de spécificité améliorée.

9. Procédé selon la revendication 8, dans lequel l'ARNsg ou l'ARNsg tronqué comprend un ou plusieurs nucléotides modifiés, et facultativement :
(a) dans lequel les un ou plusieurs nucléotides modifiés comprennent une modification dans le groupe ribose (facultativement une modification en position 2' du groupe ribose, facultativement choisie dans le groupe constitué de 2'-O-méthyle, 2'-fluoro, 2'-désoxy, 2'-O-(2-méthoxyéthyle), et d'une combinaison de ceux-ci), une modification dans le groupe phosphate, (facultativement une modification phosphorothioate), une modification dans la nucléobase, ou une combinaison de celles-ci, ou
(b) dans lequel les un ou plusieurs nucléotides modifiés sont choisis dans le groupe constitué d'un nucléotide 2'-O-méthyle (M), d'un nucléotide 2'-O-méthyl,3'-phosphorothioate (MS), d'un nucléotide 2'-O-méthyl,3-thioPACE (MSP), et d'une combinaison de ceux-ci.

10. Procédé selon la revendication 9, dans lequel :
d'environ 10 % à environ 30 % des nucléotides dans la première séquence nucléotidique sont des nucléotides modifiés et/ou d'environ 1 % à environ 10 % des nucléotides dans la seconde séquence nucléotidique sont des nucléotides modifiés.

11. Procédé selon l'une quelconque des revendications 1 à 10, dans lequel la matrice donneuse comprend en outre un marqueur sélectionnable, facultativement dans lequel le marqueur sélectionnable comprend un marqueur qui n'est pas exprimé sur une cellule du système nerveux central, facultativement dans lequel le marqueur sélectionnable est une protéine de surface cellulaire, facultativement dans lequel la protéine de surface cellulaire est sélectionnée dans le groupe constitué de CD1, CD2, CD4, CD8α, CD10, CD19, CD20, d'un variant de ceux-ci, d'un fragment de ceux-ci, d'un dérivé de ceux-ci, et d'une combinaison de ceux-ci.

12. Procédé selon la revendication 11, comprenant en outre la purification de la cellule souche neurale humaine génétiquement modifiée sur la base de l'expression du marqueur sélectionnable par la cellule souche neurale humaine génétiquement modifiée, comprenant facultativement en outre l'expansion de la cellule souche neurale humaine génétiquement modifiée purifiée.

13. Procédé selon l'une quelconque des revendications 1 à 12, dans lequel la cellule souche neurale humaine isolée exprime une combinaison de deux ou plus de deux marqueurs choisis dans le groupe constitué de CD133, CD24, CD49f, CD29, et CD15.

14. Cellule souche neurale humaine génétiquement modifiée comprenant une cassette transgénique comprenant un transgène, dans laquelle la cassette transgénique est située dans un locus refuge sûr, le locus refuge sûr étant le gène IL2Rγ ou HBB.

15. Composition destinée à être utilisée dans une méthode de prévention ou de traitement d'une maladie neurodégénérative ou d'une lésion neurologique chez un individu, comprenant des cellules souches neurales humaines génétiquement modifiées comprenant une cassette transgénique comprenant un transgène, la cassette transgénique étant située dans un locus refuge sûr, dans lequel le locus refuge sûr est le gène IL2Rγ ou HBB.
